# EUROPEAN PATENT SPECIFICATION

(11) **EP 1 551 971 B1**
(45) Date of publication and mention of the grant of the patent: **24.12.2008**
(21) Application number: 03770964.9
(22) Date of filing: 10.10.2003
(51) Int. Cl.: C12N 15/11

(54) **SIRNA MEDIATED GENE SILENCING IN TRANSGENIC ANIMALS**
SIRNA-GESTEUERTE GENEXPRESSIONSUNTERDRÜCKUNG IN TRANSGENETISCHEN TIEREN
SILENCAGE GENIQUE INDUIT PAR SIRNA CHEZ LES ANIMAUX TRANSGENIQUES

(30) Priority: 17.10.2002 EP 02023283; 02.05.2003 US 467814 P; 10.07.2003 US 485969 P
(43) Date of publication of application: 13.07.2005
(73) Proprietor: TaconicArtemis GmbH, 51063 Köln (DE)
(72) Inventor: SEIBLER, Jost, 50733 Köln (DE); SCHWENK, Frieder, 50999 Köln (DE); KÜHN, Ralf, 50937 Köln (DE); KÜTER-LUKS, Birgit, 50670 Köln (DE)
(74) Representative: von Kreisler Selting Werner
(86) International application number: PCT/EP2003/011233
(87) International publication number: WO 2004/035782

(56) References cited:
- WO-A-03/057840
- DEVROE E & SILVER P A: "Retrovirus-delivered siRNA" BMC TECHNOLOGY, BIOMED CENTRAL, LONDON, GB, 28 August 2002 (2002-08-28), pages 1-5, XP002225637 ISSN: 1472-6750 cited in the application
- BRUMMELKAMP T R ET AL: "A system for stable expression of short interfering RNAs in mammalian cells" SCIENCE, AMERICAN ASSOCIATION FOR THE ADVANCEMENT OF SCIENCE,, US, vol. 296, no. 5567, 2002, pages 550-553, XP002225638 ISSN: 0036-8075 cited in the application
- PAUL C P ET AL: "EFFECTIVE EXPRESSION OF SMALL INTERFERING RNA IN HUMAN CELLS" NATURE BIOTECHNOLOGY, NATURE PUBLISHING, US, vol. 20, no. 5, May 2002 (2002-05), pages 505-508, XP001121066 ISSN: 1087-0156 cited in the application
- KISTNER ET AL: "Doxycycline-mediated quantitative and tissue-specific control of gene expression in transgenic mice" PROCEEDINGS OF THE NATIONAL ACADEMY OF SCIENCES OF USA, NATIONAL ACADEMY OF SCIENCE. WASHINGTON, US, vol. 93, no. 20, October 1996 (1996-10), pages 10933-10938, XP002110022 ISSN: 0027-8424
- BRONSON SARAH K ET AL: "Single-copy transgenic mice with chosen-site integration" PROCEEDINGS OF THE NATIONAL ACADEMY OF SCIENCES OF THE UNITED STATES, vol. 93, no. 17, 1996, pages 9067-9072, XP002270939 1996 ISSN: 0027-8424
- HASUWA H ET AL: "Small interfering RNA and gene silencing in transgenic mice and rats" FEBS LETTERS, ELSEVIER SCIENCE PUBLISHERS, AMSTERDAM, NL, vol. 532, no. 1-2, 4 December 2002 (2002-12-04), pages 227-230, XP004395375 ISSN: 0014-5793 cited in the application
- KAWASAKI H ET AL: "Short hairpin type of dsRNAs that are controlled by tRNA Val promoter significantly induce RNAi-mediated gene silencing in the cytoplasm of human cells" NUCLEIC ACIDS RESEARCH, OXFORD UNIVERSITY PRESS, SURREY, GB, vol. 31, no. 2, January 2003 (2003-01), pages 700-707, XP002965487 ISSN: 0305-1048
- MATSUKURA SHIRO ET AL: "Establishment of conditional vectors for hairpin siRNA knockdowns." NUCLEIC ACIDS RESEARCH. ENGLAND 1 AUG 2003, vol. 31, no. 15, 1 August 2003 (2003-08-01), pages (e77) 1-5, XP002270940 ISSN: 1362-4962
- VAN DE WETERING MARC ET AL: "Specific inhibition of gene expression using a stably integrated, inducible small-interfering-RNA vector." EMBO REPORTS, vol. 4, no. 6, June 2003 (2003-06), pages 609-615, XP002270941 ISSN: 1469-221X (ISSN print)
- MERLINO G T ET AL: "ACCURATE IN-VITRO TRANSCRIPTIONAL INITIATION OF THE CHICK ALPHA-2 TYPE I COLLAGEN GENE" JOURNAL OF BIOLOGICAL CHEMISTRY, vol. 256, no. 21, 1981, pages 11251-11258, XP002270942 ISSN: 0021-9258

### SEQUENCE LISTING

<110> ARTEMIS Pharmaceuticals GmbH
<120> siRNA mediated gene silencing in transgenic animals
<130> 032282wo JH/BM/short
<140>
   <141>
<160> 18
<170> PatentIn Ver. 2.1
<210> 1
   <211> 13139
   <212> DNA
   <213> Artificial Sequence
<220>
   <223> Description of Artificial Sequence: Rosa26 locus sequence
<400> 1
<210> 2
   <211> 5409
   <212> DNA
   <213> Artificial Sequence
<220>
   <223> Description of Artificial Sequence: sequence of homology region
<400> 2
   caggccctcc gagcgtggtg gagccgttct gtgagacagc cgggtacgag tcgtgacgct 60
   ggaaggggca agcgggtggt gggcaggaat gcggtccgcc ctgcagcaac cggaggggga 120
   gggagaaggg agcggaaaag tctccaccgg acgcggccat ggctcggggg ggggggggca 180
   gcggaggagc gcttccggcc gacgtctcgt cgctgattgg cttcttttcc tcccgccgtg 240
   tgtgaaaaca caaatggcgt gttttggttg gcgtaaggcg cctgtcagtt aacggcagcc 300
   ggagtgcgca gccgccggca gcctcgctct gcccactggg tggggcggga ggtaggtggg 360
   gtgaggcgag ctggacgtgc gggcgcggtc ggcctctggc ggggcggggg aggggaggga 420
   gggtcagcga aagtagctcg cgcgcgagcg gccgcccacc ctccccttcc tctgggggag 480
   tcgttttacc cgccgccggc cgggcctcgt cgtctgattg gctctcgggg cccagaaaac 540
   tggcccttgc cattggctcg tgttcgtgca agttgagtcc atccgccggc cagcgggggc 600
   ggcgaggagg cgctcccagg ttccggccct cccctcggcc ccgcgccgca gagtctggcc 660
   gcgcgcccct gcgcaacgtg gcaggaagcg cgcgctgggg gcggggacgg gcagtagggc 720
   tgagcggctg cggggcgggt gcaagcacgt ttccgacttg agttgcctca agaggggcgt 780
   gctgagccag acctccatcg cgcactccgg ggagtggagg gaaggagcga gggctcagtt 840
   gggctgtttt ggaggcagga agcacttgct ctcccaaagt cgctctgagt tgttatcagt 900
   aagggagctg cagtggagta ggcggggaga aggccgcacc cttctccgga ggggggaggg 960
   gagtgttgca atacctttct gggagttctc tgctgcctcc tggcttctga ggaccgccct 1020
   gggcctggga gaatcccttc cccctcttcc ctcgtgatct gcaactccag tctttctaga 1080
   agatgggcgg gagtcttctg ggcaggctta aaggctaacc tggtgtgtgg gcgttgtcct 1140
   gcaggggaat tgaacaggtg taaaattgga gggacaagac ttcccacaga ttttcggttt 1200
   tgtcgggaag ttttttaata ggggcaaata aggaaaatgg gaggataggt agtcatctgg 1260
   ggttttatgc agcaaaacta caggttatta ttgcttgtga tccgcctcgg agtattttcc 1320
   atcgaggtag attaaagaca tgctcacccg agttttatac tctcctgctt gagatcctta 1380
   ctacagtatg aaattacagt gtcgcgagtt agactatgta agcagaattt taatcatttt 1440
   taaagagccc agtacttcat atccatttct cccgctcctt ctgcagcctt atcaaaaggt 1500
   attttagaac actcatttta gccccatttt catttattat actggcttat ccaaccccta 1560
   gacagagcat tggcattttc cctttcctga tcttagaagt ctgatgactc atgaaaccag 1620
   acagattagt tacatacacc acaaatcgag gctgtagctg gggcctcaac actgcagttc 1680
   ttttataact ccttagtaca ctttttgttg atcctttgcc ttgatcctta attttcagtg 1740
   tctatcacct ctcccgtcag tggtgttcca catttgggcc tattctcagt ccagggagtt 1800
   ttacaacaat agatgtattg agaatccaac ctaaagctta actttccact cccatgaatg 1860
   cctctctcct ttttctccat ttataaactg agctattaac cattaatggt tccaggtgga 1920
   tgtctcctcc ccatattacc tgatgtatct tacatattgc caggctgata ttttaagaca 1980
   ttaaaaggta tatttcatta ttgagccaca tggtattgat tactgcttac taaaattttg 2040
   tcattgtaca catctgtaaa aggtggttcc ttttggaatg caaagttcag gtgtttgttg 2100
   tctttcctga cctaaggtct tgtgagcttg tattttttct atttaagcag tgctttctct 2160
   tggactggct tgactcatgg cattctacac gttattgctg gtctaaatgt gattttgcca 2220
   agcttcttca ggacctataa ttttgcttga cttgtagcca aacacaagta aaatgattaa 2280
   gcaacaaatg tatttgtgaa gcttggtttt taggttgttg tgttgtgtgt gcttgtgctc 2340
   tataataata ctatccaggg gctggagagg tggctcggag ttcaagagca cagactgctc 2400
   ttccagaagt cctgagttca attcccagca accacatggt ggctcacaac catctgtaat 2460
   gggatctgat gccctcttct ggtgtgtctg aagaccacaa gtgtattcac attaaataaa 2520
   taaatcctcc ttcttcttct tttttttttt tttaaagaga atactgtctc cagtagaatt 2580
   tactgaagta atgaaatact ttgtgtttgt tccaatatgg tagccaataa tcaaattact 2640
   ctttaagcac tggaaatgtt accaaggaac taatttttat ttgaagtgta actgtggaca 2700
   gaggagccat aactgcagac ttgtgggata cagaagacca atgcagactt taatgtcttt 2760
   tctcttacac taagcaataa agaaataaaa attgaacttc tagtatccta tttgtttaaa 2820
   ctgctagctt tacttaactt ttgtgcttca tctatacaaa gctgaaagct aagtctgcag 2880
   ccattactaa acatgaaagc aagtaatgat aattttggat ttcaaaaatg tagggccaga 2940
   gtttagccag ccagtggtgg tgcttgcctt tatgccttta atcccagcac tctggaggca 3000
   gagacaggca gatctctgag tttgagccca gcctggtcta cacatcaagt tctatctagg 3060
   atagccagga atacacacag aaaccctgtt ggggaggggg gctctgagat ttcataaaat 3120
   tataattgaa gcattcccta atgagccact atggatgtgg ctaaatccgt ctacctttct 3180
   gatgagattt gggtattatt ttttctgtct ctgctgttgg ttgggtcttt tgacactgtg 3240
   ggctttcttt aaagcctcct tcctgccatg tggtctcttg tttgctacta acttcccatg 3300
   gcttaaatgg catggctttt tgccttctaa gggcagctgc tgagatttgc agcctgattt 3360
   ccagggtggg gttgggaaat ctttcaaaca ctaaaattgt cctttaattt tttttttaaa 3420
   aaatgggtta tataataaac ctcataaaat agttatgagg agtgaggtgg actaatatta 3480
   aatgagtccc tcccctataa aagagctatt aaggcttttt gtcttatact taactttttt 3540
   tttaaatgtg gtatctttag aaccaagggt cttagagttt tagtatacag aaactgttgc 3600
   atcgcttaat cagattttct agtttcaaat ccagagaatc caaattcttc acagccaaag 3660
   tcaaattaag aatttctgac ttttaatgtt aatttgctta ctgtgaatat aaaaatgata 3720
   gcttttcctg aggcagggtc tcactatgta tctctgcctg atctgcaaca agatatgtag 3780
   actaaagttc tgcctgcttt tgtctcctga atactaaggt taaaatgtag taatactttt 3840
   ggaacttgca ggtcagattc ttttataggg gacacactaa gggagcttgg gtgatagttg 3900
   gtaaaatgtg tttcaagtga tgaaaacttg aattattatc accgcaacct actttttaaa 3960
   aaaaaaagcc aggcctgtta gagcatgctt aagggatccc taggacttgc tgagcacaca 4020
   agagtagtta cttggcaggc tcctggtgag agcatatttc aaaaaacaag gcagacaacc 4080
   aagaaactac agttaaggtt acctgtcttt aaaccatctg catatacaca gggatattaa 4140
   aatattccaa ataatatttc attcaagttt tcccccatca aattgggaca tggatttctc 4200
   cggtgaatag gcagagttgg aaactaaaca aatgttggtt ttgtgatttg tgaaattgtt 4260
   ttcaagtgat agttaaagcc catgagatac agaacaaagc tgctatttcg aggtctcttg 4320
   gtttatactc agaagcactt ctttgggttt ccctgcacta tcctgatcat gtgctaggcc 4380
   taccttaggc tgattgttgt tcaaataaac ttaagtttcc tgtcaggtga tgtcatatga 4440
   tttcatatat caaggcaaaa catgttatat atgttaaaca tttgtactta atgtgaaagt 4500
   taggtctttg tgggtttgat ttttaatttt caaaacctga gctaaataag tcatttttac 4560
   atgtcttaca tttggtggaa ttgtataatt gtggtttgca ggcaagactc tctgacctag 4620
   taaccctacc tatagagcac tttgctgggt cacaagtcta ggagtcaagc atttcacctt 4680
   gaagttgaga cgttttgtta gtgtatacta gtttatatgt tggaggacat gtttatccag 4740
   aagatattca ggactatttt tgactgggct aaggaattga ttctgattag cactgttagt 4800
   gagcattgag tggcctttag gcttgaattg gagtcacttg tatatctcaa ataatgctgg 4860
   ccttttttaa aaagcccttg ttctttatca ccctgttttc tacataattt ttgttcaaag 4920
   aaatacttgt ttggatctcc ttttgacaac aatagcatgt tttcaagcca tatttttttt 4980
   cctttttttt tttttttttg gtttttcgag acagggtttc tctgtatagc cctggctgtc 5040
   ctggaactca ctttgtagac caggctggcc tcgaactcag aaatccgcct gcctctgcct 5100
   cctgagtgcc gggattaaag gcgtgcacca ccacgcctgg ctaagttgga tattttgtta 5160
   tataactata accaatacta actccactgg gtggattttt aattcagtca gtagtcttaa 5220
   gtggtcttta ttggcccttc attaaaatct actgttcact ctaacagagg ctgttggtac 5280
   tagtggcact taagcaactt cctacggata tactagcaga ttaagggtca gggatagaaa 5340
   ctagtctagc gttttgtata cctaccagct ttatactacc ttgttctgat agaaatattt 5400
   caggacatc                                                          5409
<210> 3
   <211> 4413
   <212> DNA
   <213> Artificial Sequence
<220>
   <223> Description of Artificial Sequence: Sequence of Fluc-hygro insert
<400> 3
   tctaggtaac cgatatccct gcaggggtga cctgcacgtc tagggcgcag tagtccaggg 60
   tttccttgat gatgtcatac ttatcctgtc cctttttttt ccacagctcg cggttgagga 120
   caaactcttc gcggtctttc cagtactcct gcaggtgact gactgagtcg agatctgcga 180
   tctaagtaag cttggcattc cggtactgtt ggtaaagcca ccatggaaga cgccaaaaac 240
   ataaagaaag gcccggcgcc attctatccg ctggaagatg gaaccgctgg agagcaactg 300
   cataaggcta tgaagagata cgccctggtt cctggaacaa ttgcttttac agatgcacat 360
   atcgaggtgg acatcactta cgctgagtac ttcgaaatgt ccgttcggtt ggcagaagct 420
   atgaaacgat atgggctgaa tacaaatcac agaatcgtcg tatgcagtga aaactctctt 480
   caattcttta tgccggtgtt gggcgcgtta tttatcggag ttgcagttgc gcccgcgaac 540
   gacatttata atgaacgtga attgctcaac agtatgggca tttcgcagcc taccgtggtg 600
   ttcgtttcca aaaaggggtt gcaaaaaatt ttgaacgtgc aaaaaaagct cccaatcatc 660
   caaaaaatta ttatcatgga ttctaaaacg gattaccagg gatttcagtc gatgtacacg 720
   ttcgtcacat ctcatctacc tcccggtttt aatgaatacg attttgtgcc agagtccttc 780
   gatagggaca agacaattgc actgatcatg aactcctctg gatctactgg tctgcctaaa 840
   ggtgtcgctc tgcctcatag aactgcctgc gtgagattct cgcatgccag agatcctatt 900
   tttggcaatc aaatcattcc ggatactgcg attttaagtg ttgttccatt ccatcacggt 960
   tttggaatgt ttactacact cggatatttg atatgtggat ttcgagtcgt cttaatgtat 1020
   agatttgaag aagagctgtt tctgaggagc cttcaggatt acaagattca aagtgcgctg 1080
   ctggtgccaa ccctattctc cttcttcgcc aaaagcactc tgattgacaa atacgattta 1140
   tctaatttac acgaaattgc ttctggtggc gctcccctct ctaaggaagt cggggaagcg 1200
   gttgccaaga ggttccatct gccaggtatc aggcaaggat atgggctcac tgagactaca 1260
   tcagctattc tgattacacc cgagggggat gataaaccgg gcgcggtcgg taaagttgtt 1320
   ccattttttg aagcgaaggt tgtggatctg gataccggga aaacgctggg cgttaatcaa 1380
   agaggcgaac tgtgtgtgag aggtcctatg attatgtccg gttatgtaaa caatccggaa 1440
   gcgaccaacg ccttgattga caaggatgga tggctacatt ctggagacat agcttactgg 1500
   gacgaagacg aacacttctt catcgttgac cgcctgaagt ctctgattaa gtacaaaggc 1560
   tatcaggtgg ctcccgctga attggaatcc atcttgctcc aacaccccaa catcttcgac 1620
   gcaggtgtcg caggtcttcc cgacgatgac gccggtgaac ttcccgccgc cgttgttgtt 1680
   ttggagcacg gaaagacgat gacggaaaaa gagatcgtgg attacgtcgc cagtcaagta 1740
   acaaccgcga aaaagttgcg cggaggagtt gtgtttgtgg acgaagtacc gaaaggtctt 1800
   accggaaaac tcgacgcaag aaaaatcaga gagatcctca taaaggccaa gaagggcgga 1860
   aagatcgccg tgtaattcta gaccggttcg agatccaggc gcggatcaat aaaagatcat 1920
   tattttcaat agatctgtgt gttggttttt tgtgtgcctt gggggagggg gaggccagaa 1980
   tgaggcgcgg ccaaggggga gggggaggcc agaatgacct tgggggaggg ggaggccaga 2040
   atgaccttgg gggaggggga ggccagaatg aggcgcgccc ccgatccgtc gacgccctaa 2100
   ggccatagcg gccgccctga ggccgcgggc gatcgcctag ggtaaccgaa agttcctata 2160
   ctttctagag aataggaact tcggaatagg aacttcttat aatctagaag atctggatcc 2220
   acgattcgag ggcccctgca ggtcaattct accgggtagg ggaggcgctt ttcccaaggc 2280
   agtctggagc atgcgcttta gcagccccgc tggcacttgg cgctacacaa gtggcctctg 2340
   gcctcgcaca cattccacat ccaccggtag cgccaaccgg ctccgttctt tggtggcccc 2400
   ttcgcgccac cttctactcc tcccctagtc aggaagttcc cccccgcccc gcagctcgcg 2460
   tcgtgcagga cgtgacaaat ggaagtagca cgtctcacta gtctcgtgca gatggacagc 2520
   accgctgagc aatggaagcg ggtaggcctt tggggcagcg gccaatagca gctttgctcc 2580
   ttcgctttct gggctcagag gctgggaagg ggtgggtccg ggggcgggct caggggcggg 2640
   ctcaggggcg gggcgggcgc gaaggtcctc ccgaggcccg gcattctcgc acgcttcaaa 2700
   agcgcacgtc tgccgcgctg ttctcctctt cctcatctcc gggcctttcg acgatccagc 2760
   cgccaccatg aaaaagcctg aactcaccgc gacgtctgtc gagaagtttc tgatcgaaaa 2820
   gttcgacagc gtctccgacc tgatgcagct ctcggagggc gaagaatctc gtgctttcag 2880
   cttcgatgta ggagggcgtg gatatgtcct gcgggtaaat agctgcgccg atggtttcta 2940
   caaagatcgt tatgtttatc ggcactttgc atcggccgcg ctcccgattc cggaagtgct 3000
   tgacattggg gaattcagcg agagcctgac ctattgcatc tcccgccgtg cacagggtgt 3060
   cacgttgcaa gacctgcctg aaaccgaact gcccgctgtt ctgcagccgg tcgcggaggc 3120
   catggatgcc atcgctgcgg ccgatcttag ccagacgagc gggttcggcc cattcggacc 3180
   gcaaggaatc ggtcaataca ctacatggcg tgatttcata tgcgcgattg ctgatcccca 3240
   tgtgtatcac tggcaaactg tgatggacga caccgtcagt gcgtccgtcg cgcaggctct 3300
   cgatgagctg atgctttggg ccgaggactg ccccgaagtc cggcacctcg tgcacgcgga 3360
   tttcggctcc aacaatgtcc tgacggacaa tggccgcata acagcggtca ttgactggag 3420
   cgaggcgatg ttcggggatt cccaatacga ggtcgccaac atcttcttct ggaggccgtg 3480
   gttggcttgt atggagcagc agacgcgcta cttcgagcgg aggcatccgg agcttgcagg 3540
   atcgccgcgg ctccgggcgt atatgctccg cattggtctt gaccaactct atcagagctt 3600
   ggttgacggc aatttcgatg atgcagcttg ggcgcagggt cgatgcgacg caatcgtccg 3660
   atccggagcc gggactgtcg ggcgtacaca aatcgcccgc agaagcgcgg ccgtctggac 3720
   cgatggctgt gtagaagtac tcgccgatag tggaaaccga cgccccagca ctcgtccgag 3780
   ggcaaaggaa tagtcgatgc agaaattgat gatctattaa acaataaaga tgtccactaa 3840
   aatggaagtt tttcctgtca tactttgtta agaagggtga gaacagagta cctacatttt 3900
   gaatggaagg attggagcta cgggggtggg ggtggggtgg gattagataa atgcctgctc 3960
   tttactgaag gctctttact attgctttat gataatgttt catagttgga tatcataatt 4020
   taaacaagca aaaccaaatt aagggccagc tcattcctcc cactcatgat ctatagatct 4080
   atagatctct cgtgggatca ttgtttttct cttgattccc actttgtggt tctaagtact 4140
   gtggtttcca aatgtgtcag tttcatagcc tgaagaacga gatcagcagc ctctgttcca 4200
   catacacttc attctcagta ttgttttgcc aagttctaat tccatcagaa gctgactcta 4260
   gatcctgcag gaattcatat gaagttccta tactttctag agaataggaa cttcggaata 4320
   ggaacttcaa aatgtcgcgg cgcgccggta accgaagttc ctatactttc tagagaatag 4380
   gaacttcgga ataggaactt caagcttaag cgc                             4413
<210> 4
   <211> 14947
   <212> DNA
   <213> Artificial Sequence
<220>
   <223> Description of Artificial Sequence: Targeting vector for Rosa26 locus with a Fluc-hygro insert
<400> 4
   ctagggataa cagggtaata tagccgcggc aggccctccg agcgtggtgg agccgttctg 60
   tgagacagcc gggtacgagt cgtgacgctg gaaggggcaa gcgggtggtg ggcaggaatg 120
   cggtccgccc tgcagcaacc ggagggggag ggagaaggga gcggaaaagt ctccaccgga 180
   cgcggccatg gctcgggggg gggggggcag cggaggagcg cttccggccg acgtctcgtc 240
   gctgattggc ttcttttcct cccgccgtgt gtgaaaacac aaatggcgtg ttttggttgg 300
   cgtaaggcgc ctgtcagtta acggcagccg gagtgcgcag ccgccggcag cctcgctctg 360
   cccactgggt ggggcgggag gtaggtgggg tgaggcgagc tggacgtgcg ggcgcggtcg 420
   gcctctggcg gggcggggga ggggagggag ggtcagcgaa agtagctcgc gcgcgagcgg 480
   ccgcccaccc tccccttcct ctgggggagt cgttttaccc gccgccggcc gggcctcgtc 540
   gtctgattgg ctctcggggc ccagaaaact ggcccttgcc attggctcgt gttcgtgcaa 600
   gttgagtcca tccgccggcc agcgggggcg gcgaggaggc gctcccaggt tccggccctc 660
   ccctcggccc cgcgccgcag agtctggccg cgcgcccctg cgcaacgtgg caggaagcgc 720
   gcgctggggg cggggacggg cagtagggct gagcggctgc ggggcgggtg caagcacgtt 780
   tccgacttga gttgcctcaa gaggggcgtg ctgagccaga cctccatcgc gcactccggg 840
   gagtggaggg aaggagcgag ggctcagttg ggctgttttg gaggcaggaa gcacttgctc 900
   tcccaaagtc gctctgagtt gttatcagta agggagctgc agtggagtag gcggggagaa 960
   ggccgcaccc ttctccggag gggggagggg agtgttgcaa tacctttctg ggagttctct 1020
   gctgcctcct ggcttctgag gaccgccctg ggcctgggag aatcccttcc ccctcttccc 1080
   tcgtgatctg caactccagt ctttctaggt aaccgatatc cctgcagggg tgacctgcac 1140
   gtctagggcg cagtagtcca gggtttcctt gatgatgtca tacttatcct gtcccttttt 1200
   tttccacagc tcgcggttga ggacaaactc ttcgcggtct ttccagtact cctgcaggtg 1260
   actgactgag tcgagatctg cgatctaagt aagcttggca ttccggtact gttggtaaag 1320
   ccaccatgga agacgccaaa aacataaaga aaggcccggc gccattctat ccgctggaag 1380
   atggaaccgc tggagagcaa ctgcataagg ctatgaagag atacgccctg gttcctggaa 1440
   caattgcttt tacagatgca catatcgagg tggacatcac ttacgctgag tacttcgaaa 1500
   tgtccgttcg gttggcagaa gctatgaaac gatatgggct gaatacaaat cacagaatcg 1560
   tcgtatgcag tgaaaactct cttcaattct ttatgccggt gttgggcgcg ttatttatcg 1620
   gagttgcagt tgcgcccgcg aacgacattt ataatgaacg tgaattgctc aacagtatgg 1680
   gcatttcgca gcctaccgtg gtgttcgttt ccaaaaaggg gttgcaaaaa attttgaacg 1740
   tgcaaaaaaa gctcccaatc atccaaaaaa ttattatcat ggattctaaa acggattacc 1800
   agggatttca gtcgatgtac acgttcgtca catctcatct acctcccggt tttaatgaat 1860
   acgattttgt gccagagtcc ttcgataggg acaagacaat tgcactgatc atgaactcct 1920
   ctggatctac tggtctgcct aaaggtgtcg ctctgcctca tagaactgcc tgcgtgagat 1980
   tctcgcatgc cagagatcct atttttggca atcaaatcat tccggatact gcgattttaa 2040
   gtgttgttcc attccatcac ggttttggaa tgtttactac actcggatat ttgatatgtg 2100
   gatttcgagt cgtcttaatg tatagatttg aagaagagct gtttctgagg agccttcagg 2160
   attacaagat tcaaagtgcg ctgctggtgc caaccctatt ctccttcttc gccaaaagca 2220
   ctctgattga caaatacgat ttatctaatt tacacgaaat tgcttctggt ggcgctcccc 2280
   tctctaagga agtcggggaa gcggttgcca agaggttcca tctgccaggt atcaggcaag 2340
   gatatgggct cactgagact acatcagcta ttctgattac acccgagggg gatgataaac 2400
   cgggcgcggt cggtaaagtt gttccatttt ttgaagcgaa ggttgtggat ctggataccg 2460
   ggaaaacgct gggcgttaat caaagaggcg aactgtgtgt gagaggtcct atgattatgt 2520
   ccggttatgt aaacaatccg gaagcgacca acgccttgat tgacaaggat ggatggctac 2580
   attctggaga catagcttac tgggacgaag acgaacactt cttcatcgtt gaccgcctga 2640
   agtctctgat taagtacaaa ggctatcagg tggctcccgc tgaattggaa tccatcttgc 2700
   tccaacaccc caacatcttc gacgcaggtg tcgcaggtct tcccgacgat gacgccggtg 2760
   aacttcccgc cgccgttgtt gttttggagc acggaaagac gatgacggaa aaagagatcg 2820
   tggattacgt cgccagtcaa gtaacaaccg cgaaaaagtt gcgcggagga gttgtgtttg 2880
   tggacgaagt accgaaaggt cttaccggaa aactcgacgc aagaaaaatc agagagatcc 2940
   tcataaaggc caagaagggc ggaaagatcg ccgtgtaatt ctagaccggt tcgagatcca 3000
   ggcgcggatc aataaaagat cattattttc aatagatctg tgtgttggtt ttttgtgtgc 3060
   cttgggggag ggggaggcca gaatgaggcg cggccaaggg ggagggggag gccagaatga 3120
   ccttggggga gggggaggcc agaatgacct tgggggaggg ggaggccaga atgaggcgcg 3180
   cccccgatcc gtcgacgccc taaggccata gcggccgccc tgaggccgcg ggcgatcgcc 3240
   taggggtaac cgaagttcct atactttcta gagaatagga acttcggaat aggaacttct 3300
   tataatctag aagatctgga tccacgattc gagggcccct gcaggtcaat tctaccgggt 3360
   aggggaggcg cttttcccaa ggcagtctgg agcatgcgct ttagcagccc cgctggcact 3420
   tggcgctaca caagtggcct ctggcctcgc acacattcca catccaccgg tagcgccaac 3480
   cggctccgtt ctttggtggc cccttcgcgc caccttctac tcctccccta gtcaggaagt 3540
   tcccccccgc cccgcagctc gcgtcgtgca ggacgtgaca aatggaagta gcacgtctca 3600
   ctagtctcgt gcagatggac agcaccgctg agcaatggaa gcgggtaggc ctttggggca 3660
   gcggccaata gcagctttgc tccttcgctt tctgggctca gaggctggga aggggtgggt 3720
   ccgggggcgg gctcaggggc gggctcaggg gcggggcggg cgcgaaggtc ctcccgaggc 3780
   ccggcattct cgcacgcttc aaaagcgcac gtctgccgcg ctgttctcct cttcctcatc 3840
   tccgggcctt tcgacgatcc agccgccacc atgaaaaagc ctgaactcac cgcgacgtct 3900
   gtcgagaagt ttctgatcga aaagttcgac agcgtctccg acctgatgca gctctcggag 3960
   ggcgaagaat ctcgtgcttt cagcttcgat gtaggagggc gtggatatgt cctgcgggta 4020
   aatagctgcg ccgatggttt ctacaaagat cgttatgttt atcggcactt tgcatcggcc 4080
   gcgctcccga ttccggaagt gcttgacatt ggggaattca gcgagagcct gacctattgc 4140
   atctcccgcc gtgcacaggg tgtcacgttg caagacctgc ctgaaaccga actgcccgct 4200
   gttctgcagc cggtcgcgga ggccatggat gccatcgctg cggccgatct tagccagacg 4260
   agcgggttcg gcccattcgg accgcaagga atcggtcaat acactacatg gcgtgatttc 4320
   atatgcgcga ttgctgatcc ccatgtgtat cactggcaaa ctgtgatgga cgacaccgtc 4380
   agtgcgtccg tcgcgcaggc tctcgatgag ctgatgcttt gggccgagga ctgccccgaa 4440
   gtccggcacc tcgtgcacgc ggatttcggc tccaacaatg tcctgacgga caatggccgc 4500
   ataacagcgg tcattgactg gagcgaggcg atgttcgggg attcccaata cgaggtcgcc 4560
   aacatcttct tctggaggcc gtggttggct tgtatggagc agcagacgcg ctacttcgag 4620
   cggaggcatc cggagcttgc aggatcgccg cggctccggg cgtatatgct ccgcattggt 4680
   cttgaccaac tctatcagag cttggttgac ggcaatttcg atgatgcagc ttgggcgcag 4740
   ggtcgatgcg acgcaatcgt ccgatccgga gccgggactg tcgggcgtac acaaatcgcc 4800
   cgcagaagcg cggccgtctg gaccgatggc tgtgtagaag tactcgccga tagtggaaac 4860
   cgacgcccca gcactcgtcc gagggcaaag gaatagtcga tgcagaaatt gatgatctat 4920
   taaacaataa agatgtccac taaaatggaa gtttttcctg tcatactttg ttaagaaggg 4980
   tgagaacaga gtacctacat tttgaatgga aggattggag ctacgggggt gggggtgggg 5040
   tgggattaga taaatgcctg ctctttactg aaggctcttt actattgctt tatgataatg 5100
   tttcatagtt ggatatcata atttaaacaa gcaaaaccaa attaagggcc agctcattcc 5160
   tcccactcat gatctataga tctatagatc tctcgtggga tcattgtttt tctcttgatt 5220
   cccactttgt ggttctaagt actgtggttt ccaaatgtgt cagtttcata gcctgaagaa 5280
   cgagatcagc agcctctgtt ccacatacac ttcattctca gtattgtttt gccaagttct 5340
   aattccatca gaagctgact ctagatcctg caggaattca tatgaagttc ctatactttc 5400
   tagagaatag gaacttcgga ataggaactt caaaatgtcg cggcgcgccg gtaaccgaag 5460
   ttcctatact ttctagagaa taggaacttc ggaataggaa cttcaagctt aagcgctaga 5520
   agatgggcgg gagtcttctg ggcaggctta aaggctaacc tggtgtgtgg gcgttgtcct 5580
   gcaggggaat tgaacaggtg taaaattgga gggacaagac ttcccacaga ttttcggttt 5640
   tgtcgggaag ttttttaata ggggcaaata aggaaaatgg gaggataggt agtcatctgg 5700
   ggttttatgc agcaaaacta caggttatta ttgcttgtga tccgcctcgg agtattttcc 5760
   atcgaggtag attaaagaca tgctcacccg agttttatac tctcctgctt gagatcctta 5820
   ctacagtatg aaattacagt gtcgcgagtt agactatgta agcagaattt taatcatttt 5880
   taaagagccc agtacttcat atccatttct cccgctcctt ctgcagcctt atcaaaaggt 5940
   attttagaac actcatttta gccccatttt catttattat actggcttat ccaaccccta 6000
   gacagagcat tggcattttc cctttcctga tcttagaagt ctgatgactc atgaaaccag 6060
   acagattagt tacatacacc acaaatcgag gctgtagctg gggcctcaac actgcagttc 6120
   ttttataact ccttagtaca ctttttgttg atcctttgcc ttgatcctta attttcagtg 6180
   tctatcacct ctcccgtcag tggtgttcca catttgggcc tattctcagt ccagggagtt 6240
   ttacaacaat agatgtattg agaatccaac ctaaagctta actttccact cccatgaatg 6300
   cctctctcct ttttctccat ttataaactg agctattaac cattaatggt tccaggtgga 6360
   tgtctcctcc ccatattacc tgatgtatct tacatattgc caggctgata ttttaagaca 6420
   ttaaaaggta tatttcatta ttgagccaca tggtattgat tactgcttac taaaattttg 6480
   tcattgtaca catctgtaaa aggtggttcc ttttggaatg caaagttcag gtgtttgttg 6540
   tctttcctga cctaaggtct tgtgagcttg tattttttct atttaagcag tgctttctct 6600
   tggactggct tgactcatgg cattctacac gttattgctg gtctaaatgt gattttgcca 6660
   agcttcttca ggacctataa ttttgcttga cttgtagcca aacacaagta aaatgattaa 6720
   gcaacaaatg tatttgtgaa gcttggtttt taggttgttg tgttgtgtgt gcttgtgctc 6780
   tataataata ctatccaggg gctggagagg tggctcggag ttcaagagca cagactgctc 6840
   ttccagaagt cctgagttca attcccagca accacatggt ggctcacaac catctgtaat 6900
   gggatctgat gccctcttct ggtgtgtctg aagaccacaa gtgtattcac attaaataaa 6960
   taaatcctcc ttcttcttct tttttttttt tttaaagaga atactgtctc cagtagaatt 7020
   tactgaagta atgaaatact ttgtgtttgt tccaatatgg tagccaataa tcaaattact 7080
   ctttaagcac tggaaatgtt accaaggaac taatttttat ttgaagtgta actgtggaca 7140
   gaggagccat aactgcagac ttgtgggata cagaagacca atgcagactt taatgtcttt 7200
   tctcttacac taagcaataa agaaataaaa attgaacttc tagtatccta tttgtttaaa 7260
   ctgctagctt tacttaactt ttgtgcttca tctatacaaa gctgaaagct aagtctgcag 7320
   ccattactaa acatgaaagc aagtaatgat aattttggat ttcaaaaatg tagggccaga 7380
   gtttagccag ccagtggtgg tgcttgcctt tatgccttta atcccagcac tctggaggca 7440
   gagacaggca gatctctgag tttgagccca gcctggtcta cacatcaagt tctatctagg 7500
   atagccagga atacacacag aaaccctgtt ggggaggggg gctctgagat ttcataaaat 7560
   tataattgaa gcattcccta atgagccact atggatgtgg ctaaatccgt ctacctttct 7620
   gatgagattt gggtattatt ttttctgtct ctgctgttgg ttgggtcttt tgacactgtg 7680
   ggctttcttt aaagcctcct tcctgccatg tggtctcttg tttgctacta acttcccatg 7740
   gcttaaatgg catggctttt tgccttctaa gggcagctgc tgagatttgc agcctgattt 7800
   ccagggtggg gttgggaaat ctttcaaaca ctaaaattgt cctttaattt tttttttaaa 7860
   aaatgggtta tataataaac ctcataaaat agttatgagg agtgaggtgg actaatatta 7920
   aatgagtccc tcccctataa aagagctatt aaggcttttt gtcttatact taactttttt 7980
   tttaaatgtg gtatctttag aaccaagggt cttagagttt tagtatacag aaactgttgc 8040
   atcgcttaat cagattttct agtttcaaat ccagagaatc caaattcttc acagccaaag 8100
   tcaaattaag aatttctgac ttttaatgtt aatttgctta ctgtgaatat aaaaatgata 8160
   gcttttcctg aggcagggtc tcactatgta tctctgcctg atctgcaaca agatatgtag 8220
   actaaagttc tgcctgcttt tgtctcctga atactaaggt taaaatgtag taatactttt 8280
   ggaacttgca ggtcagattc ttttataggg gacacactaa gggagcttgg gtgatagttg 8340
   gtaaaatgtg tttcaagtga tgaaaacttg aattattatc accgcaacct actttttaaa 8400
   aaaaaaagcc aggcctgtta gagcatgctt aagggatccc taggacttgc tgagcacaca 8460
   agagtagtta cttggcaggc tcctggtgag agcatatttc aaaaaacaag gcagacaacc 8520
   aagaaactac agttaaggtt acctgtcttt aaaccatctg catatacaca gggatattaa 8580
   aatattccaa ataatatttc attcaagttt tcccccatca aattgggaca tggatttctc 8640
   cggtgaatag gcagagttgg aaactaaaca aatgttggtt ttgtgatttg tgaaattgtt 8700
   ttcaagtgat agttaaagcc catgagatac agaacaaagc tgctatttcg aggtctcttg 8760
   gtttatactc agaagcactt ctttgggttt ccctgcacta tcctgatcat gtgctaggcc 8820
   taccttaggc tgattgttgt tcaaataaac ttaagtttcc tgtcaggtga tgtcatatga 8880
   tttcatatat caaggcaaaa catgttatat atgttaaaca tttgtactta atgtgaaagt 8940
   taggtctttg tgggtttgat ttttaatttt caaaacctga gctaaataag tcatttttac 9000
   atgtcttaca tttggtggaa ttgtataatt gtggtttgca ggcaagactc tctgacctag 9060
   taaccctacc tatagagcac tttgctgggt cacaagtcta ggagtcaagc atttcacctt 9120
   gaagttgaga cgttttgtta gtgtatacta gtttatatgt tggaggacat gtttatccag 9180
   aagatattca ggactatttt tgactgggct aaggaattga ttctgattag cactgttagt 9240
   gagcattgag tggcctttag gcttgaattg gagtcacttg tatatctcaa ataatgctgg 9300
   ccttttttaa aaagcccttg ttctttatca ccctgttttc tacataattt ttgttcaaag 9360
   aaatacttgt ttggatctcc ttttgacaac aatagcatgt tttcaagcca tatttttttt 9420
   cctttttttt tttttttttg gtttttcgag acagggtttc tctgtatagc cctggctgtc 9480
   ctggaactca ctttgtagac caggctggcc tcgaactcag aaatccgcct gcctctgcct 9540
   cctgagtgcc gggattaaag gcgtgcacca ccacgcctgg ctaagttgga tattttgtta 9600
   tataactata accaatacta actccactgg gtggattttt aattcagtca gtagtcttaa 9660
   gtggtcttta ttggcccttc attaaaatct actgttcact ctaacagagg ctgttggtac 9720
   tagtggcact taagcaactt cctacggata tactagcaga ttaagggtca gggatagaaa 9780
   ctagtctagc gttttgtata cctaccagct ttatactacc ttgttctgat agaaatattt 9840
   caggacatct agcacgtgtt aactcgagct gcaggattcg agggccccgg caggtcaatt 9900
   ctaccgggta ggggaggcgc ttttcccaag gcagtctgga gcatgcgctt tagcagcccc 9960
   gctgggcact tggcgctaca caagtggcct ctggcctcgc acacattcca catccaccgg 10020
   taggcgccaa ccggctccgt tctttggtgg ccccttcgcg ccaccttcta ctcctcccct 10080
   agtcaggaag ttcccccccg ccccgcagct cgcgtcgtgc aggacgtgac aaatggaagt 10140
   agcacgtctc actagtctcg tgcagatgga cagcaccgct gagcaatgga agcgggtagg 10200
   cctttggggc agcggccaat agcagctttg ctccttcgct ttctgggctc agaggctggg 10260
   aaggggtggg tccgggggcg ggctcagggg cgggctcagg ggcggggcgg gcgcccgaag 10320
   gtcctccgga ggcccggcat tctgcacgct tcaaaagcgc acgtctgccg cgctgttctc 10380
   ctcttcctca tctccgggcc tttcgacctg cagccaatgc accgtccttg ccatcatggc 10440
   ctcgtacccc ggccatcaac acgcgtctgc gttcgaccag gctgcgcgtt ctcgcggcca 10500
   tagcaaccga cgtacggcgt tgcgccctcg ccggcagcaa gaagccacgg aagtccgccc 10560
   ggagcagaaa atgcccacgc tactgcgggt ttatatagac ggtccccacg ggatggggaa 10620
   aaccaccacc acgcaactgc tggtggccct gggttcgcgc gacgatatcg tctacgtacc 10680
   cgagccgatg acttactggc gggtgctggg ggcttccgag acaatcgcga acatctacac 10740
   cacacaacac cgcctcgacc agggtgagat atcggccggg gacgcggcgg tggtaatgac 10800
   aagcgcccag ataacaatgg gcatgcctta tgccgtgacc gacgccgttc tggctcctca 10860
   tatcgggggg gaggctggga gctcacatgc cccgcccccg gccctcaccc tcatcttcga 10920
   ccgcccatcc atcgccgccc tcctgtgcta cccggccgcg cggtacctta tgggcagcat 10980
   gaccccccag gccgtgctgg cgttcgtggc cctcatcccg ccgaccttgc ccggcaccaa 11040
   catcgtgctt ggggcccttc cggaggacag acacatcgac cgcctggcca aacgccagcg 11100
   ccccggcgag cggctggacc tggctatgct ggctgcgatt cgccgcgttt acgggctact 11160
   tgccaatacg gtgcggtatc tgcagtgcgg cgggtcgtgg cgggaggact ggggacagct 11220
   ttcggggacg gccgtgccgc cccagggtgc cgagccccag agcaacgcgg gcccacgacc 11280
   ccatatcggg gacacgttat ttaccctgtt tcgggccccc gagttgctgg cccccaacgg 11340
   cgacctgtat aacgtgtttg cctgggcctt ggacgtcttg gccaaacgcc tccgttccat 11400
   gcacgtcttt atcctggatt acgaccaatc gcccgccggc tgccgggacg ccctgctgca 11460
   acttacctcc gggatggtcc agacccacgt caccaccccc ggctccatac cgacgatatg 11520
   cgacctggcg cgcacgtttg cccgggagat gggggaggct aactgagggg atcgatccgt 11580
   cctgtaagtc tgcagaaatt gatgatctat taaacaataa agatgtccac taaaatggaa 11640
   gtttttcctg tcatactttg ttaagaaggg tgagaacaga gtacctacat tttgaatgga 11700
   aggattggag ctacgggggt gggggtgggg tgggattaga taaatgcctg ctctttactg 11760
   aaggctcttt actattgctt tatgataatg tttcatagtt ggatatcata atttaaacaa 11820
   gcaaaaccaa attaagggcc agctcattcc tcccactcat gatctataga tctatagatc 11880
   tctcgtggga tcattgtttt tctcttgatt cccactttgt ggttctaagt actgtggttt 11940
   ccaaatgtgt cagtttcata gcctgaagaa cgagatcagc agcctctgtt ccacatacac 12000
   ttcattctca gtattgtttt gccaagttct aattccatca gaagctgact ctaggccgag 12060
   ctccaattcg ccctatagtg agtcgtatta caattcactg gccgtcgttt tacaacgtcg 12120
   tgactgggaa aaccctggcg ttacccaact taatcgcctt gcagcacatc cccctttcgc 12180
   cagctggcgt aatagcgaag aggcccgcac cgatcgccct tcccaacagt tgcgcagcct 12240
   gaatggcgaa tgggacgcgc cctgtagcgg cgcattaagc gcggcgggtg tggtggttac 12300
   gcgcagcgtg accgctacac ttgccagcgc cctagcgccc gctcctttcg ctttcttccc 12360
   ttcctttctc gccacgttcg ccggctttcc ccgtcaagct ctaaatcggg ggctcccttt 12420
   agggttccga tttagtgctt tacggcacct cgaccccaaa aaacttgatt agggtgatgg 12480
   ttcacgtagt gggccatcgc cctgatagac ggtttttcgc cctttgacgt tggagtccac 12540
   gttctttaat agtggactct tgttccaaac tggaacaaca ctcaacccta tctcggtcta 12600
   ttcttttgat ttataaggga ttttgccgat ttcggcctat tggttaaaaa atgagctgat 12660
   ttaacaaaaa tttaacgcga attttaacaa aatattaacg cttacaattt aggtggcact 12720
   tttcggggaa atgtgcgcgg aacccctatt tgtttatttt tctaaataca ttcaaatatg 12780
   tatccgctca tgagacaata accctgataa atgcttcaat aatattgaaa aaggaagagt 12840
   atgagtattc aacatttccg tgtcgccctt attccctttt ttgcggcatt ttgccttcct 12900
   gtttttgctc acccagaaac gctggtgaaa gtaaaagatg ctgaagatca gttgggtgca 12960
   cgagtgggtt acatcgaact ggatctcaac agcggtaaga tccttgagag ttttcgcccc 13020
   gaagaacgtt ttccaatgat gagcactttt aaagttctgc tatgtggcgc ggtattatcc 13080
   cgtattgacg ccgggcaaga gcaactcggt cgccgcatac actattctca gaatgacttg 13140
   gttgagtact caccagtcac agaaaagcat cttacggatg gcatgacagt aagagaatta 13200
   tgcagtgctg ccataaccat gagtgataac actgcggcca acttacttct gacaacgatc 13260
   ggaggaccga aggagctaac cgcttttttg cacaacatgg gggatcatgt aactcgcctt 13320
   gatcgttggg aaccggagct gaatgaagcc ataccaaacg acgagcgtga caccacgatg 13380
   cctgtagcaa tggcaacaac gttgcgcaaa ctattaactg gcgaactact tactctagct 13440
   tcccggcaac aattaataga ctggatggag gcggataaag ttgcaggacc acttctgcgc 13500
   tcggcccttc cggctggctg gtttattgct gataaatctg gagccggtga gcgtgggtct 13560
   cgcggtatca ttgcagcact ggggccagat ggtaagccct cccgtatcgt agttatctac 13620
   acgacgggga gtcaggcaac tatggatgaa cgaaatagac agatcgctga gataggtgcc 13680
   tcactgatta agcattggta actgtcagac caagtttact catatatact ttagattgat 13740
   ttaaaacttc atttttaatt taaaaggatc taggtgaaga tcctttttga taatctcatg 13800
   accaaaatcc cttaacgtga gttttcgttc cactgagcgt cagaccccgt agaaaagatc 13860
   aaaggatctt cttgagatcc tttttttctg cgcgtaatct gctgcttgca aacaaaaaaa 13920
   ccaccgctac cagcggtggt ttgtttgccg gatcaagagc taccaactct ttttccgaag 13980
   gtaactggct tcagcagagc gcagatacca aatactgtcc ttctagtgta gccgtagtta 14040
   ggccaccact tcaagaactc tgtagcaccg cctacatacc tcgctctgct aatcctgtta 14100
   ccagtggctg ctgccagtgg cgataagtcg tgtcttaccg ggttggactc aagacgatag 14160
   ttaccggata aggcgcagcg gtcgggctga acggggggtt cgtgcacaca gcccagcttg 14220
   gagcgaacga cctacaccga actgagatac ctacagcgtg agctatgaga aagcgccacg 14280
   cttcccgaag ggagaaaggc ggacaggtat ccggtaagcg gcagggtcgg aacaggagag 14340
   cgcacgaggg agcttccagg gggaaacgcc tggtatcttt atagtcctgt cgggtttcgc 14400
   cacctctgac ttgagcgtcg atttttgtga tgctcgtcag gggggcggag cctatggaaa 14460
   aacgccagca acgcggcctt tttacggttc ctggcctttt gctggccttt tgctcacatg 14520
   ttctttcctg cgttatcccc tgattctgtg gataaccgta ttaccgcctt tgagtgagct 14580
   gataccgctc gccgcagccg aacgaccgag cgcagcgagt cagtgagcga ggaagcggaa 14640
   gagcgcccaa tacgcaaacc gcctctcccc gcgcgttggc cgattcatta atgcagctgg 14700
   cacgacaggt ttcccgactg gaaagcgggc agtgagcgca acgcaattaa tgtgagttag 14760
   ctcactcatt aggcacccca ggctttacac tttatgcttc cggctcgtat gttgtgtgga 14820
   attgtgagcg gataacaatt tcacacagga aacagctatg accatgatta cgccaagcgc 14880
   gcaattaacc ctcactaaag ggaacaaaag ctgtcgagat ctagatatcg atggccatag 14940
   agttacg                                                           14947
<210> 5
   <211> 4665
   <212> DNA
   <213> Artificial Sequence
<220>
   <223> Description of Artificial Sequence: Sequence of Rluc-H1-shRNA neo insert
<400> 5
   tctaggtaac cgatatccct gcaggggtga cctgcacgtc tagggcgcag tagtccaggg 60
   tttccttgat gatgtcatac ttatcctgtc cctttttttt ccacagctcg cggttgagga 120
   caaactcttc gcggtctttc cagtactcct gcaggtgact gactgagtcg agatctgcga 180
   tctaagtaag cttggcattc cggtactgtt ggtaaagcca ccatggcttc caaggtgtac 240
   gaccccgagc aacgcaaacg catgatcact gggcctcagt ggtgggctcg ctgcaagcaa 300
   atgaacgtgc tggactcctt catcaactac tatgattccg agaagcacgc cgagaacgcc 360
   gtgatttttc tgcatggtaa cgctgcctcc agctacctgt ggaggcacgt cgtgcctcac 420
   atcgagcccg tggctagatg catcatccct gatctgatcg gaatgggtaa gtccggcaag 480
   agcgggaatg gctcatatcg cctcctggat cactacaagt acctcaccgc ttggttcgag 540
   ctgctgaacc ttccaaagaa aatcatcttt gtgggccacg actggggggc ttgtctggcc 600
   tttcactact cctacgagca ccaagacaag atcaaggcca tcgtccatgc tgagagtgtc 660
   gtggacgtga tcgagtcctg ggacgagtgg cctgacatcg aggaggatat cgccctgatc 720
   aagagcgaag agggcgagaa aatggtgctt gagaataact tcttcgtcga gaccatgctc 780
   ccaagcaaga tcatgcggaa actggagcct gaggagttcg ctgcctacct ggagccattc 840
   aaggagaagg gcgaggttag acggcctacc ctctcctggc ctcgcgagat ccctctcgtt 900
   aagggaggca agcccgacgt cgtccagatt gtccgcaact acaacgccta ccttcgggcc 960
   agcgacgatc tgcctaagat gttcatcgag tccgaccctg ggttcttttc caacgctatt 1020
   gtcgagggag ctaagaagtt ccctaacacc gagttcgtga aggtgaaggg cctccacttc 1080
   agccaggagg acgctccaga tgaaatgggt aagtacatca agagcttcgt ggagcgcgtg 1140
   ctgaagaacg agcagtaatt ctagaccggt tcgagatcca ggcgcggatc aataaaagat 1200
   cattattttc aatagatctg tgtgttggtt ttttgtgtgc cttgggggag ggggaggcca 1260
   gaatgaggcg cggccaaggg ggagggggag gccagaatga ccttggggga gggggaggcc 1320
   agaatgacct tgggggaggg ggaggccaga atgaggcgcg gatccgtcga cttaattaag 1380
   gccagggatc ttcaagcaga cctacagcaa gttcgacaca aactcacaca acgatgacgc 1440
   actactcaag aactacgggc tgctctactg cttcaggaag gacatggaca aggtcgagac 1500
   attcctgcgc atcgtgcagt gccgctctgt ggagggcagc tgtggcttct agctgcccgg 1560
   gtggcatccc tgtgacccct ccccagtgcc tctcctggcc ctggaagttg ccactccagt 1620
   gcccaccagc cttgtcctaa taaaattaag ttgcatcatt ttgtctgact aggtgtcctt 1680
   ctataatatt atggggtgga ggggggtggt atggagcaag gggcaagttg ggaagacaac 1740
   ctgtagggcc tgcggggtct attgggaacc aagctggagt gcagtggcac aatcttggct 1800
   cactgcaatc tccgcctcct gggttcaagc gattctcctg cctcagcctc ccgagttgtt 1860
   gggattccag gcatgcatga ccaggctcag ctaatttttg tttttttggt agagacgggg 1920
   tttcaccata ttggccaggc tggtctccaa ctcctaatct caggtgatct acccaccttg 1980
   gcctcccaaa ttgctgggat tacaggcgtg aaccactgct cccttccctg tccttctgat 2040
   tttaaaataa ctataccagc aggaggacgt ccagacacag cataggctac ctggccatgc 2100
   ccaaccggtg ggacatttga gttgcttgct tggcactgtc ctctcatgcg ttgggtccac 2160
   tcagtagatg cctgttgaat taagcttatt taaataggcc ggccataact tcgtataatg 2220
   tatgctatac gaagttatgg atcctcacag taggtggcat cgttcctttc tgactgcccg 2280
   ccccccgcat gccgtcccgc gatattgagc tccgaacctc tcgccctgcc gccgccggtg 2340
   ctccgtcgcc gccgcgccgc catggaattc gaacgctgac gtcatcaacc cgctccaagg 2400
   aatcgcgggc ccagtgtcac taggcgggaa cacccagcgc gcgtgcgccc tggcaggaag 2460
   atggctgtga gggacagggg agtggcgccc tgcaatattt gcatgtcgct atgtgttctg 2520
   ggaaatcacc ataaacgtga aatgtctttg gatttgggaa tcttataagt tctgtatgag 2580
   accactcttt cccaggattc caattcagcg ggagccacct gatgaagctt gatcgggtgg 2640
   ctctcgctga gttggaatcc atttttttct agactcgaga taacttcgta taatgtatgc 2700
   tatacgaagt tatggcgcgc cggtaaccga agttcctata ctttctagag aataggaact 2760
   tcggaatagg aacttcttag gtcaattcta ccgggtaggg gaggcgcttt tcccaaggca 2820
   gtctggagca tgcgctttag cagccccgct gggcacttgg cgctacacaa gtggcctctg 2880
   gcctcgcaca cattccacat ccaccggtag gcgccaaccg gctccgttct ttggtggccc 2940
   cttcgcgcca ccttctactc ctcccctagt caggaagttc ccccccgccc cgcagctcgc 3000
   gtcgtgcagg acgtgacaaa tggaagtagc acgtctcact agtctcgtgc agatggacag 3060
   caccgctgag caatggaagc gggtaggcct ttggggcagc ggccaatagc agctttgctc 3120
   cttcgctttc tgggctcaga ggctgggaag gggtgggtcc gggggcgggc tcaggggcgg 3180
   gctcaggggc ggggcgggcg cccgaaggtc ctccggaggc ccggcattct gcacgcttca 3240
   aaagcgcacg tctgccgcgc tgttctcctc ttcctcatct ccgggccttt cgacctgcag 3300
   ccaatatggg atcggccatt gaacaagatg gattgcacgc aggttctccg gccgcttggg 3360
   tggagaggct attcggctat gactgggcac aacagacaat cggctgctct gatgccgccg 3420
   tgttccggct gtcagcgcag gggcgcccgg ttctttttgt caagaccgac ctgtccggtg 3480
   ccctgaatga actgcaggac gaggcagcgc ggctatcgtg gctggccacg acgggcgttc 3540
   cttgcgcagc tgtgctcgac gttgtcactg aagcgggaag ggactggctg ctattgggcg 3600
   aagtgccggg gcaggatctc ctgtcatctc accttgctcc tgccgagaaa gtatccatca 3660
   tggctgatgc aatgcggcgg ctgcatacgc ttgatccggc tacctgccca ttcgaccacc 3720
   aagcgaaaca tcgcatcgag cgagcacgta ctcggatgga agccggtctt gtcgatcagg 3780
   atgatctgga cgaagagcat caggggctcg cgccagccga actgttcgcc aggctcaagg 3840
   cgcgcatgcc cgacggcgag gatctcgtcg tgacccatgg cgatgcctgc ttgccgaata 3900
   tcatggtgga aaatggccgc ttttctggat tcatcgactg tggccggctg ggtgtggcgg 3960
   accgctatca ggacatagcg ttggctaccc gtgatattgc tgaagagctt ggcggcgaat 4020
   gggctgaccg cttcctcgtg ctttacggta tcgccgctcc cgattcgcag cgcatcgcct 4080
   tctatcgcct tcttgacgag ttcttctgag gggatcgatc cgctgtaagt ctgcagaaat 4140
   tgatgatcta ttaaacaata aagatgtcca ctaaaatgga agtttttcct gtcatacttt 4200
   gttaagaagg gtgagaacag agtacctaca ttttgaatgg aaggattgga gctacggggg 4260
   tgggggtggg gtgggattag ataaatgcct gctctttact gaaggctctt tactattgct 4320
   ttatgataat gtttcatagt tggatatcat aatttaaaca agcaaaacca aattaagggc 4380
   cagctcattc ctcccactca tgatctatag atctatagat ctctcgtggg atcattgttt 4440
   ttctcttgat tcccactttg tggttctaag tactgtggtt tccaaatgtg tcagtttcat 4500
   agcctgaaga acgagatcag cagcctctgt tccacataca cttcattctc agtattgttt 4560
   tgccaagttc taattccatc agaagctgac tctagatccc gcgccgaagt tcctatactt 4620
   tctagagaat aggaacttcg gaataggaac ttcaagctta agcgc 4665
<210> 6
   <211> 15199
   <212> DNA
   <213> Artificial Sequence
<220>
   <223> Description of Artificial Sequence: Targeting vector for Rosa26 locus with Rluc-H1-shRNA neo insert
<400> 6
   ctagggataa cagggtaata tagccgcggc aggccctccg agcgtggtgg agccgttctg 60
   tgagacagcc gggtacgagt cgtgacgctg gaaggggcaa gcgggtggtg ggcaggaatg 120
   cggtccgccc tgcagcaacc ggagggggag ggagaaggga gcggaaaagt ctccaccgga 180
   cgcggccatg gctcgggggg gggggggcag cggaggagcg cttccggccg acgtctcgtc 240
   gctgattggc ttcttttcct cccgccgtgt gtgaaaacac aaatggcgtg ttttggttgg 300
   cgtaaggcgc ctgtcagtta acggcagccg gagtgcgcag ccgccggcag cctcgctctg 360
   cccactgggt ggggcgggag gtaggtgggg tgaggcgagc tggacgtgcg ggcgcggtcg 420
   gcctctggcg gggcggggga ggggagggag ggtcagcgaa agtagctcgc gcgcgagcgg 480
   ccgcccaccc tccccttcct ctgggggagt cgttttaccc gccgccggcc gggcctcgtc 540
   gtctgattgg ctctcggggc ccagaaaact ggcccttgcc attggctcgt gttcgtgcaa 600
   gttgagtcca tccgccggcc agcgggggcg gcgaggaggc gctcccaggt tccggccctc 660
   ccctcggccc cgcgccgcag agtctggccg cgcgcccctg cgcaacgtgg caggaagcgc 720
   gcgctggggg cggggacggg cagtagggct gagcggctgc ggggcgggtg caagcacgtt 780
   tccgacttga gttgcctcaa gaggggcgtg ctgagccaga cctccatcgc gcactccggg 840
   gagtggaggg aaggagcgag ggctcagttg ggctgttttg gaggcaggaa gcacttgctc 900
   tcccaaagtc gctctgagtt gttatcagta agggagctgc agtggagtag gcggggagaa 960
   ggccgcaccc ttctccggag gggggagggg agtgttgcaa tacctttctg ggagttctct 1020
   gctgcctcct ggcttctgag gaccgccctg ggcctgggag aatcccttcc ccctcttccc 1080
   tcgtgatctg caactccagt ctttctaggt aaccgatatc cctgcagggg tgacctgcac 1140
   gtctagggcg cagtagtcca gggtttcctt gatgatgtca tacttatcct gtcccttttt 1200
   tttccacagc tcgcggttga ggacaaactc ttcgcggtct ttccagtact cctgcaggtg 1260
   actgactgag tcgagatctg cgatctaagt aagcttggca ttccggtact gttggtaaag 1320
   ccaccatggc ttccaaggtg tacgaccccg agcaacgcaa acgcatgatc actgggcctc 1380
   agtggtgggc tcgctgcaag caaatgaacg tgctggactc cttcatcaac tactatgatt 1440
   ccgagaagca cgccgagaac gccgtgattt ttctgcatgg taacgctgcc tccagctacc 1500
   tgtggaggca cgtcgtgcct cacatcgagc ccgtggctag atgcatcatc cctgatctga 1560
   tcggaatggg taagtccggc aagagcggga atggctcata tcgcctcctg gatcactaca 1620
   agtacctcac cgcttggttc gagctgctga accttccaaa gaaaatcatc tttgtgggcc 1680
   acgactgggg ggcttgtctg gcctttcact actcctacga gcaccaagac aagatcaagg 1740
   ccatcgtcca tgctgagagt gtcgtggacg tgatcgagtc ctgggacgag tggcctgaca 1800
   tcgaggagga tatcgccctg atcaagagcg aagagggcga gaaaatggtg cttgagaata 1860
   acttcttcgt cgagaccatg ctcccaagca agatcatgcg gaaactggag cctgaggagt 1920
   tcgctgccta cctggagcca ttcaaggaga agggcgaggt tagacggcct accctctcct 1980
   ggcctcgcga gatccctctc gttaagggag gcaagcccga cgtcgtccag attgtccgca 2040
   actacaacgc ctaccttcgg gccagcgacg atctgcctaa gatgttcatc gagtccgacc 2100
   ctgggttctt ttccaacgct attgtcgagg gagctaagaa gttccctaac accgagttcg 2160
   tgaaggtgaa gggcctccac ttcagccagg aggacgctcc agatgaaatg ggtaagtaca 2220
   tcaagagctt cgtggagcgc gtgctgaaga acgagcagta attctagacc ggttcgagat 2280
   ccaggcgcgg atcaataaaa gatcattatt ttcaatagat ctgtgtgttg gttttttgtg 2340
   tgccttgggg gagggggagg ccagaatgag gcgcggccaa gggggagggg gaggccagaa 2400
   tgaccttggg ggagggggag gccagaatga ccttggggga gggggaggcc agaatgaggc 2460
   gcggatccgt cgacttaatt aaggccaggg atcttcaagc agacctacag caagttcgac 2520
   acaaactcac acaacgatga cgcactactc aagaactacg ggctgctcta ctgcttcagg 2580
   aaggacatgg acaaggtcga gacattcctg cgcatcgtgc agtgccgctc tgtggagggc 2640
   agctgtggct tctagctgcc cgggtggcat ccctgtgacc cctccccagt gcctctcctg 2700
   gccctggaag ttgccactcc agtgcccacc agccttgtcc taataaaatt aagttgcatc 2760
   attttgtctg actaggtgtc cttctataat attatggggt ggaggggggt ggtatggagc 2820
   aaggggcaag ttgggaagac aacctgtagg gcctgcgggg tctattggga accaagctgg 2880
   agtgcagtgg cacaatcttg gctcactgca atctccgcct cctgggttca agcgattctc 2940
   ctgcctcagc ctcccgagtt gttgggattc caggcatgca tgaccaggct cagctaattt 3000
   ttgttttttt ggtagagacg gggtttcacc atattggcca ggctggtctc caactcctaa 3060
   tctcaggtga tctacccacc ttggcctccc aaattgctgg gattacaggc gtgaaccact 3120
   gctcccttcc ctgtccttct gattttaaaa taactatacc agcaggagga cgtccagaca 3180
   cagcataggc tacctggcca tgcccaaccg gtgggacatt tgagttgctt gcttggcact 3240
   gtcctctcat gcgttgggtc cactcagtag atgcctgttg aattaagctt atttaaatag 3300
   gccggccata acttcgtata atgtatgcta tacgaagtta tggatcctca cagtaggtgg 3360
   catcgttcct ttctgactgc ccgccccccg catgccgtcc cgcgatattg agctccgaac 3420
   ctctcgccct gccgccgccg gtgctccgtc gccgccgcgc cgccatggaa ttcgaacgct 3480
   gacgtcatca acccgctcca aggaatcgcg ggcccagtgt cactaggcgg gaacacccag 3540
   cgcgcgtgcg ccctggcagg aagatggctg tgagggacag gggagtggcg ccctgcaata 3600
   tttgcatgtc gctatgtgtt ctgggaaatc accataaacg tgaaatgtct ttggatttgg 3660
   gaatcttata agttctgtat gagaccactc tttcccagga ttccaattca gcgggagcca 3720
   cctgatgaag cttgatcggg tggctctcgc tgagttggaa tccatttttt tctagactcg 3780
   agataacttc gtataatgta tgctatacga agttatggcg cgccggtaac cgaagttcct 3840
   atactttcta gagaatagga acttcggaat aggaacttct taggtcaatt ctaccgggta 3900
   ggggaggcgc ttttcccaag gcagtctgga gcatgcgctt tagcagcccc gctgggcact 3960
   tggcgctaca caagtggcct ctggcctcgc acacattcca catccaccgg taggcgccaa 4020
   ccggctccgt tctttggtgg ccccttcgcg ccaccttcta ctcctcccct agtcaggaag 4080
   ttcccccccg ccccgcagct cgcgtcgtgc aggacgtgac aaatggaagt agcacgtctc 4140
   actagtctcg tgcagatgga cagcaccgct gagcaatgga agcgggtagg cctttggggc 4200
   agcggccaat agcagctttg ctccttcgct ttctgggctc agaggctggg aaggggtggg 4260
   tccgggggcg ggctcagggg cgggctcagg ggcggggcgg gcgcccgaag gtcctccgga 4320
   ggcccggcat tctgcacgct tcaaaagcgc acgtctgccg cgctgttctc ctcttcctca 4380
   tctccgggcc tttcgacctg cagccaatat gggatcggcc attgaacaag atggattgca 4440
   cgcaggttct ccggccgctt gggtggagag gctattcggc tatgactggg cacaacagac 4500
   aatcggctgc tctgatgccg ccgtgttccg gctgtcagcg caggggcgcc cggttctttt 4560
   tgtcaagacc gacctgtccg gtgccctgaa tgaactgcag gacgaggcag cgcggctatc 4620
   gtggctggcc acgacgggcg ttccttgcgc agctgtgctc gacgttgtca ctgaagcggg 4680
   aagggactgg ctgctattgg gcgaagtgcc ggggcaggat ctcctgtcat ctcaccttgc 4740
   tcctgccgag aaagtatcca tcatggctga tgcaatgcgg cggctgcata cgcttgatcc 4800
   ggctacctgc ccattcgacc accaagcgaa acatcgcatc gagcgagcac gtactcggat 4860
   ggaagccggt cttgtcgatc aggatgatct ggacgaagag catcaggggc tcgcgccagc 4920
   cgaactgttc gccaggctca aggcgcgcat gcccgacggc gaggatctcg tcgtgaccca 4980
   tggcgatgcc tgcttgccga atatcatggt ggaaaatggc cgcttttctg gattcatcga 5040
   ctgtggccgg ctgggtgtgg cggaccgcta tcaggacata gcgttggcta cccgtgatat 5100
   tgctgaagag cttggcggcg aatgggctga ccgcttcctc gtgctttacg gtatcgccgc 5160
   tcccgattcg cagcgcatcg ccttctatcg ccttcttgac gagttcttct gaggggatcg 5220
   atccgctgta agtctgcaga aattgatgat ctattaaaca ataaagatgt ccactaaaat 5280
   ggaagttttt cctgtcatac tttgttaaga agggtgagaa cagagtacct acattttgaa 5340
   tggaaggatt ggagctacgg gggtgggggt ggggtgggat tagataaatg cctgctcttt 5400
   actgaaggct ctttactatt gctttatgat aatgtttcat agttggatat cataatttaa 5460
   acaagcaaaa ccaaattaag ggccagctca ttcctcccac tcatgatcta tagatctata 5520
   gatctctcgt gggatcattg tttttctctt gattcccact ttgtggttct aagtactgtg 5580
   gtttccaaat gtgtcagttt catagcctga agaacgagat cagcagcctc tgttccacat 5640
   acacttcatt ctcagtattg ttttgccaag ttctaattcc atcagaagct gactctagat 5700
   cccgcgccga agttcctata ctttctagag aataggaact tcggaatagg aacttcaagc 5760
   ttaagcgcta gaagatgggc gggagtcttc tgggcaggct taaaggctaa cctggtgtgt 5820
   gggcgttgtc ctgcagggga attgaacagg tgtaaaattg gagggacaag acttcccaca 5880
   gattttcggt tttgtcggga agttttttaa taggggcaaa taaggaaaat gggaggatag 5940
   gtagtcatct ggggttttat gcagcaaaac tacaggttat tattgcttgt gatccgcctc 6000
   ggagtatttt ccatcgaggt agattaaaga catgctcacc cgagttttat actctcctgc 6060
   ttgagatcct tactacagta tgaaattaca gtgtcgcgag ttagactatg taagcagaat 6120
   tttaatcatt tttaaagagc ccagtacttc atatccattt ctcccgctcc ttctgcagcc 6180
   ttatcaaaag gtattttaga acactcattt tagccccatt ttcatttatt atactggctt 6240
   atccaacccc tagacagagc attggcattt tccctttcct gatcttagaa gtctgatgac 6300
   tcatgaaacc agacagatta gttacataca ccacaaatcg aggctgtagc tggggcctca 6360
   acactgcagt tcttttataa ctccttagta cactttttgt tgatcctttg ccttgatcct 6420
   taattttcag tgtctatcac ctctcccgtc agtggtgttc cacatttggg cctattctca 6480
   gtccagggag ttttacaaca atagatgtat tgagaatcca acctaaagct taactttcca 6540
   ctcccatgaa tgcctctctc ctttttctcc atttataaac tgagctatta accattaatg 6600
   gttccaggtg gatgtctcct ccccatatta cctgatgtat cttacatatt gccaggctga 6660
   tattttaaga cattaaaagg tatatttcat tattgagcca catggtattg attactgctt 6720
   actaaaattt tgtcattgta cacatctgta aaaggtggtt ccttttggaa tgcaaagttc 6780
   aggtgtttgt tgtctttcct gacctaaggt cttgtgagct tgtatttttt ctatttaagc 6840
   agtgctttct cttggactgg cttgactcat ggcattctac acgttattgc tggtctaaat 6900
   gtgattttgc caagcttctt caggacctat aattttgctt gacttgtagc caaacacaag 6960
   taaaatgatt aagcaacaaa tgtatttgtg aagcttggtt tttaggttgt tgtgttgtgt 7020
   gtgcttgtgc tctataataa tactatccag gggctggaga ggtggctcgg agttcaagag 7080
   cacagactgc tcttccagaa gtcctgagtt caattcccag caaccacatg gtggctcaca 7140
   accatctgta atgggatctg atgccctctt ctggtgtgtc tgaagaccac aagtgtattc 7200
   acattaaata aataaatcct ccttcttctt cttttttttt tttttaaaga gaatactgtc 7260
   tccagtagaa tttactgaag taatgaaata ctttgtgttt gttccaatat ggtagccaat 7320
   aatcaaatta ctctttaagc actggaaatg ttaccaagga actaattttt atttgaagtg 7380
   taactgtgga cagaggagcc ataactgcag acttgtggga tacagaagac caatgcagac 7440
   tttaatgtct tttctcttac actaagcaat aaagaaataa aaattgaact tctagtatcc 7500
   tatttgttta aactgctagc tttacttaac ttttgtgctt catctataca aagctgaaag 7560
   ctaagtctgc agccattact aaacatgaaa gcaagtaatg ataattttgg atttcaaaaa 7620
   tgtagggcca gagtttagcc agccagtggt ggtgcttgcc tttatgcctt taatcccagc 7680
   actctggagg cagagacagg cagatctctg agtttgagcc cagcctggtc tacacatcaa 7740
   gttctatcta ggatagccag gaatacacac agaaaccctg ttggggaggg gggctctgag 7800
   atttcataaa attataattg aagcattccc taatgagcca ctatggatgt ggctaaatcc 7860
   gtctaccttt ctgatgagat ttgggtatta ttttttctgt ctctgctgtt ggttgggtct 7920
   tttgacactg tgggctttct ttaaagcctc cttcctgcca tgtggtctct tgtttgctac 7980
   taacttccca tggcttaaat ggcatggctt tttgccttct aagggcagct gctgagattt 8040
   gcagcctgat ttccagggtg gggttgggaa atctttcaaa cactaaaatt gtcctttaat 8100
   ttttttttta aaaaatgggt tatataataa acctcataaa atagttatga ggagtgaggt 8160
   ggactaatat taaatgagtc cctcccctat aaaagagcta ttaaggcttt ttgtcttata 8220
   cttaactttt tttttaaatg tggtatcttt agaaccaagg gtcttagagt tttagtatac 8280
   agaaactgtt gcatcgctta atcagatttt ctagtttcaa atccagagaa tccaaattct 8340
   tcacagccaa agtcaaatta agaatttctg acttttaatg ttaatttgct tactgtgaat 8400
   ataaaaatga tagcttttcc tgaggcaggg tctcactatg tatctctgcc tgatctgcaa 8460
   caagatatgt agactaaagt tctgcctgct tttgtctcct gaatactaag gttaaaatgt 8520
   agtaatactt ttggaacttg caggtcagat tcttttatag gggacacact aagggagctt 8580
   gggtgatagt tggtaaaatg tgtttcaagt gatgaaaact tgaattatta tcaccgcaac 8640
   ctacttttta aaaaaaaaag ccaggcctgt tagagcatgc ttaagggatc cctaggactt 8700
   gctgagcaca caagagtagt tacttggcag gctcctggtg agagcatatt tcaaaaaaca 8760
   aggcagacaa ccaagaaact acagttaagg ttacctgtct ttaaaccatc tgcatataca 8820
   cagggatatt aaaatattcc aaataatatt tcattcaagt tttcccccat caaattggga 8880
   catggatttc tccggtgaat aggcagagtt ggaaactaaa caaatgttgg ttttgtgatt 8940
   tgtgaaattg ttttcaagtg atagttaaag cccatgagat acagaacaaa gctgctattt 9000
   cgaggtctct tggtttatac tcagaagcac ttctttgggt ttccctgcac tatcctgatc 9060
   atgtgctagg cctaccttag gctgattgtt gttcaaataa acttaagttt cctgtcaggt 9120
   gatgtcatat gatttcatat atcaaggcaa aacatgttat atatgttaaa catttgtact 9180
   taatgtgaaa gttaggtctt tgtgggtttg atttttaatt ttcaaaacct gagctaaata 9240
   agtcattttt acatgtctta catttggtgg aattgtataa ttgtggtttg caggcaagac 9300
   tctctgacct agtaacccta cctatagagc actttgctgg gtcacaagtc taggagtcaa 9360
   gcatttcacc ttgaagttga gacgttttgt tagtgtatac tagtttatat gttggaggac 9420
   atgtttatcc agaagatatt caggactatt tttgactggg ctaaggaatt gattctgatt 9480
   agcactgtta gtgagcattg agtggccttt aggcttgaat tggagtcact tgtatatctc 9540
   aaataatgct ggcctttttt aaaaagccct tgttctttat caccctgttt tctacataat 9600
   ttttgttcaa agaaatactt gtttggatct ccttttgaca acaatagcat gttttcaagc 9660
   catatttttt ttcctttttt tttttttttt tggtttttcg agacagggtt tctctgtata 9720
   gccctggctg tcctggaact cactttgtag accaggctgg cctcgaactc agaaatccgc 9780
   ctgcctctgc ctcctgagtg ccgggattaa aggcgtgcac caccacgcct ggctaagttg 9840
   gatattttgt tatataacta taaccaatac taactccact gggtggattt ttaattcagt 9900
   cagtagtctt aagtggtctt tattggccct tcattaaaat ctactgttca ctctaacaga 9960
   ggctgttggt actagtggca cttaagcaac ttcctacgga tatactagca gattaagggt 10020
   cagggataga aactagtcta gcgttttgta tacctaccag ctttatacta ccttgttctg 10080
   atagaaatat ttcaggacat ctagcacgtg ttaactcgag ctgcaggatt cgagggcccc 10140
   ggcaggtcaa ttctaccggg taggggaggc gcttttccca aggcagtctg gagcatgcgc 10200
   tttagcagcc ccgctgggca cttggcgcta cacaagtggc ctctggcctc gcacacattc 10260
   cacatccacc ggtaggcgcc aaccggctcc gttctttggt ggccccttcg cgccaccttc 10320
   tactcctccc ctagtcagga agttcccccc cgccccgcag ctcgcgtcgt gcaggacgtg 10380
   acaaatggaa gtagcacgtc tcactagtct cgtgcagatg gacagcaccg ctgagcaatg 10440
   gaagcgggta ggcctttggg gcagcggcca atagcagctt tgctccttcg ctttctgggc 10500
   tcagaggctg ggaaggggtg ggtccggggg cgggctcagg ggcgggctca ggggcggggc 10560
   gggcgcccga aggtcctccg gaggcccggc attctgcacg cttcaaaagc gcacgtctgc 10620
   cgcgctgttc tcctcttcct catctccggg cctttcgacc tgcagccaat gcaccgtcct 10680
   tgccatcatg gcctcgtacc ccggccatca acacgcgtct gcgttcgacc aggctgcgcg 10740
   ttctcgcggc catagcaacc gacgtacggc gttgcgccct cgccggcagc aagaagccac 10800
   ggaagtccgc ccggagcaga aaatgcccac gctactgcgg gtttatatag acggtcccca 10860
   cgggatgggg aaaaccacca ccacgcaact gctggtggcc ctgggttcgc gcgacgatat 10920
   cgtctacgta cccgagccga tgacttactg gcgggtgctg ggggcttccg agacaatcgc 10980
   gaacatctac accacacaac accgcctcga ccagggtgag atatcggccg gggacgcggc 11040
   ggtggtaatg acaagcgccc agataacaat gggcatgcct tatgccgtga ccgacgccgt 11100
   tctggctcct catatcgggg gggaggctgg gagctcacat gccccgcccc cggccctcac 11160
   cctcatcttc gaccgccatc ccatcgccgc cctcctgtgc tacccggccg cgcggtacct 11220
   tatgggcagc atgacccccc aggccgtgct ggcgttcgtg gccctcatcc cgccgacctt 11280
   gcccggcacc aacatcgtgc ttggggccct tccggaggac agacacatcg accgcctggc 11340
   caaacgccag cgccccggcg agcggctgga cctggctatg ctggctgcga ttcgccgcgt 11400
   ttacgggcta cttgccaata cggtgcggta tctgcagtgc ggcgggtcgt ggcgggagga 11460
   ctggggacag ctttcgggga cggccgtgcc gccccagggt gccgagcccc agagcaacgc 11520
   gggcccacga ccccatatcg gggacacgtt atttaccctg tttcgggccc ccgagttgct 11580
   ggcccccaac ggcgacctgt ataacgtgtt tgcctgggcc ttggacgtct tggccaaacg 11640
   cctccgttcc atgcacgtct ttatcctgga ttacgaccaa tcgcccgccg gctgccggga 11700
   cgccctgctg caacttacct ccgggatggt ccagacccac gtcaccaccc ccggctccat 11760
   accgacgata tgcgacctgg cgcgcacgtt tgcccgggag atgggggagg ctaactgagg 11820
   ggatcgatcc gtcctgtaag tctgcagaaa ttgatgatct attaaacaat aaagatgtcc 11880
   actaaaatgg aagtttttcc tgtcatactt tgttaagaag ggtgagaaca gagtacctac 11940
   attttgaatg gaaggattgg agctacgggg gtgggggtgg ggtgggatta gataaatgcc 12000
   tgctctttac tgaaggctct ttactattgc tttatgataa tgtttcatag ttggatatca 12060
   taatttaaac aagcaaaacc aaattaaggg ccagctcatt cctcccactc atgatctata 12120
   gatctataga tctctcgtgg gatcattgtt tttctcttga ttcccacttt gtggttctaa 12180
   gtactgtggt ttccaaatgt gtcagtttca tagcctgaag aacgagatca gcagcctctg 12240
   ttccacatac acttcattct cagtattgtt ttgccaagtt ctaattccat cagaagctga 12300
   ctctaggccg agctccaatt cgccctatag tgagtcgtat tacaattcac tggccgtcgt 12360
   tttacaacgt cgtgactggg aaaaccctgg cgttacccaa cttaatcgcc ttgcagcaca 12420
   tccccctttc gccagctggc gtaatagcga agaggcccgc accgatcgcc cttcccaaca 12480
   gttgcgcagc ctgaatggcg aatgggacgc gccctgtagc ggcgcattaa gcgcggcggg 12540
   tgtggtggtt acgcgcagcg tgaccgctac acttgccagc gccctagcgc ccgctccttt 12600
   cgctttcttc ccttcctttc tcgccacgtt cgccggcttt ccccgtcaag ctctaaatcg 12660
   ggggctccct ttagggttcc gatttagtgc tttacggcac ctcgacccca aaaaacttga 12720
   ttagggtgat ggttcacgta gtgggccatc gccctgatag acggtttttc gccctttgac 12780
   gttggagtcc acgttcttta atagtggact cttgttccaa actggaacaa cactcaaccc 12840
   tatctcggtc tattcttttg atttataagg gattttgccg atttcggcct attggttaaa 12900
   aaatgagctg atttaacaaa aatttaacgc gaattttaac aaaatattaa cgcttacaat 12960
   ttaggtggca cttttcgggg aaatgtgcgc ggaaccccta tttgtttatt tttctaaata 13020
   cattcaaata tgtatccgct catgagacaa taaccctgat aaatgcttca ataatattga 13080
   aaaaggaaga gtatgagtat tcaacatttc cgtgtcgccc ttattccctt ttttgcggca 13140
   ttttgccttc ctgtttttgc tcacccagaa acgctggtga aagtaaaaga tgctgaagat 13200
   cagttgggtg cacgagtggg ttacatcgaa ctggatctca acagcggtaa gatccttgag 13260
   agttttcgcc ccgaagaacg ttttccaatg atgagcactt ttaaagttct gctatgtggc 13320
   gcggtattat cccgtattga cgccgggcaa gagcaactcg gtcgccgcat acactattct 13380
   cagaatgact tggttgagta ctcaccagtc acagaaaagc atcttacgga tggcatgaca 13440
   gtaagagaat tatgcagtgc tgccataacc atgagtgata acactgcggc caacttactt 13500
   ctgacaacga tcggaggacc gaaggagcta accgcttttt tgcacaacat gggggatcat 13560
   gtaactcgcc ttgatcgttg ggaaccggag ctgaatgaag ccataccaaa cgacgagcgt 13620
   gacaccacga tgcctgtagc aatggcaaca acgttgcgca aactattaac tggcgaacta 13680
   cttactctag cttcccggca acaattaata gactggatgg aggcggataa agttgcagga 13740
   ccacttctgc gctcggccct tccggctggc tggtttattg ctgataaatc tggagccggt 13800
   gagcgtgggt ctcgcggtat cattgcagca ctggggccag atggtaagcc ctcccgtatc 13860
   gtagttatct acacgacggg gagtcaggca actatggatg aacgaaatag acagatcgct 13920
   gagataggtg cctcactgat taagcattgg taactgtcag accaagttta ctcatatata 13980
   ctttagattg atttaaaact tcatttttaa tttaaaagga tctaggtgaa gatccttttt 14040
   gataatctca tgaccaaaat cccttaacgt gagttttcgt tccactgagc gtcagacccc 14100
   gtagaaaaga tcaaaggatc ttcttgagat cctttttttc tgcgcgtaat ctgctgcttg 14160
   caaacaaaaa aaccaccgct accagcggtg gtttgtttgc cggatcaaga gctaccaact 14220
   ctttttccga aggtaactgg cttcagcaga gcgcagatac caaatactgt ccttctagtg 14280
   tagccgtagt taggccacca cttcaagaac tctgtagcac cgcctacata cctcgctctg 14340
   ctaatcctgt taccagtggc tgctgccagt ggcgataagt cgtgtcttac cgggttggac 14400
   tcaagacgat agttaccgga taaggcgcag cggtcgggct gaacgggggg ttcgtgcaca 14460
   cagcccagct tggagcgaac gacctacacc gaactgagat acctacagcg tgagctatga 14520
   gaaagcgcca cgcttcccga agggagaaag gcggacaggt atccggtaag cggcagggtc 14580
   ggaacaggag agcgcacgag ggagcttcca gggggaaacg cctggtatct ttatagtcct 14640
   gtcgggtttc gccacctctg acttgagcgt cgatttttgt gatgctcgtc aggggggcgg 14700
   agcctatgga aaaacgccag caacgcggcc tttttacggt tcctggcctt ttgctggcct 14760
   tttgctcaca tgttctttcc tgcgttatcc cctgattctg tggataaccg tattaccgcc 14820
   tttgagtgag ctgataccgc tcgccgcagc cgaacgaccg agcgcagcga gtcagtgagc 14880
   gaggaagcgg aagagcgccc aatacgcaaa ccgcctctcc ccgcgcgttg gccgattcat 14940
   taatgcagct ggcacgacag gtttcccgac tggaaagcgg gcagtgagcg caacgcaatt 15000
   aatgtgagtt agctcactca ttaggcaccc caggctttac actttatgct tccggctcgt 15060
   atgttgtgtg gaattgtgag cggataacaa tttcacacag gaaacagcta tgaccatgat 15120
   tacgccaagc gcgcaattaa ccctcactaa agggaacaaa agctgtcgag atctagatat 15180
   cgatggccat agagttacg 15199
<210> 7
   <211> 4640
   <212> DNA
   <213> Artificial Sequence
<220>
   <223> Description of Artificial Sequence: Sequence of Rluc-U6-shRNA neo insert
<400> 7
   tctaggtaac cgatatccct gcaggggtga cctgcacgtc tagggcgcag tagtccaggg 60
   tttccttgat gatgtcatac ttatcctgtc cctttttttt ccacagctcg cggttgagga 120
   caaactcttc gcggtctttc cagtactcct gcaggtgact gactgagtcg agatctgcga 180
   tctaagtaag cttggcattc cggtactgtt ggtaaagcca ccatggcttc caaggtgtac 240
   gaccccgagc aacgcaaacg catgatcact gggcctcagt ggtgggctcg ctgcaagcaa 300
   atgaacgtgc tggactcctt catcaactac tatgattccg agaagcacgc cgagaacgcc 360
   gtgatttttc tgcatggtaa cgctgcctcc agctacctgt ggaggcacgt cgtgcctcac 420
   atcgagcccg tggctagatg catcatccct gatctgatcg gaatgggtaa gtccggcaag 480
   agcgggaatg gctcatatcg cctcctggat cactacaagt acctcaccgc ttggttcgag 540
   ctgctgaacc ttccaaagaa aatcatcttt gtgggccacg actggggggc ttgtctggcc 600
   tttcactact cctacgagca ccaagacaag atcaaggcca tcgtccatgc tgagagtgtc 660
   gtggacgtga tcgagtcctg ggacgagtgg cctgacatcg aggaggatat cgccctgatc 720
   aagagcgaag agggcgagaa aatggtgctt gagaataact tcttcgtcga gaccatgctc 780
   ccaagcaaga tcatgcggaa actggagcct gaggagttcg ctgcctacct ggagccattc 840
   aaggagaagg gcgaggttag acggcctacc ctctcctggc ctcgcgagat ccctctcgtt 900
   aagggaggca agcccgacgt cgtccagatt gtccgcaact acaacgccta ccttcgggcc 960
   agcgacgatc tgcctaagat gttcatcgag tccgaccctg ggttcttttc caacgctatt 1020
   gtcgagggag ctaagaagtt ccctaacacc gagttcgtga aggtgaaggg cctccacttc 1080
   agccaggagg acgctccaga tgaaatgggt aagtacatca agagcttcgt ggagcgcgtg 1140
   ctgaagaacg agcagtaatt ctagaccggt tcgagatcca ggcgcggatc aataaaagat 1200
   cattattttc aatagatctg tgtgttggtt ttttgtgtgc cttgggggag ggggaggcca 1260
   gaatgaggcg cggccaaggg ggagggggag gccagaatga ccttggggga gggggaggcc 1320
   agaatgacct tgggggaggg ggaggccaga atgaggcgcg gatccgtcga cttaattaag 1380
   gccagggatc ttcaagcaga cctacagcaa gttcgacaca aactcacaca acgatgacgc 1440
   actactcaag aactacgggc tgctctactg cttcaggaag gacatggaca aggtcgagac 1500
   attcctgcgc atcgtgcagt gccgctctgt ggagggcagc tgtggcttct agctgcccgg 1560
   gtggcatccc tgtgacccct ccccagtgcc tctcctggcc ctggaagttg ccactccagt 1620
   gcccaccagc cttgtcctaa taaaattaag ttgcatcatt ttgtctgact aggtgtcctt 1680
   ctataatatt atggggtgga ggggggtggt atggagcaag gggcaagttg ggaagacaac 1740
   ctgtagggcc tgcggggtct attgggaacc aagctggagt gcagtggcac aatcttggct 1800
   cactgcaatc tccgcctcct gggttcaagc gattctcctg cctcagcctc ccgagttgtt 1860
   gggattccag gcatgcatga ccaggctcag ctaatttttg tttttttggt agagacgggg 1920
   tttcaccata ttggccaggc tggtctccaa ctcctaatct caggtgatct acccaccttg 1980
   gcctcccaaa ttgctgggat tacaggcgtg aaccactgct cccttccctg tccttctgat 2040
   tttaaaataa ctataccagc aggaggacgt ccagacacag cataggctac ctggccatgc 2100
   ccaaccggtg ggacatttga gttgcttgct tggcactgtc ctctcatgcg ttgggtccac 2160
   tcagtagatg cctgttgaat taagcttatt taaataggcc ggccataact tcgtataatg 2220
   tatgctatac gaagttatgg atccagtgga aagacgcgca ggcaaaacgc accacgtgac 2280
   ggagcgtgac cgcgcgccga gcccaaggtc gggcaggaag agggcctatt tcccatgatt 2340
   ccttcatatt tgcatatacg atacaaggct gttagagaga taattagaat taatttgact 2400
   gtaaacacaa agatattagt acaaaatacg tgacgtagaa agtaataatt tcttgggtag 2460
   tttgcagttt taaaattatg ttttaaaatg gactatcata tgcttaccgt aacttgaaag 2520
   tatttcgatt tcttggcttt atatatcttg tggaaaggac gaaacaccgg gattccaatt 2580
   cagcgggagc cacctgatga agcttgatcg ggtggctctc gctgagttgg aatccatttt 2640
   tttctagact cgagataact tcgtataatg tatgctatac gaagttatgg cgcgccggta 2700
   accgaagttc ctatactttc tagagaatag gaacttcgga ataggaactt cttaggtcaa 2760
   ttctaccggg taggggaggc gcttttccca aggcagtctg gagcatgcgc tttagcagcc 2820
   ccgctgggca cttggcgcta cacaagtggc ctctggcctc gcacacattc cacatccacc 2880
   ggtaggcgcc aaccggctcc gttctttggt ggccccttcg cgccaccttc tactcctccc 2940
   ctagtcagga agttcccccc cgccccgcag ctcgcgtcgt gcaggacgtg acaaatggaa 3000
   gtagcacgtc tcactagtct cgtgcagatg gacagcaccg ctgagcaatg gaagcgggta 3060
   ggcctttggg gcagcggcca atagcagctt tgctccttcg ctttctgggc tcagaggctg 3120
   ggaaggggtg ggtccggggg cgggctcagg ggcgggctca ggggcggggc gggcgcccga 3180
   aggtcctccg gaggcccggc attctgcacg cttcaaaagc gcacgtctgc cgcgctgttc 3240
   tcctcttcct catctccggg cctttcgacc tgcagccaat atgggatcgg ccattgaaca 3300
   agatggattg cacgcaggtt ctccggccgc ttgggtggag aggctattcg gctatgactg 3360
   ggcacaacag acaatcggct gctctgatgc cgccgtgttc cggctgtcag cgcaggggcg 3420
   cccggttctt tttgtcaaga ccgacctgtc cggtgccctg aatgaactgc aggacgaggc 3480
   agcgcggcta tcgtggctgg ccacgacggg cgttccttgc gcagctgtgc tcgacgttgt 3540
   cactgaagcg ggaagggact ggctgctatt gggcgaagtg ccggggcagg atctcctgtc 3600
   atctcacctt gctcctgccg agaaagtatc catcatggct gatgcaatgc ggcggctgca 3660
   tacgcttgat ccggctacct gcccattcga ccaccaagcg aaacatcgca tcgagcgagc 3720
   acgtactcgg atggaagccg gtcttgtcga tcaggatgat ctggacgaag agcatcaggg 3780
   gctcgcgcca gccgaactgt tcgccaggct caaggcgcgc atgcccgacg gcgaggatct 3840
   cgtcgtgacc catggcgatg cctgcttgcc gaatatcatg gtggaaaatg gccgcttttc 3900
   tggattcatc gactgtggcc ggctgggtgt ggcggaccgc tatcaggaca tagcgttggc 3960
   tacccgtgat attgctgaag agcttggcgg cgaatgggct gaccgcttcc tcgtgcttta 4020
   cggtatcgcc gctcccgatt cgcagcgcat cgccttctat cgccttcttg acgagttctt 4080
   ctgaggggat cgatccgctg taagtctgca gaaattgatg atctattaaa caataaagat 4140
   gtccactaaa atggaagttt ttcctgtcat actttgttaa gaagggtgag aacagagtac 4200
   ctacattttg aatggaagga ttggagctac gggggtgggg gtggggtggg attagataaa 4260
   tgcctgctct ttactgaagg ctctttacta ttgctttatg ataatgtttc atagttggat 4320
   atcataattt aaacaagcaa aaccaaatta agggccagct cattcctccc actcatgatc 4380
   tatagatcta tagatctctc gtgggatcat tgtttttctc ttgattccca ctttgtggtt 4440
   ctaagtactg tggtttccaa atgtgtcagt ttcatagcct gaagaacgag atcagcagcc 4500
   tctgttccac atacacttca ttctcagtat tgttttgcca agttctaatt ccatcagaag 4560
   ctgactctag atcccgcgcc gaagttccta tactttctag agaataggaa cttcggaata 4620
   ggaacttcaa gcttaagcgc                                             4640
<210> 8
   <211> 15174
   <212> DNA
   <213> Artificial Sequence
<220>
   <223> Description of Artificial Sequence: Targeting vector for Rosa26 locus with a Rluc-U6-shRNA neo insert
<400> 8
   ctagggataa cagggtaata tagccgcggc aggccctccg agcgtggtgg agccgttctg 60
   tgagacagcc gggtacgagt cgtgacgctg gaaggggcaa gcgggtggtg ggcaggaatg 120
   cggtccgccc tgcagcaacc ggagggggag ggagaaggga gcggaaaagt ctccaccgga 180
   cgcggccatg gctcgggggg gggggggcag cggaggagcg cttccggccg acgtctcgtc 240
   gctgattggc ttcttttcct cccgccgtgt gtgaaaacac aaatggcgtg ttttggttgg 300
   cgtaaggcgc ctgtcagtta acggcagccg gagtgcgcag ccgccggcag cctcgctctg 360
   cccactgggt ggggcgggag gtaggtgggg tgaggcgagc tggacgtgcg ggcgcggtcg 420
   gcctctggcg ggcgggggga ggggagggag ggtcagcgaa agtagctcgc gcgcgagcgg 480
   ccgcccaccc tccccttcct ctgggggagt cgttttaccc gccgccggcc gggcctcgtc 540
   gtctgattgg ctctcggggc ccagaaaact ggcccttgcc attggctcgt gttcgtgcaa 600
   gttgagtcca tccgccggcc agcgggggcg gcgaggaggc gctcccaggt tccggccctc 660
   ccctcggccc cgcgccgcag agtctggccg cgcgcccctg cgcaacgtgg caggaagcgc 720
   gcgctggggg cggggacggg cagtagggct gagcggctgc ggggcgggtg caagcacgtt 780
   tccgacttga gttgcctcaa gaggggcgtg ctgagccaga cctccatcgc gcactccggg 840
   gagtggaggg aaggagcgag ggctcagttg ggctgttttg gaggcaggaa gcacttgctc 900
   tcccaaagtc gctctgagtt gttatcagta agggagctgc agtggagtag gcggggagaa 960
   ggccgcaccc ttctccggag gggggagggg agtgttgcaa tacctttctg ggagttctct 1020
   gctgcctcct ggcttctgag gaccgccctg ggcctgggag aatcccttcc ccctcttccc 1080
   tcgtgatctg caactccagt ctttctaggt aaccgatatc cctgcagggg tgacctgcac 1140
   gtctagggcg cagtagtcca gggtttcctt gatgatgtca tacttatcct gtcccttttt 1200
   tttccacagc tcgcggttga ggacaaactc ttcgcggtct ttccagtact cctgcaggtg 1260
   actgactgag tcgagatctg cgatctaagt aagcttggca ttccggtact gttggtaaag 1320
   ccaccatggc ttccaaggtg tacgaccccg agcaacgcaa acgcatgatc actgggcctc 1380
   agtggtgggc tcgctgcaag caaatgaacg tgctggactc cttcatcaac tactatgatt 1440
   ccgagaagca cgccgagaac gccgtgattt ttctgcatgg taacgctgcc tccagctacc 1500
   tgtggaggca cgtcgtgcct cacatcgagc ccgtggctag atgcatcatc cctgatctga 1560
   tcggaatggg taagtccggc aagagcggga atggctcata tcgcctcctg gatcactaca 1620
   agtacctcac cgcttggttc gagctgctga accttccaaa gaaaatcatc tttgtgggcc 1680
   acgactgggg ggcttgtctg gcctttcact actcctacga gcaccaagac aagatcaagg 1740
   ccatcgtcca tgctgagagt gtcgtggacg tgatcgagtc ctgggacgag tggcctgaca 1800
   tcgaggagga tatcgccctg atcaagagcg aagagggcga gaaaatggtg cttgagaata 1860
   acttcttcgt cgagaccatg ctcccaagca agatcatgcg gaaactggag cctgaggagt 1920
   tcgctgccta cctggagcca ttcaaggaga agggcgaggt tagacggcct accctctcct 1980
   ggcctcgcga gatccctctc gttaagggag gcaagcccga cgtcgtccag attgtccgca 2040
   actacaacgc ctaccttcgg gccagcgacg atctgcctaa gatgttcatc gagtccgacc 2100
   ctgggttctt ttccaacgct attgtcgagg gagctaagaa gttccctaac accgagttcg 2160
   tgaaggtgaa gggcctccac ttcagccagg aggacgctcc agatgaaatg ggtaagtaca 2220
   tcaagagctt cgtggagcgc gtgctgaaga acgagcagta attctagacc ggttcgagat 2280
   ccaggcgcgg atcaataaaa gatcattatt ttcaatagat ctgtgtgttg gttttttgtg 2340
   tgccttgggg gagggggagg ccagaatgag gcgcggccaa gggggagggg gaggccagaa 2400
   tgaccttggg ggagggggag gccagaatga ccttggggga gggggaggcc agaatgaggc 2460
   gcggatccgt cgacttaatt aaggccaggg atcttcaagc agacctacag caagttcgac 2520
   acaaactcac acaacgatga cgcactactc aagaactacg ggctgctcta ctgcttcagg 2580
   aaggacatgg acaaggtcga gacattcctg cgcatcgtgc agtgccgctc tgtggagggc 2640
   agctgtggct tctagctgcc cgggtggcat ccctgtgacc cctccccagt gcctctcctg 2700
   gccctggaag ttgccactcc agtgcccacc agccttgtcc taataaaatt aagttgcatc 2760
   attttgtctg actaggtgtc cttctataat attatggggt ggaggggggt ggtatggagc 2820
   aaggggcaag ttgggaagac aacctgtagg gcctgcgggg tctattggga accaagctgg 2880
   agtgcagtgg cacaatcttg gctcactgca atctccgcct cctgggttca agcgattctc 2940
   ctgcctcagc ctcccgagtt gttgggattc caggcatgca tgaccaggct cagctaattt 3000
   ttgttttttt ggtagagacg gggtttcacc atattggcca ggctggtctc caactcctaa 3060
   tctcaggtga tctacccacc ttggcctccc aaattgctgg gattacaggc gtgaaccact 3120
   gctcccttcc ctgtccttct gattttaaaa taactatacc agcaggagga cgtccagaca 3180
   cagcataggc tacctggcca tgcccaaccg gtgggacatt tgagttgctt gcttggcact 3240
   gtcctctcat gcgttgggtc cactcagtag atgcctgttg aattaagctt atttaaatag 3300
   gccggccata acttcgtata atgtatgcta tacgaagtta tggatccagt ggaaagacgc 3360
   gcaggcaaaa cgcaccacgt gacggagcgt gaccgcgcgc cgagcccaag gtcgggcagg 3420
   aagagggcct atttcccatg attccttcat atttgcatat acgatacaag gctgttagag 3480
   agataattag aattaatttg actgtaaaca caaagatatt agtacaaaat acgtgacgta 3540
   gaaagtaata atttcttggg tagtttgcag ttttaaaatt atgttttaaa atggactatc 3600
   atatgcttac cgtaacttga aagtatttcg atttcttggc tttatatatc ttgtggaaag 3660
   gacgaaacac cgggattcca attcagcggg agccacctga tgaagcttga tcgggtggct 3720
   ctcgctgagt tggaatccat ttttttctag actcgagata acttcgtata atgtatgcta 3780
   tacgaagtta tggcgcgccg gtaaccgaag ttcctatact ttctagagaa taggaacttc 3840
   ggaataggaa cttcttaggt caattctacc gggtagggga ggcgcttttc ccaaggcagt 3900
   ctggagcatg cgctttagca gccccgctgg gcacttggcg ctacacaagt ggcctctggc 3960
   ctcgcacaca ttccacatcc accggtaggc gccaaccggc tccgttcttt ggtggcccct 4020
   tcgcgccacc ttctactcct cccctagtca ggaagttccc ccccgccccg cagctcgcgt 4080
   cgtgcaggac gtgacaaatg gaagtagcac gtctcactag tctcgtgcag atggacagca 4140
   ccgctgagca atggaagcgg gtaggccttt ggggcagcgg ccaatagcag ctttgctcct 4200
   tcgctttctg ggctcagagg ctgggaaggg gtgggtccgg gggcgggctc aggggcgggc 4260
   tcaggggcgg ggcgggcgcc cgaaggtcct ccggaggccc ggcattctgc acgcttcaaa 4320
   agcgcacgtc tgccgcgctg ttctcctctt cctcatctcc gggcctttcg acctgcagcc 4380
   aatatgggat cggccattga acaagatgga ttgcacgcag gttctccggc cgcttgggtg 4440
   gagaggctat tcggctatga ctgggcacaa cagacaatcg gctgctctga tgccgccgtg 4500
   ttccggctgt cagcgcaggg gcgcccggtt ctttttgtca agaccgacct gtccggtgcc 4560
   ctgaatgaac tgcaggacga ggcagcgcgg ctatcgtggc tggccacgac gggcgttcct 4620
   tgcgcagctg tgctcgacgt tgtcactgaa gcgggaaggg actggctgct attgggcgaa 4680
   gtgccggggc aggatctcct gtcatctcac cttgctcctg ccgagaaagt atccatcatg 4740
   gctgatgcaa tgcggcggct gcatacgctt gatccggcta cctgcccatt cgaccaccaa 4800
   gcgaaacatc gcatcgagcg agcacgtact cggatggaag ccggtcttgt cgatcaggat 4860
   gatctggacg aagagcatca ggggctcgcg ccagccgaac tgttcgccag gctcaaggcg 4920
   cgcatgcccg acggcgagga tctcgtcgtg acccatggcg atgcctgctt gccgaatatc 4980
   atggtggaaa atggccgctt ttctggattc atcgactgtg gccggctggg tgtggcggac 5040
   cgctatcagg acatagcgtt ggctacccgt gatattgctg aagagcttgg cggcgaatgg 5100
   gctgaccgct tcctcgtgct ttacggtatc gccgctcccg attcgcagcg catcgccttc 5160
   tatcgccttc ttgacgagtt cttctgaggg gatcgatccg ctgtaagtct gcagaaattg 5220
   atgatctatt aaacaataaa gatgtccact aaaatggaag tttttcctgt catactttgt 5280
   taagaagggt gagaacagag tacctacatt ttgaatggaa ggattggagc tacgggggtg 5340
   ggggtggggt gggattagat aaatgcctgc tctttactga aggctcttta ctattgcttt 5400
   atgataatgt ttcatagttg gatatcataa tttaaacaag caaaaccaaa ttaagggcca 5460
   gctcattcct cccactcatg atctatagat ctatagatct ctcgtgggat cattgttttt 5520
   ctcttgattc ccactttgtg gttctaagta ctgtggtttc caaatgtgtc agtttcatag 5580
   cctgaagaac gagatcagca gcctctgttc cacatacact tcattctcag tattgttttg 5640
   ccaagttcta attccatcag aagctgactc tagatcccgc gccgaagttc ctatactttc 5700
   tagagaatag gaacttcgga ataggaactt caagcttaag cgctagaaga tgggcgggag 5760
   tcttctgggc aggcttaaag gctaacctgg tgtgtgggcg ttgtcctgca ggggaattga 5820
   acaggtgtaa aattggaggg acaagacttc ccacagattt tcggttttgt cgggaagttt 5880
   tttaataggg gcaaataagg aaaatgggag gataggtagt catctggggt tttatgcagc 5940
   aaaactacag gttattattg cttgtgatcc gcctcggagt attttccatc gaggtagatt 6000
   aaagacatgc tcacccgagt tttatactct cctgcttgag atccttacta cagtatgaaa 6060
   ttacagtgtc gcgagttaga ctatgtaagc agaattttaa tcatttttaa agagcccagt 6120
   acttcatatc catttctccc gctccttctg cagccttatc aaaaggtatt ttagaacact 6180
   cattttagcc ccattttcat ttattatact ggcttatcca acccctagac agagcattgg 6240
   cattttccct ttcctgatct tagaagtctg atgactcatg aaaccagaca gattagttac 6300
   atacaccaca aatcgaggct gtagctgggg cctcaacact gcagttcttt tataactcct 6360
   tagtacactt tttgttgatc ctttgccttg atccttaatt ttcagtgtct atcacctctc 6420
   ccgtcagtgg tgttccacat ttgggcctat tctcagtcca gggagtttta caacaataga 6480
   tgtattgaga atccaaccta aagcttaact ttccactccc atgaatgcct ctctcctttt 6540
   tctccattta taaactgagc tattaaccat taatggttcc aggtggatgt ctcctcccca 6600
   tattacctga tgtatcttac atattgccag gctgatattt taagacatta aaaggtatat 6660
   ttcattattg agccacatgg tattgattac tgcttactaa aattttgtca ttgtacacat 6720
   ctgtaaaagg tggttccttt tggaatgcaa agttcaggtg tttgttgtct ttcctgacct 6780
   aaggtcttgt gagcttgtat tttttctatt taagcagtgc tttctcttgg actggcttga 6840
   ctcatggcat tctacacgtt attgctggtc taaatgtgat tttgccaagc ttcttcagga 6900
   cctataattt tgcttgactt gtagccaaac acaagtaaaa tgattaagca acaaatgtat 6960
   ttgtgaagct tggtttttag gttgttgtgt tgtgtgtgct tgtgctctat aataatacta 7020
   tccaggggct ggagaggtgg ctcggagttc aagagcacag actgctcttc cagaagtcct 7080
   gagttcaatt cccagcaacc acatggtggc tcacaaccat ctgtaatggg atctgatgcc 7140
   ctcttctggt gtgtctgaag accacaagtg tattcacatt aaataaataa atcctccttc 7200
   ttcttctttt tttttttttt aaagagaata ctgtctccag tagaatttac tgaagtaatg 7260
   aaatactttg tgtttgttcc aatatggtag ccaataatca aattactctt taagcactgg 7320
   aaatgttacc aaggaactaa tttttatttg aagtgtaact gtggacagag gagccataac 7380
   tgcagacttg tgggatacag aagaccaatg cagactttaa tgtcttttct cttacactaa 7440
   gcaataaaga aataaaaatt gaacttctag tatcctattt gtttaaactg ctagctttac 7500
   ttaacttttg tgcttcatct atacaaagct gaaagctaag tctgcagcca ttactaaaca 7560
   tgaaagcaag taatgataat tttggatttc aaaaatgtag ggccagagtt tagccagcca 7620
   gtggtggtgc ttgcctttat gcctttaatc ccagcactct ggaggcagag acaggcagat 7680
   ctctgagttt gagcccagcc tggtctacac atcaagttct atctaggata gccaggaata 7740
   cacacagaaa ccctgttggg gaggggggct ctgagatttc ataaaattat aattgaagca 7800
   ttccctaatg agccactatg gatgtggcta aatccgtcta cctttctgat gagatttggg 7860
   tattattttt tctgtctctg ctgttggttg ggtcttttga cactgtgggc tttctttaaa 7920
   gcctccttcc tgccatgtgg tctcttgttt gctactaact tcccatggct taaatggcat 7980
   ggctttttgc cttctaaggg cagctgctga gatttgcagc ctgatttcca gggtggggtt 8040
   gggaaatctt tcaaacacta aaattgtcct ttaatttttt ttttaaaaaa tgggttatat 8100
   aataaacctc ataaaatagt tatgaggagt gaggtggact aatattaaat gagtccctcc 8160
   cctataaaag agctattaag gctttttgtc ttatacttaa cttttttttt aaatgtggta 8220
   tctttagaac caagggtctt agagttttag tatacagaaa ctgttgcatc gcttaatcag 8280
   attttctagt ttcaaatcca gagaatccaa attcttcaca gccaaagtca aattaagaat 8340
   ttctgacttt taatgttaat ttgcttactg tgaatataaa aatgatagct tttcctgagg 8400
   cagggtctca ctatgtatct ctgcctgatc tgcaacaaga tatgtagact aaagttctgc 8460
   ctgcttttgt ctcctgaata ctaaggttaa aatgtagtaa tacttttgga acttgcaggt 8520
   cagattcttt tataggggac acactaaggg agcttgggtg atagttggta aaatgtgttt 8580
   caagtgatga aaacttgaat tattatcacc gcaacctact ttttaaaaaa aaaagccagg 8640
   cctgttagag catgcttaag ggatccctag gacttgctga gcacacaaga gtagttactt 8700
   ggcaggctcc tggtgagagc atatttcaaa aaacaaggca gacaaccaag aaactacagt 8760
   taaggttacc tgtctttaaa ccatctgcat atacacaggg atattaaaat attccaaata 8820
   atatttcatt caagttttcc cccatcaaat tgggacatgg atttctccgg tgaataggca 8880
   gagttggaaa ctaaacaaat gttggttttg tgatttgtga aattgttttc aagtgatagt 8940
   taaagcccat gagatacaga acaaagctgc tatttcgagg tctcttggtt tatactcaga 9000
   agcacttctt tgggtttccc tgcactatcc tgatcatgtg ctaggcctac cttaggctga 9060
   ttgttgttca aataaactta agtttcctgt caggtgatgt catatgattt catatatcaa 9120
   ggcaaaacat gttatatatg ttaaacattt gtacttaatg tgaaagttag gtctttgtgg 9180
   gtttgatttt taattttcaa aacctgagct aaataagtca tttttacatg tcttacattt 9240
   ggtggaattg tataattgtg gtttgcaggc aagactctct gacctagtaa ccctacctat 9300
   agagcacttt gctgggtcac aagtctagga gtcaagcatt tcaccttgaa gttgagacgt 9360
   tttgttagtg tatactagtt tatatgttgg aggacatgtt tatccagaag atattcagga 9420
   ctatttttga ctgggctaag gaattgattc tgattagcac tgttagtgag cattgagtgg 9480
   cctttaggct tgaattggag tcacttgtat atctcaaata atgctggcct tttttaaaaa 9540
   gcccttgttc tttatcaccc tgttttctac ataatttttg ttcaaagaaa tacttgtttg 9600
   gatctccttt tgacaacaat agcatgtttt caagccatat tttttttcct tttttttttt 9660
   ttttttggtt tttcgagaca gggtttctct gtatagccct ggctgtcctg gaactcactt 9720
   tgtagaccag gctggcctcg aactcagaaa tccgcctgcc tctgcctcct gagtgccggg 9780
   attaaaggcg tgcaccacca cgcctggcta agttggatat tttgttatat aactataacc 9840
   aatactaact ccactgggtg gatttttaat tcagtcagta gtcttaagtg gtctttattg 9900
   gcccttcatt aaaatctact gttcactcta acagaggctg ttggtactag tggcacttaa 9960
   gcaacttcct acggatatac tagcagatta agggtcaggg atagaaacta gtctagcgtt 10020
   ttgtatacct accagcttta tactaccttg ttctgataga aatatttcag gacatctagc 10080
   acgtgttaac tcgagctgca ggattcgagg gccccggcag gtcaattcta ccgggtaggg 10140
   gaggcgcttt tcccaaggca gtctggagca tgcgctttag cagccccgct gggcacttgg 10200
   cgctacacaa gtggcctctg gcctcgcaca cattccacat ccaccggtag gcgccaaccg 10260
   gctccgttct ttggtggccc cttcgcgcca ccttctactc ctcccctagt caggaagttc 10320
   ccccccgccc cgcagctcgc gtcgtgcagg acgtgacaaa tggaagtagc acgtctcact 10380
   agtctcgtgc agatggacag caccgctgag caatggaagc gggtaggcct ttggggcagc 10440
   ggccaatagc agctttgctc cttcgctttc tgggctcaga ggctgggaag gggtgggtcc 10500
   gggggcgggc tcaggggcgg gctcaggggc ggggcgggcg cccgaaggtc ctccggaggc 10560
   ccggcattct gcacgcttca aaagcgcacg tctgccgcgc tgttctcctc ttcctcatct 10620
   ccgggccttt cgacctgcag ccaatgcacc gtccttgcca tcatggcctc gtaccccggc 10680
   catcaacacg cgtctgcgtt cgaccaggct gcgcgttctc gcggccatag caaccgacgt 10740
   acggcgttgc gccctcgccg gcagcaagaa gccacggaag tccgcccgga gcagaaaatg 10800
   cccacgctac tgcgggttta tatagacggt ccccacggga tggggaaaac caccaccacg 10860
   caactgctgg tggccctggg ttcgcgcgac gatatcgtct acgtacccga gccgatgact 10920
   tactggcggg tgctgggggc ttccgagaca atcgcgaaca tctacaccac acaacaccgc 10980
   ctcgaccagg gtgagatatc ggccggggac gcggcggtgg taatgacaag cgcccagata 11040
   acaatgggca tgccttatgc cgtgaccgac gccgttctgg ctcctcatat cgggggggag 11100
   gctgggagct cacatgcccc gcccccggcc ctcaccctca tcttcgaccg ccatcccatc 11160
   gccgccctcc tgtgctaccc ggccgcgcgg taccttatgg gcagcatgac cccccaggcc 11220
   gtgctggcgt tcgtggccct catcccgccg accttgcccg gcaccaacat cgtgcttggg 11280
   gcccttccgg aggacagaca catcgaccgc ctggccaaac gccagcgccc cggcgagcgg 11340
   ctggacctgg ctatgctggc tgcgattcgc cgcgtttacg ggctacttgc caatacggtg 11400
   cggtatctgc agtgcggcgg gtcgtggcgg gaggactggg gacagctttc ggggacggcc 11460
   gtgccgcccc agggtgccga gccccagagc aacgcgggcc cacgacccca tatcggggac 11520
   acgttattta ccctgtttcg ggcccccgag ttgctggccc ccaacggcga cctgtataac 11580
   gtgtttgcct gggccttgga cgtcttggcc aaacgcctcc gttccatgca cgtctttatc 11640
   ctggattacg accaatcgcc cgccggctgc cgggacgccc tgctgcaact tacctccggg 11700
   atggtccaga cccacgtcac cacccccggc tccataccga cgatatgcga cctggcgcgc 11760
   acgtttgccc gggagatggg ggaggctaac tgaggggatc gatccgtcct gtaagtctgc 11820
   agaaattgat gatctattaa acaataaaga tgtccactaa aatggaagtt tttcctgtca 11880
   tactttgtta agaagggtga gaacagagta cctacatttt gaatggaagg attggagcta 11940
   cgggggtggg ggtggggtgg gattagataa atgcctgctc tttactgaag gctctttact 12000
   attgctttat gataatgttt catagttgga tatcataatt taaacaagca aaaccaaatt 12060
   aagggccagc tcattcctcc cactcatgat ctatagatct atagatctct cgtgggatca 12120
   ttgtttttct cttgattccc actttgtggt tctaagtact gtggtttcca aatgtgtcag 12180
   tttcatagcc tgaagaacga gatcagcagc ctctgttcca catacacttc attctcagta 12240
   ttgttttgcc aagttctaat tccatcagaa gctgactcta ggccgagctc caattcgccc 12300
   tatagtgagt cgtattacaa ttcactggcc gtcgttttac aacgtcgtga ctgggaaaac 12360
   cctggcgtta cccaacttaa tcgccttgca gcacatcccc ctttcgccag ctggcgtaat 12420
   agcgaagagg cccgcaccga tcgcccttcc caacagttgc gcagcctgaa tggcgaatgg 12480
   gacgcgccct gtagcggcgc attaagcgcg gcgggtgtgg tggttacgcg cagcgtgacc 12540
   gctacacttg ccagcgccct agcgcccgct cctttcgctt tcttcccttc ctttctcgcc 12600
   acgttcgccg gctttccccg tcaagctcta aatcgggggc tccctttagg gttccgattt 12660
   agtgctttac ggcacctcga ccccaaaaaa cttgattagg gtgatggttc acgtagtggg 12720
   ccatcgccct gatagacggt ttttcgccct ttgacgttgg agtccacgtt ctttaatagt 12780
   ggactcttgt tccaaactgg aacaacactc aaccctatct cggtctattc ttttgattta 12840
   taagggattt tgccgatttc ggcctattgg ttaaaaaatg agctgattta acaaaaattt 12900
   aacgcgaatt ttaacaaaat attaacgctt acaatttagg tggcactttt cggggaaatg 12960
   tgcgcggaac ccctatttgt ttatttttct aaatacattc aaatatgtat ccgctcatga 13020
   gacaataacc ctgataaatg cttcaataat attgaaaaag gaagagtatg agtattcaac 13080
   atttccgtgt cgcccttatt cccttttttg cggcattttg ccttcctgtt tttgctcacc 13140
   cagaaacgct ggtgaaagta aaagatgctg aagatcagtt gggtgcacga gtgggttaca 13200
   tcgaactgga tctcaacagc ggtaagatcc ttgagagttt tcgccccgaa gaacgttttc 13260
   caatgatgag cacttttaaa gttctgctat gtggcgcggt attatcccgt attgacgccg 13320
   ggcaagagca actcggtcgc cgcatacact attctcagaa tgacttggtt gagtactcac 13380
   cagtcacaga aaagcatctt acggatggca tgacagtaag agaattatgc agtgctgcca 13440
   taaccatgag tgataacact gcggccaact tacttctgac aacgatcgga ggaccgaagg 13500
   agctaaccgc ttttttgcac aacatggggg atcatgtaac tcgccttgat cgttgggaac 13560
   cggagctgaa tgaagccata ccaaacgacg agcgtgacac cacgatgcct gtagcaatgg 13620
   caacaacgtt gcgcaaacta ttaactggcg aactacttac tctagcttcc cggcaacaat 13680
   taatagactg gatggaggcg gataaagttg caggaccact tctgcgctcg gcccttccgg 13740
   ctggctggtt tattgctgat aaatctggag ccggtgagcg tgggtctcgc ggtatcattg 13800
   cagcactggg gccagatggt aagccctccc gtatcgtagt tatctacacg acggggagtc 13860
   aggcaactat ggatgaacga aatagacaga tcgctgagat aggtgcctca ctgattaagc 13920
   attggtaact gtcagaccaa gtttactcat atatacttta gattgattta aaacttcatt 13980
   tttaatttaa aaggatctag gtgaagatcc tttttgataa tctcatgacc aaaatccctt 14040
   aacgtgagtt ttcgttccac tgagcgtcag accccgtaga aaagatcaaa ggatcttctt 14100
   gagatccttt ttttctgcgc gtaatctgct gcttgcaaac aaaaaaacca ccgctaccag 14160
   cggtggtttg tttgccggat caagagctac caactctttt tccgaaggta actggcttca 14220
   gcagagcgca gataccaaat actgtccttc tagtgtagcc gtagttaggc caccacttca 14280
   agaactctgt agcaccgcct acatacctcg ctctgctaat cctgttacca gtggctgctg 14340
   ccagtggcga taagtcgtgt cttaccgggt tggactcaag acgatagtta ccggataagg 14400
   cgcagcggtc gggctgaacg gggggttcgt gcacacagcc cagcttggag cgaacgacct 14460
   acaccgaact gagataccta cagcgtgagc tatgagaaag cgccacgctt cccgaaggga 14520
   gaaaggcgga caggtatccg gtaagcggca gggtcggaac aggagagcgc acgagggagc 14580
   ttccaggggg aaacgcctgg tatctttata gtcctgtcgg gtttcgccac ctctgacttg 14640
   agcgtcgatt tttgtgatgc tcgtcagggg ggcggagcct atggaaaaac gccagcaacg 14700
   cggccttttt acggttcctg gccttttgct ggccttttgc tcacatgttc tttcctgcgt 14760
   tatcccctga ttctgtggat aaccgtatta ccgcctttga gtgagctgat accgctcgcc 14820
   gcagccgaac gaccgagcgc agcgagtcag tgagcgagga agcggaagag cgcccaatac 14880
   gcaaaccgcc tctccccgcg cgttggccga ttcattaatg cagctggcac gacaggtttc 14940
   ccgactggaa agcgggcagt gagcgcaacg caattaatgt gagttagctc actcattagg 15000
   caccccaggc tttacacttt atgcttccgg ctcgtatgtt gtgtggaatt gtgagcggat 15060
   aacaatttca cacaggaaac agctatgacc atgattacgc caagcgcgca attaaccctc 15120
   actaaaggga acaaaagctg tcgagatcta gatatcgatg gccatagagt tacg 15174
<210> 9
   <211> 4641
   <212> DNA
   <213> Artificial Sequence
<220>
   <223> Description of Artificial Sequence: Rluc tet01 insert
<400> 9
   tctaggtaac cgatatccct gcaggggtga cctgcacgtc tagggcgcag tagtccaggg 60
   tttccttgat gatgtcatac ttatcctgtc cctttttttt ccacagctcg cggttgagga 120
   caaactcttc gcggtctttc cagtactcct gcaggtgact gactgagtcg agatctgcga 180
   tctaagtaag cttggcattc cggtactgtt ggtaaagcca ccatggcttc caaggtgtac 240
   gaccccgagc aacgcaaacg catgatcact gggcctcagt ggtgggctcg ctgcaagcaa 300
   atgaacgtgc tggactcctt catcaactac tatgattccg agaagcacgc cgagaacgcc 360
   gtgatttttc tgcatggtaa cgctgcctcc agctacctgt ggaggcacgt cgtgcctcac 420
   atcgagcccg tggctagatg catcatccct gatctgatcg gaatgggtaa gtccggcaag 480
   agcgggaatg gctcatatcg cctcctggat cactacaagt acctcaccgc ttggttcgag 540
   ctgctgaacc ttccaaagaa aatcatcttt gtgggccacg actggggggc ttgtctggcc 600
   tttcactact cctacgagca ccaagacaag atcaaggcca tcgtccatgc tgagagtgtc 660
   gtggacgtga tcgagtcctg ggacgagtgg cctgacatcg aggaggatat cgccctgatc 720
   aagagcgaag agggcgagaa aatggtgctt gagaataact tcttcgtcga gaccatgctc 780
   ccaagcaaga tcatgcggaa actggagcct gaggagttcg ctgcctacct ggagccattc 840
   aaggagaagg gcgaggttag acggcctacc ctctcctggc ctcgcgagat ccctctcgtt 900
   aagggaggca agcccgacgt cgtccagatt gtccgcaact acaacgccta ccttcgggcc 960
   agcgacgatc tgcctaagat gttcatcgag tccgaccctg ggttcttttc caacgctatt 1020
   gtcgagggag ctaagaagtt ccctaacacc gagttcgtga aggtgaaggg cctccacttc 1080
   agccaggagg acgctccaga tgaaatgggt aagtacatca agagcttcgt ggagcgcgtg 1140
   ctgaagaacg agcagtaatt ctagaccggt tcgagatcca ggcgcggatc aataaaagat 1200
   cattattttc aatagatctg tgtgttggtt ttttgtgtgc cttgggggag gggaggccag 1260
   gaatgaggcg cggccaaggg ggagggggag gccagaatga ccttggggga gggggaggcc 1320
   agaatgacct tgggggaggg ggaggccaga atgaggcgcg gatccgtcga cttaattaag 1380
   gccagggatc ttcaagcaga cctacagcaa gttcgacaca aactcacaca acgatgacgc 1440
   actactcaag aactacgggc tgctctactg cttcaggaag gacatggaca aggtcgagac 1500
   attcctgcgc atcgtgcagt gccgctctgt ggagggcagc tgtggcttct agctgcccgg 1560
   gtggcatccc tgtgacccct ccccagtgcc tctcctggcc ctggaagttg ccactccagt 1620
   gcccaccagc cttgtcctaa taaaattaag ttgcatcatt ttgtctgact aggtgtcctt 1680
   ctataatatt atggggtgga ggggggtggt atggagcaag gggcaagttg ggaagacaac 1740
   ctgtagggcc tgcggggtct attgggaacc aagctggagt gcagtggcac aatcttggct 1800
   cactgcaatc tccgcctcct gggttcaagc gattctcctg cctcagcctc ccgagttgtt 1860
   gggattccag gcatgcatga ccaggctcag ctaatttttg tttttttggt agagacgggg 1920
   tttcaccata ttggccaggc tggtctccaa ctcctaatct caggtgatct acccaccttg 1980
   gcctcccaaa ttgctgggat tacaggcgtg aaccactgct cccttccctg tccttctgat 2040
   tttaaaataa ctataccagc aggaggacgt ccagacacag cataggctac ctggccatgc 2100
   ccaaccggtg ggacatttga gttgcttgct tggcactgtc ctctcatgcg ttgggtccac 2160
   tcagtagatg cctgttgaat taagcttatt taaataggcc ggccataact tcgtataatg 2220
   tatgctatac gaagttatgg atccagtgga aagacgcgca ggcaaaacgc accacgtgac 2280
   ggagcgtgac cgcgcgccga gcccaaggtc gggcaggaag agggcctatt tcccatgatt 2340
   ccttcatatt tgcatatacg atacaaggct gttagagaga taattagaat taatttgact 2400
   gtaaacacaa agatattagt acaaaatacg tgacgtagaa agtaataatt tcttgggtag 2460
   tttgcagttt taaaattatg ttttaaaatg gactatcata tgcttaccgt aacttgaaag 2520
   tactctatca ttgatagagt tatatatctt gtggaaagga cgaaacaccg ggattccaat 2580
   tcagcgggag ccacctgatg aagcttgatc gggtggctct cgctgagttg gaatccattt 2640
   ttttctagac tcgagataac ttcgtataat gtatgctata cgaagttatg gcgcgccggt 2700
   aaccgaagtt cctatacttt ctagagaata ggaacttcgg aataggaact tcttaggtca 2760
   attctaccgg gtaggggagg cgcttttccc aaggcagtct ggagcatgcg ctttagcagc 2820
   cccgctgggc acttggcgct acacaagtgg cctctggcct cgcacacatt ccacatccac 2880
   cggtaggcgc caaccggctc cgttctttgg tggccccttc gcgccacctt ctactcctcc 2940
   cctagtcagg aagttccccc ccgccccgca gctcgcgtcg tgcaggacgt gacaaatgga 3000
   agtagcacgt ctcactagtc tcgtgcagat ggacagcacc gctgagcaat ggaagcgggt 3060
   aggcctttgg ggcagcggcc aatagcagct ttgctccttc gctttctggg ctcagaggct 3120
   gggaaggggt gggtccgggg gcgggctcag gggcgggctc aggggcgggg cgggcgcccg 3180
   aaggtcctcc ggaggcccgg cattctgcac gcttcaaaag cgcacgtctg ccgcgctgtt 3240
   ctcctcttcc tcatctccgg gcctttcgac ctgcagccaa tatgggatcg gccattgaac 3300
   aagatggatt gcacgcaggt tctccggccg cttgggtgga gaggctattc ggctatgact 3360
   gggcacaaca gacaatcggc tgctctgatg ccgccgtgtt ccggctgtca gcgcaggggc 3420
   gcccggttct ttttgtcaag accgacctgt ccggtgccct gaatgaactg caggacgagg 3480
   cagcgcggct atcgtggctg gccacgacgg gcgttccttg cgcagctgtg ctcgacgttg 3540
   tcactgaagc gggaagggac tggctgctat tgggcgaagt gccggggcag gatctcctgt 3600
   catctcacct tgctcctgcc gagaaagtat ccatcatggc tgatgcaatg cggcggctgc 3660
   atacgcttga tccggctacc tgcccattcg accaccaagc gaaacatcgc atcgagcgag 3720
   cacgtactcg gatggaagcc ggtcttgtcg atcaggatga tctggacgaa gagcatcagg 3780
   ggctcgcgcc agccgaactg ttcgccaggc tcaaggcgcg catgcccgac ggcgaggatc 3840
   tcgtcgtgac ccatggcgat gcctgcttgc cgaatatcat ggtggaaaat ggccgctttt 3900
   ctggattcat cgactgtggc cggctgggtg tggcggaccg ctatcaggac atagcgttgg 3960
   ctacccgtga tattgctgaa gagcttggcg gcgaatgggc tgaccgcttc ctcgtgcttt 4020
   acggtatcgc cgctcccgat tcgcagcgca tcgccttcta tcgccttctt gacgagttct 4080
   tctgagggga tcgatccgct gtaagtctgc agaaattgat gatctattaa acaataaaga 4140
   tgtccactaa aatggaagtt tttcctgtca tactttgtta agaagggtga gaacagagta 4200
   cctacatttt gaatggaagg attggagcta cgggggtggg ggtggggtgg gattagataa 4260
   atgcctgctc tttactgaag gctctttact attgctttat gataatgttt catagttgga 4320
   tatcataatt taaacaagca aaaccaaatt aagggccagc tcattcctcc cactcatgat 4380
   ctatagatct atagatctct cgtgggatca ttgtttttct cttgattccc actttgtggt 4440
   tctaagtact gtggtttcca aatgtgtcag tttcatagcc tgaagaacga gatcagcagc 4500
   ctctgttcca catacacttc attctcagta ttgttttgcc aagttctaat tccatcagaa 4560
   gctgactcta gatcccgcgc cgaagttcct atactttcta gagaatagga acttcggaat 4620
   aggaacttca agcttaagcg c                                          4641
<210> 10
   <211> 4640
   <212> DNA
   <213> Artificial Sequence
<220>
   <223> Description of Artificial Sequence: Rluc tet02 insert
<400> 10
   tctaggtaac cgatatccct gcaggggtga cctgcacgtc tagggcgcag tagtccaggg 60
   tttccttgat gatgtcatac ttatcctgtc cctttttttt ccacagctcg cggttgagga 120
   caaactcttc gcggtctttc cagtactcct gcaggtgact gactgagtcg agatctgcga 180
   tctaagtaag cttggcattc cggtactgtt ggtaaagcca ccatggcttc caaggtgtac 240
   gaccccgagc aacgcaaacg catgatcact gggcctcagt ggtgggctcg ctgcaagcaa 300
   atgaacgtgc tggactcctt catcaactac tatgattccg agaagcacgc cgagaacgcc 360
   gtgatttttc tgcatggtaa cgctgcctcc agctacctgt ggaggcacgt cgtgcctcac 420
   atcgagcccg tggctagatg catcatccct gatctgatcg gaatgggtaa gtccggcaag 480
   agcgggaatg gctcatatcg cctcctggat cactacaagt acctcaccgc ttggttcgag 540
   ctgctgaacc ttccaaagaa aatcatcttt gtgggccacg actggggggc ttgtctggcc 600
   tttcactact cctacgagca ccaagacaag atcaaggcca tcgtccatgc tgagagtgtc 660
   gtggacgtga tcgagtcctg ggacgagtgg cctgacatcg aggaggatat cgccctgatc 720
   aagagcgaag agggcgagaa aatggtgctt gagaataact tcttcgtcga gaccatgctc 780
   ccaagcaaga tcatgcggaa actggagcct gaggagttcg ctgcctacct ggagccattc 840
   aaggagaagg gcgaggttag acggcctacc ctctcctggc ctcgcgagat ccctctcgtt 900
   aagggaggca agcccgacgt cgtccagatt gtccgcaact acaacgccta ccttcgggcc 960
   agcgacgatc tgcctaagat gttcatcgag tccgaccctg ggttcttttc caacgctatt 1020
   gtcgagggag ctaagaagtt ccctaacacc gagttcgtga aggtgaaggg cctccacttc 1080
   agccaggagg acgctccaga tgaaatgggt aagtacatca agagcttcgt ggagcgcgtg 1140
   ctgaagaacg agcagtaatt ctagaccggt tcgagatcca ggcgcggatc aataaaagat 1200
   cattattttc aatagatctg tgtgttggtt ttttgtgtgc cttgggggag ggggaggcca 1260
   gaatgaggcg cggccaaggg ggagggggag gccagaatga ccttggggga gggggaggcc 1320
   agaatgacct tgggggaggg ggaggccaga atgaggcgcg gatccgtcga cttaattaag 1380
   gccagggatc ttcaagcaga cctacagcaa gttcgacaca aactcacaca acgatgacgc 1440
   actactcaag aactacgggc tgctctactg cttcaggaag gacatggaca aggtcgagac 1500
   attcctgcgc atcgtgcagt gccgctctgt ggagggcagc tgtggcttct agctgcccgg 1560
   gtggcatccc tgtgacccct ccccagtgcc tctcctggcc ctggaagttg ccactccagt 1620
   gcccaccagc cttgtcctaa taaaattaag ttgcatcatt ttgtctgact aggtgtcctt 1680
   ctataatatt atggggtgga ggggggtggt atggagcaag gggcaagttg ggaagacaac 1740
   ctgtagggcc tgcggggtct attgggaacc aagctggagt gcagtggcac aatcttggct 1800
   cactgcaatc tccgcctcct gggttcaagc gattctcctg cctcagcctc ccgagttgtt 1860
   gggattccag gcatgcatga ccaggctcag ctaatttttg tttttttggt agagacgggg 1920
   tttcaccata ttggccaggc tggtctccaa ctcctaatct caggtgatct acccaccttg 1980
   gcctcccaaa ttgctgggat tacaggcgtg aaccactgct cccttccctg tccttctgat 2040
   tttaaaataa ctataccagc aggaggacgt ccagacacag cataggctac ctggccatgc 2100
   ccaaccggtg ggacatttga gttgcttgct tggcactgtc ctctcatgcg ttgggtccac 2160
   tcagtagatg cctgttgaat taagcttatt taaataggcc ggccataact tcgtataatg 2220
   tatgctatac gaagttatgg atccagtgga aagacgcgca ggcaaaacgc accacgtgac 2280
   ggagcgtgac cgcgcgccga gcccaaggtc gggcaggaag agggcctatt tcccatgatt 2340
   ccttcatatt tgcatatacg atacaaggct gttagagaga taattagaat taatttgact 2400
   gtaaacacaa agatattagt acaaaatacg tgacgtagaa agtaataatt tcttgggtag 2460
   tttgcagttt taaaattatg ttttaaaatg gactatcata tgcttaccgt aacttgaaag 2520
   tatttcgatt tcttggcttt atatatctcc ctatcagtga tagagaaagg gattccaatt 2580
   cagcgggagc cacctgatga agcttgatcg ggtggctctc gctgagttgg aatccatttt 2640
   tttctagact cgagataact tcgtataatg tatgctatac gaagttatgg cgcgccggta 2700
   accgaagttc ctatactttc tagagaatag gaacttcgga ataggaactt cttaggtcaa 2760
   ttctaccggg taggggaggc gcttttccca aggcagtctg gagcatgcgc tttagcagcc 2820
   ccgctgggca cttggcgcta cacaagtggc ctctggcctc gcacacattc cacatccacc 2880
   ggtaggcgcc aaccggctcc gttctttggt ggccccttcg cgccaccttc tactcctccc 2940
   ctagtcagga agttcccccc cgccccgcag ctcgcgtcgt gcaggacgtg acaaatggaa 3000
   gtagcacgtc tcactagtct cgtgcagatg gacagcaccg ctgagcaatg gaagcgggta 3060
   ggcctttggg gcagcggcca atagcagctt tgctccttcg ctttctgggc tcagaggctg 3120
   ggaaggggtg ggtccggggg cgggctcagg ggcgggctca ggggcggggc gggcgcccga 3180
   aggtcctccg gaggcccggc attctgcacg cttcaaaagc gcacgtctgc cgcgctgttc 3240
   tcctcttcct catctccggg cctttcgacc tgcagccaat atgggatcgg ccattgaaca 3300
   agatggattg cacgcaggtt ctccggccgc ttgggtggag aggctattcg gctatgactg 3360
   ggcacaacag acaatcggct gctctgatgc cgccgtgttc cggctgtcag cgcaggggcg 3420
   cccggttctt tttgtcaaga ccgacctgtc cggtgccctg aatgaactgc aggacgaggc 3480
   agcgcggcta tcgtggctgg ccacgacggg cgttccttgc gcagctgtgc tcgacgttgt 3540
   cactgaagcg ggaagggact ggctgctatt gggcgaagtg ccggggcagg atctcctgtc 3600
   atctcacctt gctcctgccg agaaagtatc catcatggct gatgcaatgc ggcggctgca 3660
   tacgcttgat ccggctacct gcccattcga ccaccaagcg aaacatcgca tcgagcgagc 3720
   acgtactcgg atggaagccg gtcttgtcga tcaggatgat ctggacgaag agcatcaggg 3780
   gctcgcgcca gccgaactgt tcgccaggct caaggcgcgc atgcccgacg gcgaggatct 3840
   cgtcgtgacc catggcgatg cctgcttgcc gaatatcatg gtggaaaatg gccgcttttc 3900
   tggattcatc gactgtggcc ggctgggtgt ggcggaccgc tatcaggaca tagcgttggc 3960
   tacccgtgat attgctgaag agcttggcgg cgaatgggct gaccgcttcc tcgtgcttta 4020
   cggtatcgcc gctcccgatt cgcagcgcat cgccttctat cgccttcttg acgagttctt 4080
   ctgaggggat cgatccgctg taagtctgca gaaattgatg atctattaaa caataaagat 4140
   gtccactaaa atggaagttt ttcctgtcat actttgttaa gaagggtgag aacagagtac 4200
   ctacattttg aatggaagga ttggagctac gggggtgggg gtggggtggg attagataaa 4260
   tgcctgctct ttactgaagg ctctttacta ttgctttatg ataatgtttc atagttggat 4320
   atcataattt aaacaagcaa aaccaaatta agggccagct cattcctccc actcatgatc 4380
   tatagatcta tagatctctc gtgggatcat tgtttttctc ttgattccca ctttgtggtt 4440
   ctaagtactg tggtttccaa atgtgtcagt ttcatagcct gaagaacgag atcagcagcc 4500
   tctgttccac atacacttca ttctcagtat tgttttgcca agttctaatt ccatcagaag 4560
   ctgactctag atccccgcgc gaagttccta tactttctag agaataggaa cttcggaata 4620
   ggaacttcaa gcttaagcgc                                             4640
<210> 11
   <211> 3387
   <212> DNA
   <213> Artificial Sequence
<220>
   <223> Description of Artificial Sequence: H1 tet5' shRNA vector
<400> 11
   agctcgatat cggccggcca taacttcgta taatgtatgc tatacgaagt tatggatcct 60
   cacagtaggt ggcatcgttc ctttctgact gcccgccccc cgcatgccgt cccgcgatat 120
   tgagctccga acctctcgcc ctgccgccgc cggtgctccg tcgccgccgc gccgccatgg 180
   aattcgaacg ctgacgtcat caacccgctc caaggaatcg cgggcccagt gtcactaggc 240
   gggaacaccc agcgcgcgtg cgccctggca ggaagatggc tgtgagggac aggggagtgg 300
   cgccctgcaa tatttgcatg tcgctatgtg ttctgggaaa tcaccataaa cgtgaaattc 360
   cctatcagtg atagagatta taagttctgt atgagaccac tctttcccag gattccaatt 420
   cagcgggagc cacctgatga agcttgatcg ggtggctctc gctgagttgg aatccatttt 480
   tttctaggat aacttcgtat aatgtatgct atacgaagtt atggcgcgcc ggtacccagc 540
   ttttgttccc tttagtgagg gttaatttcg agcttggcgt aatcatggtc atagctgttt 600
   cctgtgtgaa attgttatcc gctcacaatt ccacacaaca tacgagccgg aagcataaag 660
   tgtaaagcct ggggtgccta atgagtgagc taactcacat taattgcgtt gcgctcactg 720
   cccgctttcc agtcgggaaa cctgtcgtgc cagctgcatt aatgaatcgg ccaacgcgcg 780
   gggagaggcg gtttgcgtat tgggcgctct tccgcttcct cgctcactga ctcgctgcgc 840
   tcggtcgttc ggctgcggcg agcggtatca gctcactcaa aggcggtaat acggttatcc 900
   acagaatcag gggataacgc aggaaagaac atgtgagcaa aaggccagca aaaggccagg 960
   aaccgtaaaa aggccgcgtt gctggcgttt ttccataggc tccgcccccc tgacgagcat 1020
   cacaaaaatc gacgctcaag tcagaggtgg cgaaacccga caggactata aagataccag 1080
   gcgtttcccc ctggaagctc cctcgtgcgc tctcctgttc cgaccctgcc gcttaccgga 1140
   tacctgtccg cctttctccc ttcgggaagc gtggcgcttt ctcatagctc acgctgtagg 1200
   tatctcagtt cggtgtaggt cgttcgctcc aagctgggct gtgtgcacga accccccgtt 1260
   cagcccgacc gctgcgcctt atccggtaac tatcgtcttg agtccaaccc ggtaagacac 1320
   gacttatcgc cactggcagc agccactggt aacaggatta gcagagcgag gtatgtaggc 1380
   ggtgctacag agttcttgaa gtggtggcct aactacggct acactagaag gacagtattt 1440
   ggtatctgcg ctctgctgaa gccagttacc ttcggaaaaa gagttggtag ctcttgatcc 1500
   ggcaaacaaa ccaccgctgg tagcggtggt ttttttgttt gcaagcagca gattacgcgc 1560
   agaaaaaaag gatctcaaga agatcctttg atcttttcta cggggtctga cgctcagtgg 1620
   aacgaaaact cacgttaagg gattttggtc atgagattat caaaaaggat cttcacctag 1680
   atccttttaa attaaaaatg aagttttaaa tcaatctaaa gtatatatga gtaaacttgg 1740
   tctgacagtt accaatgctt aatcagtgag gcacctatct cagcgatctg tctatttcgt 1800
   tcatccatag ttgcctgact ccccgtcgtg tagataacta cgatacggga gggcttacca 1860
   tctggcccca gtgctgcaat gataccgcga gacccacgct caccggctcc agatttatca 1920
   gcaataaacc agccagccgg aagggccgag cgcagaagtg gtcctgcaac tttatccgcc 1980
   tccatccagt ctattaattg ttgccgggaa gctagagtaa gtagttcgcc agttaatagt 2040
   ttgcgcaacg ttgttgccat tgctacaggc atcgtggtgt cacgctcgtc gtttggtatg 2100
   gcttcattca gctccggttc ccaacgatca aggcgagtta catgatcccc catgttgtgc 2160
   aaaaaagcgg ttagctcctt cggtcctccg atcgttgtca gaagtaagtt ggccgcagtg 2220
   ttatcactca tggttatggc agcactgcat aattctctta ctgtcatgcc atccgtaaga 2280
   tgcttttctg tgactggtga gtactcaacc aagtcattct gagaatagtg tatgcggcga 2340
   ccgagttgct cttgcccggc gtcaatacgg gataataccg cgccacatag cagaacttta 2400
   aaagtgctca tcattggaaa acgttcttcg gggcgaaaac tctcaaggat cttaccgctg 2460
   ttgagatcca gttcgatgta acccactcgt gcacccaact gatcttcagc atcttttact 2520
   ttcaccagcg tttctgggtg agcaaaaaca ggaaggcaaa atgccgcaaa aaagggaata 2580
   agggcgacac ggaaatgttg aatactcata ctcttccttt ttcaatatta ttgaagcatt 2640
   tatcagggtt attgtctcat gagcggatac atatttgaat gtatttagaa aaataaacaa 2700
   ataggggttc cgcgcacatt tccccgaaaa gtgccaccta aattgtaagc gttaatattt 2760
   tgttaaaatt cgcgttaaat ttttgttaaa tcagctcatt ttttaaccaa taggccgaaa 2820
   tcggcaaaat cccttataaa tcaaaagaat agaccgagat agggttgagt gttgttccag 2880
   tttggaacaa gagtccacta ttaaagaacg tggactccaa cgtcaaaggg cgaaaaaccg 2940
   tctatcaggg cgatggccca ctacgtgaac catcacccta atcaagtttt ttggggtcga 3000
   ggtgccgtaa agcactaaat cggaacccta aagggagccc ccgatttaga gcttgacggg 3060
   gaaagccggc gaacgtggcg agaaaggaag ggaagaaagc gaaaggagcg ggcgctaggg 3120
   cgctggcaag tgtagcggtc acgctgcgcg taaccaccac acccgccgcg cttaatgcgc 3180
   cgctacaggg cgcgtcccat tcgccattca ggctgcgcaa ctgttgggaa gggcgatcgg 3240
   tgcgggcctc ttcgctatta cgccagctgg cgaaaggggg atgtgctgca aggcgattaa 3300
   gttgggtaac gccagggttt tcccagtcac gacgttgtaa aacgacggcc agtgaattgt 3360
   aatacgactc actataggcc gaattgg 3387
<210> 12
   <211> 3387
   <212> DNA
   <213> Artificial Sequence
<220>
   <223> Description of Artificial Sequence: H1 tet3' shRNA vector
<400> 12
   agctcgatat cggccggcca taacttcgta taatgtatgc tatacgaagt tatggatcct 60
   cacagtaggt ggcatcgttc ctttctgact gcccgccccc cgcatgccgt cccgcgatat 120
   tgagctccga acctctcgcc ctgccgccgc cggtgctccg tcgccgccgc gccgccatgg 180
   aattcgaacg ctgacgtcat caacccgctc caaggaatcg cgggcccagt gtcactaggc 240
   gggaacaccc agcgcgcgtg cgccctggca ggaagatggc tgtgagggac aggggagtgg 300
   cgccctgcaa tatttgcatg tcgctatgtg ttctgggaaa tcaccataaa cgtgaaatgt 360
   ctttggattt gggaatctta taagtcccta tcagtgatag agattcccag gattccaatt 420
   cagcgggagc cacctgatga agcttgatcg ggtggctctc gctgagttgg aatccatttt 480
   tttctaggat aacttcgtat aatgtatgct atacgaagtt atggcgcgcc ggtacccagc 540
   ttttgttccc tttagtgagg gttaatttcg agcttggcgt aatcatggtc atagctgttt 600
   cctgtgtgaa attgttatcc gctcacaatt ccacacaaca tacgagccgg aagcataaag 660
   tgtaaagcct ggggtgccta atgagtgagc taactcacat taattgcgtt gcgctcactg 720
   cccgctttcc agtcgggaaa cctgtcgtgc cagctgcatt aatgaatcgg ccaacgcgcg 780
   gggagaggcg gtttgcgtat tgggcgctct tccgcttcct cgctcactga ctcgctgcgc 840
   tcggtcgttc ggctgcggcg agcggtatca gctcactcaa aggcggtaat acggttatcc 900
   acagaatcag gggataacgc aggaaagaac atgtgagcaa aaggccagca aaaggccagg 960
   aaccgtaaaa aggccgcgtt gctggcgttt ttccataggc tccgcccccc tgacgagcat 1020
   cacaaaaatc gacgctcaag tcagaggtgg cgaaacccga caggactata aagataccag 1080
   gcgtttcccc ctggaagctc cctcgtgcgc tctcctgttc cgaccctgcc gcttaccgga 1140
   tacctgtccg cctttctccc ttcgggaagc gtggcgcttt ctcatagctc acgctgtagg 1200
   tatctcagtt cggtgtaggt cgttcgctcc aagctgggct gtgtgcacga accccccgtt 1260
   cagcccgacc gctgcgcctt atccggtaac tatcgtcttg agtccaaccc ggtaagacac 1320
   gacttatcgc cactggcagc agccactggt aacaggatta gcagagcgag gtatgtaggc 1380
   ggtgctacag agttcttgaa gtggtggcct aactacggct acactagaag gacagtattt 1440
   ggtatctgcg ctctgctgaa gccagttacc ttcggaaaaa gagttggtag ctcttgatcc 1500
   ggcaaacaaa ccaccgctgg tagcggtggt ttttttgttt gcaagcagca gattacgcgc 1560
   agaaaaaaag gatctcaaga agatcctttg atcttttcta cggggtctga cgctcagtgg 1620
   aacgaaaact cacgttaagg gattttggtc atgagattat caaaaaggat cttcacctag 1680
   atccttttaa attaaaaatg aagttttaaa tcaatctaaa gtatatatga gtaaacttgg 1740
   tctgacagtt accaatgctt aatcagtgag gcacctatct cagcgatctg tctatttcgt 1800
   tcatccatag ttgcctgact ccccgtcgtg tagataacta cgatacggga gggcttacca 1860
   tctggcccca gtgctgcaat gataccgcga gacccacgct caccggctcc agatttatca 1920
   gcaataaacc agccagccgg aagggccgag cgcagaagtg gtcctgcaac tttatccgcc 1980
   tccatccagt ctattaattg ttgccgggaa gctagagtaa gtagttcgcc agttaatagt 2040
   ttgcgcaacg ttgttgccat tgctacaggc atcgtggtgt cacgctcgtc gtttggtatg 2100
   gcttcattca gctccggttc ccaacgatca aggcgagtta catgatcccc catgttgtgc 2160
   aaaaaagcgg ttagctcctt cggtcctccg atcgttgtca gaagtaagtt ggccgcagtg 2220
   ttatcactca tggttatggc agcactgcat aattctctta ctgtcatgcc atccgtaaga 2280
   tgcttttctg tgactggtga gtactcaacc aagtcattct gagaatagtg tatgcggcga 2340
   ccgagttgct cttgcccggc gtcaatacgg gataataccg cgccacatag cagaacttta 2400
   aaagtgctca tcattggaaa acgttcttcg gggcgaaaac tctcaaggat cttaccgctg 2460
   ttgagatcca gttcgatgta acccactcgt gcacccaact gatcttcagc atcttttact 2520
   ttcaccagcg tttctgggtg agcaaaaaca ggaaggcaaa atgccgcaaa aaagggaata 2580
   agggcgacac ggaaatgttg aatactcata ctcttccttt ttcaatatta ttgaagcatt 2640
   tatcagggtt attgtctcat gagcggatac atatttgaat gtatttagaa aaataaacaa 2700
   ataggggttc cgcgcacatt tccccgaaaa gtgccaccta aattgtaagc gttaatattt 2760
   tgttaaaatt cgcgttaaat ttttgttaaa tcagctcatt ttttaaccaa taggccgaaa 2820
   tcggcaaaat cccttataaa tcaaaagaat agaccgagat agggttgagt gttgttccag 2880
   tttggaacaa gagtccacta ttaaagaacg tggactccaa cgtcaaaggg cgaaaaaccg 2940
   tctatcaggg cgatggccca ctacgtgaac catcacccta atcaagtttt ttggggtcga 3000
   ggtgccgtaa agcactaaat cggaacccta aagggagccc ccgatttaga gcttgacggg 3060
   gaaagccggc gaacgtggcg agaaaggaag ggaagaaagc gaaaggagcg ggcgctaggg 3120
   cgctggcaag tgtagcggtc acgctgcgcg taaccaccac acccgccgcg cttaatgcgc 3180
   cgctacaggg cgcgtcccat tcgccattca ggctgcgcaa ctgttgggaa gggcgatcgg 3240
   tgcgggcctc ttcgctatta cgccagctgg cgaaaggggg atgtgctgca aggcgattaa 3300
   gttgggtaac gccagggttt tcccagtcac gacgttgtaa aacgacggcc agtgaattgt 3360
   aatacgactc actatagggc gaattgg 3387
<210> 13
   <211> 3387
   <212> DNA
   <213> Artificial Sequence
<220>
   <223> Description of Artificial Sequence: H1 tet5' + 3' shRNA vector
<400> 13
   agctcgatat cggccggcca taacttcgta taatgtatgc tatacgaagt tatggatcct 60
   cacagtaggt ggcatcgttc ctttctgact gcccgccccc cgcatgccgt cccgcgatat 120
   tgagctccga acctctcgcc ctgccgccgc cggtgctccg tcgccgccgc gccgccatgg 180
   aattcgaacg ctgacgtcat caacccgctc caaggaatcg cgggcccagt gtcactaggc 240
   gggaacaccc agcgcgcgtg cgccctggca ggaagatggc tgtgagggac aggggagtgg 300
   cgccctgcaa tatttgcatg tcgctatgtg ttctgggaaa tcaccataaa cgtgaaattc 360
   cctatcagtg atagagatta taagtcccta tcagtgatag agattcccag gattccaatt 420
   cagcgggagc cacctgatga agcttgatcg ggtggctctc gctgagttgg aatccatttt 480
   tttctaggat aacttcgtat aatgtatgct atacgaagtt atggcgcgcc ggtacccagc 540
   ttttgttccc tttagtgagg gttaatttcg agcttggcgt aatcatggtc atagctgttt 600
   cctgtgtgaa attgttatcc gctcacaatt ccacacaaca tacgagccgg aagcataaag 660
   tgtaaagcct ggggtgccta atgagtgagc taactcacat taattgcgtt gcgctcactg 720
   cccgctttcc agtcgggaaa cctgtcgtgc cagctgcatt aatgaatcgg ccaacgcgcg 780
   gggagaggcg gtttgcgtat tgggcgctct tccgcttcct cgctcactga ctcgctgcgc 840
   tcggtcgttc ggctgcggcg agcggtatca gctcactcaa aggcggtaat acggttatcc 900
   acagaatcag gggataacgc aggaaagaac atgtgagcaa aaggccagca aaaggccagg 960
   aaccgtaaaa aggccgcgtt gctggcgttt ttccataggc tccgcccccc tgacgagcat 1020
   cacaaaaatc gacgctcaag tcagaggtgg cgaaacccga caggactata aagataccag 1080
   gcgtttcccc ctggaagctc cctcgtgcgc tctcctgttc cgaccctgcc gcttaccgga 1140
   tacctgtccg cctttctccc ttcgggaagc gtggcgcttt ctcatagctc acgctgtagg 1200
   tatctcagtt cggtgtaggt cgttcgctcc aagctgggct gtgtgcacga accccccgtt 1260
   cagcccgacc gctgcgcctt atccggtaac tatcgtcttg agtccaaccc ggtaagacac 1320
   gacttatcgc cactggcagc agccactggt aacaggatta gcagagcgag gtatgtaggc 1380
   ggtgctacag agttcttgaa gtggtggcct aactacggct acactagaag gacagtattt 1440
   ggtatctgcg ctctgctgaa gccagttacc ttcggaaaaa gagttggtag ctcttgatcc 1500
   ggcaaacaaa ccaccgctgg tagcggtggt ttttttgttt gcaagcagca gattacgcgc 1560
   agaaaaaaag gatctcaaga agatcctttg atcttttcta cggggtctga cgctcagtgg 1620
   aacgaaaact cacgttaagg gattttggtc atgagattat caaaaaggat cttcacctag 1680
   atccttttaa attaaaaatg aagttttaaa tcaatctaaa gtatatatga gtaaacttgg 1740
   tctgacagtt accaatgctt aatcagtgag gcacctatct cagcgatctg tctatttcgt 1800
   tcatccatag ttgcctgact ccccgtcgtg tagataacta cgatacggga gggcttacca 1860
   tctggcccca gtgctgcaat gataccgcga gacccacgct caccggctcc agatttatca 1920
   gcaataaacc agccagccgg aagggccgag cgcagaagtg gtcctgcaac tttatccgcc 1980
   tccatccagt ctattaattg ttgccgggaa gctagagtaa gtagttcgcc agttaatagt 2040
   ttgcgcaacg ttgttgccat tgctacaggc atcgtggtgt cacgctcgtc gtttggtatg 2100
   gcttcattca gctccggttc ccaacgatca aggcgagtta catgatcccc catgttgtgc 2160
   aaaaaagcgg ttagctcctt cggtcctccg atcgttgtca gaagtaagtt ggccgcagtg 2220
   ttatcactca tggttatggc agcactgcat aattctctta ctgtcatgcc atccgtaaga 2280
   tgcttttctg tgactggtga gtactcaacc aagtcattct gagaatagtg tatgcggcga 2340
   ccgagttgct cttgcccggc gtcaatacgg gataataccg cgccacatag cagaacttta 2400
   aaagtgctca tcattggaaa acgttcttcg gggcgaaaac tctcaaggat cttaccgctg 2460
   ttgagatcca gttcgatgta acccactcgt gcacccaact gatcttcagc atcttttact 2520
   ttcaccagcg tttctgggtg agcaaaaaca ggaaggcaaa atgccgcaaa aaagggaata 2580
   agggcgacac ggaaatgttg aatactcata ctcttccttt ttcaatatta ttgaagcatt 2640
   tatcagggtt attgtctcat gagcggatac atatttgaat gtatttagaa aaataaacaa 2700
   ataggggttc cgcgcacatt tccccgaaaa gtgccaccta aattgtaagc gttaatattt 2760
   tgttaaaatt cgcgttaaat ttttgttaaa tcagctcatt ttttaaccaa taggccgaaa 2820
   tcggcaaaat cccttataaa tcaaaagaat agaccgagat agggttgagt gttgttccag 2880
   tttggaacaa gagtccacta ttaaagaacg tggactccaa cgtcaaaggg cgaaaaaccg 2940
   tctatcaggg cgatggccca ctacgtgaac catcacccta atcaagtttt ttggggtcga 3000
   ggtgccgtaa agcactaaat cggaacccta aagggagccc ccgatttaga gcttgacggg 3060
   gaaagccggc gaacgtggcg agaaaggaag ggaagaaagc gaaaggagcg ggcgctaggg 3120
   cgctggcaag tgtagcggtc acgctgcgcg taaccaccac acccgccgcg cttaatgcgc 3180
   cgctacaggg cgcgtcccat tcgccattca ggctgcgcaa ctgttgggaa gggcgatcgg 3240
   tgcgggcctc ttcgctatta cgccagctgg cgaaaggggg atgtgctgca aggcgattaa 3300
   gttgggtaac gccagggttt tcccagtcac gacgttgtaa aacgacggcc agtgaattgt 3360
   aatacgactc actatagggc gaattgg 3387
<210> 14
   <211> 7209
   <212> DNA
   <213> Artificial Sequence
<220>
   <223> Description of Artificial Sequence: Fluc-GAGGS-tetR-hygro insert
<400> 14
   tctaggtaac cgatatccct gcaggggtga cctgcacgtc tagggcgcag tagtccaggg 60
   tttccttgat gatgtcatac ttatcctgtc cctttttttt ccacagctcg cggttgagga 120
   caaactcttc gcggtctttc cagtactcct gcaggtgact gactgagtcg agatctgcga 180
   tctaagtaag cttggcattc cggtactgtt ggtaaagcca ccatggaaga cgccaaaaac 240
   ataaagaaag gcccggcgcc attctatccg ctggaagatg gaaccgctgg agagcaactg 300
   cataaggcta tgaagagata cgccctggtt cctggaacaa ttgcttttac agatgcacat 360
   atcgaggtgg acatcactta cgctgagtac ttcgaaatgt ccgttcggtt ggcagaagct 420
   atgaaacgat atgggctgaa tacaaatcac agaatcgtcg tatgcagtga aaactctctt 480
   caattcttta tgccggtgtt gggcgcgtta tttatcggag ttgcagttgc gcccgcgaac 540
   gacatttata atgaacgtga attgctcaac agtatgggca tttcgcagcc taccgtggtg 600
   ttcgtttcca aaaaggggtt gcaaaaaatt ttgaacgtgc aaaaaaagct cccaatcatc 660
   caaaaaatta ttatcatgga ttctaaaacg gattaccagg gatttcagtc gatgtacacg 720
   ttcgtcacat ctcatctacc tcccggtttt aatgaatacg attttgtgcc agagtccttc 780
   gatagggaca agacaattgc actgatcatg aactcctctg gatctactgg tctgcctaaa 840
   ggtgtcgctc tgcctcatag aactgcctgc gtgagattct cgcatgccag agatcctatt 900
   tttggcaatc aaatcattcc ggatactgcg attttaagtg ttgttccatt ccatcacggt 960
   tttggaatgt ttactacact cggatatttg atatgtggat ttcgagtcgt cttaatgtat 1020
   agatttgaag aagagctgtt tctgaggagc cttcaggatt acaagattca aagtgcgctg 1080
   ctggtgccaa ccctattctc cttcttcgcc aaaagcactc tgattgacaa atacgattta 1140
   tctaatttac acgaaattgc ttctggtggc gctcccctct ctaaggaagt cggggaagcg 1200
   gttgccaaga ggttccatct gccaggtatc aggcaaggat atgggctcac tgagactaca 1260
   tcagctattc tgattacacc cgagggggat gataaaccgg gcgcggtcgg taaagttgtt 1320
   ccattttttg aagcgaaggt tgtggatctg gataccggga aaacgctggg cgttaatcaa 1380
   agaggcgaac tgtgtgtgag aggtcctatg attatgtccg gttatgtaaa caatccggaa 1440
   gcgaccaacg ccttgattga caaggatgga tggctacatt ctggagacat agcttactgg 1500
   gacgaagacg aacacttctt catcgttgac cgcctgaagt ctctgattaa gtacaaaggc 1560
   tatcaggtgg ctcccgctga attggaatcc atcttgctcc aacaccccaa catcttcgac 1620
   gcaggtgtcg caggtcttcc cgacgatgac gccggtgaac ttcccgccgc cgttgttgtt 1680
   ttggagcacg gaaagacgat gacggaaaaa gagatcgtgg attacgtcgc cagtcaagta 1740
   acaaccgcga aaaagttgcg cggaggagtt gtgtttgtgg acgaagtacc gaaaggtctt 1800
   accggaaaac tcgacgcaag aaaaatcaga gagatcctca taaaggccaa gaagggcgga 1860
   aagatcgccg tgtaattcta gaccggttcg agatccaggc gcggatcaat aaaagatcat 1920
   tattttcaat agatctgtgt gttggttttt tgtgtgcctt gggggagggg gaggccagaa 1980
   tgaggcgcgg ccaaggggga gggggaggcc agaatgacct tgggggaggg ggaggccaga 2040
   atgaccttgg gggaggggga ggccagaatg aggcgcgccc ccgatccgtc gacgcccggg 2100
   ctagcttgca tgcctgcagg ttttcgacat tgattattga ctagttatta atagtaatca 2160
   attacggggt cattagttca tagcccatat atggagttcc gcgttacata acttacggta 2220
   aatggcccgc ctggctgacc gcccaacgac ccccgcccat tgacgtcaat aatgacgtat 2280
   gttcccatag taacgccaat agggactttc cattgacgtc aatgggtgga ctatttacgg 2340
   taaactgccc acttggcagt acatcaagtg tatcatatgc caagtacgcc ccctattgac 2400
   gtcaatgacg gtaaatggcc cgcctggcat tatgcccagt acatgacctt atgggacttt 2460
   cctacttggc agtacatcta cgtattagtc atcgctatta ccatgggtcg aggtgagccc 2520
   cacgttctgc ttcactctcc ccatctcccc cccctcccca cccccaattt tgtatttatt 2580
   tattttttaa ttattttgtg cagcgatggg ggcggggggg gggggggcgc gcgccaggcg 2640
   gggcggggcg gggcgagggg cggggcgggg cgaggcggag aggtgcggcg gcagccaatc 2700
   agagcggcgc gctccgaaag tttcctttta tggcgaggcg gcggcggcgg cggccctata 2760
   aaaagcgaag cgcgcggcgg gcgggagtcg ctgcgttgcc ttcgccccgt gccccgctcc 2820
   gcgccgcctc gcgccgcccg ccccggctct gactgaccgc gttactccca caggtgagcg 2880
   ggcgggacgg cccttctcct ccgggctgta attagcgctt ggtttaatga cggctcgttt 2940
   cttttctgtg gctgcgtgaa agccttaaag ggctccggga gggccctttg tgcggggggg 3000
   agcggctcgg ggggtgcgtg cgtgtgtgtg tgcgtgggga gcgccgcgtg cggcccgcgc 3060
   tgcccggcgg ctgtgagcgc tgcgggcgcg gcgcggggct ttgtgcgctc cgcgtgtgcg 3120
   cgaggggagc gcggccgggg gcggtgcccc gcggtgcggg ggggctgcga ggggaacaaa 3180
   ggctgcgtgc ggggtgtgtg cgtggggggg tgagcagggg gtgtgggcgc ggcggtcggg 3240
   ctgtaacccc cccctgcacc cccctccccg agttgctgag cacggcccgg cttcgggtgc 3300
   ggggctccgt gcggggcgtg gcgcggggct cgccgtgccg ggcggggggt ggcggcaggt 3360
   gggggtgccg ggcggggcgg ggccgcctcg ggccggggag ggctcggggg aggggcgcgg 3420
   cggccccgga gcgccggcgg ctgtcgaggc gcggcgagcc gcagccattg ccttttatgg 3480
   taatcgtgcg agagggcgca gggacttcct ttgtcccaaa tctggcggag ccgaaatctg 3540
   ggaggcgccg ccgcaccccc tctagcgggc gcgggcgaag cggtgcggcg ccggcaggaa 3600
   ggaaatgggc ggggagggcc ttcgtgcgtc gccgcgccgc cgtccccttc tccatctcca 3660
   gcctcggggc tgccgcaggg ggacggctgc cttcgggggg gacggggcag ggcggggttc 3720
   ggcttctggc gtgtgaccgg cggctctaga agcgttgggg tgagtactcc ctctcaaaag 3780
   cgggcatgac ttctgcgcta agattgtcag tttccaaaaa cgaggaggat ttgatattca 3840
   cctggcccgc ggtgatgcct ttgagggtgg ccgcgtccat ctggtcagaa aagacaatct 3900
   ttttgttgtc aagcttgagg tgtggcaggc ttgagatctg gccatacact tgagtgacat 3960
   tgacatccac tttgcctttc tctccacagg tgtccactcc cagggcggcc tccggagcga 4020
   tcgccgatcc gcctagcatt caaccatggc tagattagat aaaagtaaag tgattaacag 4080
   cgcattagag ctgcttaatg aggtcggaat cgaaggttta acaacccgta aactcgccca 4140
   gaagctaggt gtagagcagc ctacattgta ttggcatgta aaaaataagc gggctttgct 4200
   cgacgcctta gccattgaga tgttagatag gcaccatact cacttttgcc ctttagaagg 4260
   ggaaagctgg caagattttt tacgtaataa cgctaaaagt tttagatgtg ctttactaag 4320
   tcatcgcgat ggagcaaaag tacatttagg tacacggcct acagaaaaac agtatgaaac 4380
   tctcgaaaat caattagcct ttttatgcca acaaggtttt tcactagaga atgcattata 4440
   tgcactcagc gctgtggggc attttacttt aggttgcgta ttggaagatc aagagcatca 4500
   agtcgctaaa gaagaaaggg aaacacctac tactgatagt atgccgccat tattacgaca 4560
   agctatcgaa ttatttgatc accaaggtgc agagccagcc ttcttattcg gccttgaatt 4620
   gatcatatgc ggattagaaa aacaacttaa atgtgaaagt gggtcgtaac cggttcgaga 4680
   tccaggcgcg gatcaataaa agatcattat tttcaataga tctgtgtgtt ggttttttgt 4740
   gtgccttggg ggagggggag gccagaatga ggcgcggcca agggggaggg ggaggccaga 4800
   atgaccttgg gggaggggga ggccagaatg accttggggg agggggaggc cagaatgagg 4860
   cgcgcccccg ggtaccgagc tcgaattgtt aattaaggcc atagcggccg ccctgaggcc 4920
   gcgggcgatc gcctaggggt aaccgaagtt cctatacttt ctagagaata ggaacttcgg 4980
   aataggaact tcttataatc tagaagatct ggatccacga ttcgagggcc cctgcaggtc 5040
   aattctaccg ggtaggggag gcgcttttcc caaggcagtc tggagcatgc gctttagcag 5100
   ccccgctggc acttggcgct acacaagtgg cctctggcct cgcacacatt ccacatccac 5160
   cggtagcgcc aaccggctcc gttctttggt ggccccttcg cgccaccttc tactcctccc 5220
   ctagtcagga agttcccccc cgccccgcag ctcgcgtcgt gcaggacgtg acaaatggaa 5280
   gtagcacgtc tcactagtct cgtgcagatg gacagcaccg ctgagcaatg gaagcgggta 5340
   ggcctttggg gcagcggcca atagcagctt tgctccttcg ctttctgggc tcagaggctg 5400
   ggaaggggtg ggtccggggg cgggctcagg ggcgggctca ggggcggggc gggcgcgaag 5460
   gtcctcccga ggcccggcat tctcgcacgc ttcaaaagcg cacgtctgcc gcgctgttct 5520
   cctcttcctc atctccgggc ctttcgacga tccagccgcc accatgaaaa agcctgaact 5580
   caccgcgacg tctgtcgaga agtttctgat cgaaaagttc gacagcgtct ccgacctgat 5640
   gcagctctcg gagggcgaag aatctcgtgc tttcagcttc gatgtaggag ggcgtggata 5700
   tgtcctgcgg gtaaatagct gcgccgatgg tttctacaaa gatcgttatg tttatcggca 5760
   ctttgcatcg gccgcgctcc cgattccgga agtgcttgac attggggaat tcagcgagag 5820
   cctgacctat tgcatctccc gccgtgcaca gggtgtcacg ttgcaagacc tgcctgaaac 5880
   cgaactgccc gctgttctgc agccggtcgc ggaggccatg gatgccatcg ctgcggccga 5940
   tcttagccag acgagcgggt tcggcccatt cggaccgcaa ggaatcggtc aatacactac 6000
   atggcgtgat ttcatatgcg cgattgctga tccccatgtg tatcactggc aaactgtgat 6060
   ggacgacacc gtcagtgcgt ccgtcgcgca ggctctcgat gagctgatgc tttgggccga 6120
   ggactgcccc gaagtccggc acctcgtgca cgcggatttc ggctccaaca atgtcctgac 6180
   ggacaatggc cgcataacag cggtcattga ctggagcgag gcgatgttcg gggattccca 6240
   atacgaggtc gccaacatct tcttctggag gccgtggttg gcttgtatgg agcagcagac 6300
   gcgctacttc gagcggaggc atccggagct tgcaggatcg ccgcggctcc gggcgtatat 6360
   gctccgcatt ggtcttgacc aactctatca gagcttggtt gacggcaatt tcgatgatgc 6420
   agcttgggcg cagggtcgat gcgacgcaat cgtccgatcc ggagccggga ctgtcgggcg 6480
   tacacaaatc gcccgcagaa gcgcggccgt ctggaccgat ggctgtgtag aagtactcgc 6540
   cgatagtgga aaccgacgcc ccagcactcg tccgagggca aaggaatagt cgatgcagaa 6600
   attgatgatc tattaaacaa taaagatgtc cactaaaatg gaagtttttc ctgtcatact 6660
   ttgttaagaa gggtgagaac agagtaccta cattttgaat ggaaggattg gagctacggg 6720
   ggtgggggtg gggtgggatt agataaatgc ctgctcttta ctgaaggctc tttactattg 6780
   ctttatgata atgtttcata gttggatatc ataatttaaa caagcaaaac caaattaagg 6840
   gccagctcat tcctcccact catgatctat agatctatag atctctcgtg ggatcattgt 6900
   ttttctcttg attcccactt tgtggttcta agtactgtgg tttccaaatg tgtcagtttc 6960
   atagcctgaa gaacgagatc agcagcctct gttccacata cacttcattc tcagtattgt 7020
   tttgccaagt tctaattcca tcagaagctg actctagatc ctgcaggaat tcatatgaag 7080
   ttcctatact ttctagagaa taggaacttc ggaataggaa cttcaaaatg tcgcggcgcg 7140
   ccggtaaccg aagttcctat actttctaga gaataggaac ttcggaatag gaacttcaag 7200
   cttaagcgc                                                         7209
<210> 15
   <211> 7014
   <212> DNA
   <213> Artificial Sequence
<220>
   <223> Description of Artificial Sequence: GAGGS-Fluc
<400> 15
   tagcccgggc tagcttgcat gcctgcaggt tttcgacatt gattattgac tagttattaa 60
   tagtaatcaa ttacggggtc attagttcat agcccatata tggagttccg cgttacataa 120
   cttacggtaa atggcccgcc tggctgaccg cccaacgacc cccgcccatt gacgtcaata 180
   atgacgtatg ttcccatagt aacgccaata gggactttcc attgacgtca atgggtggac 240
   tatttacggt aaactgccca cttggcagta catcaagtgt atcatatgcc aagtacgccc 300
   cctattgacg tcaatgacgg taaatggccc gcctggcatt atgcccagta catgacctta 360
   tgggactttc ctacttggca gtacatctac gtattagtca tcgctattac catgggtcga 420
   ggtgagcccc acgttctgct tcactctccc catctccccc ccctccccac ccccaatttt 480
   gtatttattt attttttaat tattttgtgc agcgatgggg gcgggggggg ggggggcgcg 540
   cgccaggcgg ggcggggcgg ggcgaggggc ggggcggggc gaggcggaga ggtgcggcgg 600
   cagccaatca gagcggcgcg ctccgaaagt ttccttttat ggcgaggcgg cggcggcggc 660
   ggccctataa aaagcgaagc gcgcggcggg cgggagtcgc tgcgttgcct tcgccccgtg 720
   ccccgctccg cgccgcctcg cgccgcccgc cccggctctg actgaccgcg ttactcccac 780
   aggtgagcgg gcgggacggc ccttctcctc cgggctgtaa ttagcgcttg gtttaatgac 840
   ggctcgtttc ttttctgtgg ctgcgtgaaa gccttaaagg gctccgggag ggccctttgt 900
   gcggggggga gcggctcggg gggtgcgtgc gtgtgtgtgt gcgtggggag cgccgcgtgc 960
   ggcccgcgct gcccggcggc tgtgagcgct gcgggcgcgg cgcggggctt tgtgcgctcc 1020
   gcgtgtgcgc gaggggagcg cggccggggg cggtgccccg cggtgcgggg gggctgcgag 1080
   gggaacaaag gctgcgtgcg gggtgtgtgc gtgggggggt gagcaggggg tgtgggcgcg 1140
   gcggtcgggc tgtaaccccc ccctgcaccc ccctccccga gttgctgagc acggcccggc 1200
   ttcgggtgcg gggctccgtg cggggcgtgg cgcggggctc gccgtgccgg gcggggggtg 1260
   gcggcaggtg ggggtgccgg gcggggcggg gccgcctcgg gccggggagg gctcggggga 1320
   ggggcgcggc ggccccggag cgccggcggc tgtcgaggcg cggcgagccg cagccattgc 1380
   cttttatggt aatcgtgcga gagggcgcag ggacttcctt tgtcccaaat ctggcggagc 1440
   cgaaatctgg gaggcgccgc cgcacccccc ctagcgggcg cgggcgaagc ggtgcggcgc 1500
   cggcaggaag gaaatgggcg gggagggcct tcgtgcgtcg ccgcgccgcc gtccccttct 1560
   ccatctccag cctcggggct gccgcagggg gacggctgcc ttcggggggg acggggcagg 1620
   gcggggttcg gcttctggcg tgtgaccggc ggctctagaa gcgttggggt gagtactccc 1680
   tctcaaaagc gggcatgact tctgcgctaa gattgtcagt ttccaaaaac gaggaggatt 1740
   tgatattcac ctggcccgcg gtgatgcctt tgagggtggc cgcgtccatc tggtcagaaa 1800
   agacaatctt tttgttgtca agcttgaggt gtggcaggct tgagatctgg ccatacactt 1860
   gagtgacatt gacatccact ttgcctttct ctccacaggt gtccactccc agggcggcct 1920
   ccggagcgat cgccggtccg cctaggcaat tgtttaaact cgaggatctg cgatctaagt 1980
   aagcttggca ttccggtact gttggtaaag ccaccatgga agacgccaaa aacataaaga 2040
   aaggcccggc gccattctat ccgctggaag atggaaccgc tggagagcaa ctgcataagg 2100
   ctatgaagag atacgccctg gttcctggaa caattgcttt tacagatgca catatcgagg 2160
   tggacatcac ttacgctgag tacttcgaaa tgtccgttcg gttggcagaa gctatgaaac 2220
   gatatgggct gaatacaaat cacagaatcg tcgtatgcag tgaaaactct cttcaattct 2280
   ttatgccggt gttgggcgcg ttatttatcg gagttgcagt tgcgcccgcg aacgacattt 2340
   ataatgaacg tgaattgctc aacagtatgg gcatttcgca gcctaccgtg gtgttcgttt 2400
   ccaaaaaggg gttgcaaaaa attttgaacg tgcaaaaaaa gctcccaatc atccaaaaaa 2460
   ttattatcat ggattctaaa acggattacc agggatttca gtcgatgtac acgttcgtca 2520
   catctcatct acctcccggt tttaatgaat acgattttgt gccagagtcc ttcgataggg 2580
   acaagacaat tgcactgatc atgaactcct ctggatctac tggtctgcct aaaggtgtcg 2640
   ctctgcctca tagaactgcc tgcgtgagat tctcgcatgc cagagatcct atttttggca 2700
   atcaaatcat tccggatact gcgattttaa gtgttgttcc attccatcac ggttttggaa 2760
   tgtttactac actcggatat ttgatatgtg gatttcgagt cgtcttaatg tatagatttg 2820
   aagaagagct gtttctgagg agccttcagg attacaagat tcaaagtgcg ctgctggtgc 2880
   caaccctatt ctccttcttc gccaaaagca ctctgattga caaatacgat ttatctaatt 2940
   tacacgaaat tgcttctggt ggcgctcccc tctctaagga agtcggggaa gcggttgcca 3000
   agaggttcca tctgccaggt atcaggcaag gatatgggct cactgagact acatcagcta 3060
   ttctgattac acccgagggg gatgataaac cgggcgcggt cggtaaagtt gttccatttt 3120
   ttgaagcgaa ggttgtggat ctggataccg ggaaaacgct gggcgttaat caaagaggcg 3180
   aactgtgtgt gagaggtcct atgattatgt ccggttatgt aaacaatccg gaagcgacca 3240
   acgccttgat tgacaaggat ggatggctac attctggaga catagcttac tgggacgaag 3300
   acgaacactt cttcatcgtt gaccgcctga agtctctgat taagtacaaa ggctatcagg 3360
   tggctcccgc tgaattggaa tccatcttgc tccaacaccc caacatcttc gacgcaggtg 3420
   tcgcaggtct tcccgacgat gacgccggtg aacttcccgc cgccgttgtt gttttggagc 3480
   acggaaagac gatgacggaa aaagagatcg tggattacgt cgccagtcaa gtaacaaccg 3540
   cgaaaaagtt gcgcggagga gttgtgtttg tggacgaagt accgaaaggt cttaccggaa 3600
   aactcgacgc aagaaaaatc agagagatcc tcataaaggc caagaagggc ggaaagatcg 3660
   ccgtgtaatt ctagagtcgg ggcggccggc cgcttcgagc agacatgata agatacattg 3720
   atgagtttgg acaaaccaca actagaatgc agtgaaaaaa atgctttatt tgtgaaattt 3780
   gtgatgctat tgctttattt gtaaccatta taagctgcaa taaacaagtt aacaacaaca 3840
   attgcattca ttttatgttt caggttcagg gggaggtgtg ggaggttttt taaagcaagt 3900
   aaaacctcta caaatgtggt aaaatcgata aggatctgaa cgatggagcg gagaatgggc 3960
   ggaactgggc ggagttaggg gcgggatggg cggagttagg ggcgggacta tggttgctga 4020
   ctaattgaga tgcatgcttt gcatacttct gcctgctggg gagcctgggg actttccaca 4080
   cctggttgct gactaattga gatgcatgct ttgcatactt ctgcctgctg gggagcctgg 4140
   ggactttcca caccctaact gacacacatt ccacagcgga tccgtcgacc gatgcccttg 4200
   agagccttca acccagtcag ctccttccgg tgggcgcggg gcatgactat cgtcgccgca 4260
   cttatgactg tcttctttat catgcaactc gtaggacagg tgccggcagc gctcttccgc 4320
   ttcctcgctc actgactcgc tgcgctcggt cgttcggctg cggcgagcgg tatcagctca 4380
   ctcaaaggcg gtaatacggt tatccacaga atcaggggat aacgcaggaa agaacatgtg 4440
   agcaaaaggc cagcaaaagg ccaggaaccg taaaaaggcc gcgttgctgg cgtttttcca 4500
   taggctccgc ccccctgacg agcatcacaa aaatcgacgc tcaagtcaga ggtggcgaaa 4560
   cccgacagga ctataaagat accaggcgtt tccccctgga agctccctcg tgcgctctcc 4620
   tgttccgacc ctgccgctta ccggatacct gtccgccttt ctcccttcgg gaagcgtggc 4680
   gctttctcat agctcacgct gtaggtatct cagttcggtg taggtcgttc gctccaagct 4740
   gggctgtgtg cacgaacccc ccgttcagcc cgaccgctgc gccttatccg gtaactatcg 4800
   tcttgagtcc aacccggtaa gacacgactt atcgccactg gcagcagcca ctggtaacag 4860
   gattagcaga gcgaggtatg taggcggtgc tacagagttc ttgaagtggt ggcctaacta 4920
   cggctacact agaagaacag tatttggtat ctgcgctctg ctgaagccag ttaccttcgg 4980
   aaaaagagtt ggtagctctt gatccggcaa acaaaccacc gctggtagcg gtggtttttt 5040
   tgtttgcaag cagcagatta cgcgcagaaa aaaaggatct caagaagatc ctttgatctt 5100
   ttctacgggg tctgacgctc agtggaacga aaactcacgt taagggattt tggtcatgag 5160
   attatcaaaa aggatcttca cctagatcct tttaaattaa aaatgaagtt ttaaatcaat 5220
   ctaaagtata tatgagtaaa cttggtctga cagttaccaa tgcttaatca gtgaggcacc 5280
   tatctcagcg atctgtctat ttcgttcatc catagttgcc tgactccccg tcgtgtagat 5340
   aactacgata cgggagggct taccatctgg ccccagtgct gcaatgatac cgcgagaccc 5400
   acgctcaccg gctccagatt tatcagcaat aaaccagcca gccggaaggg ccgagcgcag 5460
   aagtggtcct gcaactttat ccgcctccat ccagtctatt aattgttgcc gggaagctag 5520
   agtaagtagt tcgccagtta atagtttgcg caacgttgtt gccattgcta caggcatcgt 5580
   ggtgtcacgc tcgtcgtttg gtatggcttc attcagctcc ggttcccaac gatcaaggcg 5640
   agttacatga tcccccatgt tgtgcaaaaa agcggttagc tccttcggtc ctccgatcgt 5700
   tgtcagaagt aagttggccg cagtgttatc actcatggtt atggcagcac tgcataattc 5760
   tcttactgtc atgccatccg taagatgctt ttctgtgact ggtgagtact caaccaagtc 5820
   attctgagaa tagtgtatgc ggcgaccgag ttgctcttgc ccggcgtcaa tacgggataa 5880
   taccgcgcca catagcagaa ctttaaaagt gctcatcatt ggaaaacgtt cttcggggcg 5940
   aaaactctca aggatcttac cgctgttgag atccagttcg atgtaaccca ctcgtgcacc 6000
   caactgatct tcagcatctt ttactttcac cagcgtttct gggtgagcaa aaacaggaag 6060
   gcaaaatgcc gcaaaaaagg gaataagggc gacacggaaa tgttgaatac tcatactctt 6120
   cctttttcaa tattattgaa gcatttatca gggttattgt ctcatgagcg gatacatatt 6180
   tgaatgtatt tagaaaaata aacaaatagg ggttccgcgc acatttcccc gaaaagtgcc 6240
   acctgacgcg ccctgtagcg gcgcattaag cgcggcgggt gtggtggtta cgcgcagcgt 6300
   gaccgctaca cttgccagcg ccctagcgcc cgctcctttc gctttcttcc cttcctttct 6360
   cgccacgttc gccggctttc cccgtcaagc tctaaatcgg gggctccctt tagggttccg 6420
   atttagtgct ttacggcacc tcgaccccaa aaaacttgat tagggtgatg gttcacgtag 6480
   tgggccatcg ccctgataga cggtttttcg ccctttgacg ttggagtcca cgttctttaa 6540
   tagtggactc ttgttccaaa ctggaacaac actcaaccct atctcggtct attcttttga 6600
   tttataaggg attttgccga tttcggccta ttggttaaaa aatgagctga tttaacaaaa 6660
   atttaacgcg aattttaaca aaatattaac gcttacaatt tgccattcgc cattcaggct 6720
   gcgcaactgt tgggaagggc gatcggtgcg ggcctcttcg ctattacgcc agcccaagct 6780
   accatgataa gtaagtaata ttaaggtacg ggaggtactt ggagcggccg caataaaata 6840
   tctttatttt cattacatct gtgtgttggt tttttgtgtg aatcgatagt actaacatac 6900
   gctctccatc aaaacaaaac gaaacaaaac aaactagcaa aataggctgt ccccagtgca 6960
   agtgcaggtg ccagaacatt tctctatcga taggtaccga gctcttacgc gtgc       7014
<210> 16
   <211> 5430
   <212> DNA
   <213> Artificial Sequence
<220>
   <223> Description of Artificial Sequence: GAGGS-tetR
<400> 16
   ccgggctagc ttgcatgcct gcaggttttc gacattgatt attgactagt tattaatagt 60
   aatcaattac ggggtcatta gttcatagcc catatatgga gttccgcgtt acataactta 120
   cggtaaatgg cccgcctggc tgaccgccca acgacccccg cccattgacg tcaataatga 180
   cgtatgttcc catagtaacg ccaataggga ctttccattg acgtcaatgg gtggactatt 240
   tacggtaaac tgcccacttg gcagtacatc aagtgtatca tatgccaagt acgcccccta 300
   ttgacgtcaa tgacggtaaa tggcccgcct ggcattatgc ccagtacatg accttatggg 360
   actttcctac ttggcagtac atctacgtat tagtcatcgc tattaccatg ggtcgaggtg 420
   agccccacgt tctgcttcac tctccccatc tcccccccct ccccaccccc aattttgtat 480
   ttatttattt tttaattatt ttgtgcagcg atgggggcgg gggggggggg ggcgcgcgcc 540
   aggcggggcg gggcggggcg aggggcgggg cggggcgagg cggagaggtg cggcggcagc 600
   caatcagagc ggcgcgctcc gaaagtttcc ttttatggcg aggcggcggc ggcggcggcc 660
   ctataaaaag cgaagcgcgc ggcgggcggg agtcgctgcg ttgccttcgc cccgtgcccc 720
   gctccgcgcc gcctcgcgcc gcccgccccg gctctgactg accgcgttac tcccacaggt 780
   gagcgggcgg gacggccctt ctcctccggg ctgtaattag cgcttggttt aatgacggct 840
   cgtttctttt ctgtggctgc gtgaaagcct taaagggctc cgggagggcc ctttgtgcgg 900
   gggggagcgg ctcggggggt gcgtgcgtgt gtgtgtgcgt ggggagcgcc gcgtgcggcc 960
   cgcgctgccc ggcggctgtg agcgctgcgg gcgcggcgcg gggctttgtg cgctccgcgt 1020
   gtgcgcgagg ggagcgcggc cgggggcggt gccccgcggt gcgggggggc tgcgagggga 1080
   acaaaggctg cgtgcggggt gtgtgcgtgg gggggtgagc agggggtgtg ggcgcggcgg 1140
   tcgggctgta acccccccct gcacccccct ccccgagttg ctgagcacgg cccggcttcg 1200
   ggtgcggggc tccgtgcggg gcgtggcgcg gggctcgccg tgccgggcgg ggggtggcgg 1260
   caggtggggg tgccgggcgg ggcggggccg cctcgggccg gggagggctc gggggagggg 1320
   cgcggcggcc ccggagcgcc ggcggctgtc gaggcgcggc gagccgcagc cattgccttt 1380
   tatggtaatc gtgcgagagg gcgcagggac ttcctttgtc ccaaatctgg cggagccgaa 1440
   atctgggagg cgccgccgca ccccctctag cgggcgcggg cgaagcggtg cggcgccggc 1500
   aggaaggaaa tgggcgggga gggccttcgt gcgtcgccgc gccgccgtcc ccttctccat 1560
   ctccagcctc ggggctgccg cagggggacg gctgccttcg ggggggacgg ggcagggcgg 1620
   ggttcggctt ctggcgtgtg accggcggct ctagaagcgt tggggtgagt actccctctc 1680
   aaaagcgggc atgacttctg cgctaagatt gtcagtttcc aaaaacgagg aggatttgat 1740
   attcacctgg cccgcggtga tgcctttgag ggtggccgcg tccatctggt cagaaaagac 1800
   aatctttttg ttgtcaagct tgaggtgtgg caggcttgag atctggccat acacttgagt 1860
   gacattgaca tccactttgc ctttctctcc acaggtgtcc actcccaggg cggcctccgg 1920
   agcgatcgcc gatccgccta gcattcaacc atggctagat tagataaaag taaagtgatt 1980
   aacagcgcat tagagctgct taatgaggtc ggaatcgaag gtttaacaac ccgtaaactc 2040
   gcccagaagc taggtgtaga gcagcctaca ttgtattggc atgtaaaaaa taagcgggct 2100
   ttgctcgacg ccttagccat tgagatgtta gataggcacc atactcactt ttgcccttta 2160
   gaaggggaaa gctggcaaga ttttttacgt aataacgcta aaagttttag atgtgcttta 2220
   ctaagtcatc gcgatggagc aaaagtacat ttaggtacac ggcctacaga aaaacagtat 2280
   gaaactctcg aaaatcaatt agccttttta tgccaacaag gtttttcact agagaatgca 2340
   ttatatgcac tcagcgctgt ggggcatttt actttaggtt gcgtattgga agatcaagag 2400
   catcaagtcg ctaaagaaga aagggaaaca cctactactg atagtatgcc gccattatta 2460
   cgacaagcta tcgaattatt tgatcaccaa ggtgcagagc cagccttctt attcggcctt 2520
   gaattgatca tatgcggatt agaaaaacaa cttaaatgtg aaagtgggtc gtaaccggtt 2580
   cgagatccag gcgcggatca ataaaagatc attattttca atagatctgt gtgttggttt 2640
   tttgtgtgcc ttgggggagg gggaggccag aatgaggcgc ggccaagggg gagggggagg 2700
   ccagaatgac cttgggggag gggaggccca gaatgacctt gggggagggg gaggccagaa 2760
   tgaggcgcgc ccccgggtac cgagctcgaa ttcactggcc gtcgttttac aacgtcgtga 2820
   ctgggaaaac cctggcgtta cccaacttaa tcgccttgca gcacatcccc ctttcgccag 2880
   ctggcgtaat agcgaagagg cccgcaccga tcgcccttcc caacagttgc gcagcctgaa 2940
   tggcgaatgg cgcctgatgc ggtattttct ccttacgcat ctgtgcggta tttcacaccg 3000
   catatggtgc actctcagta caatctgctc tgatgccgca tagttaagcc agccccgaca 3060
   cccgccaaca cccgctgacg cgccctgacg ggcttgtctg ctcccggcat ccgcttacag 3120
   acaagctgtg accgtctccg ggagctgcat gtgtcagagg ttttcaccgt catcaccgaa 3180
   acgcgcgaga cgaaagggcc tcgtgatacg cctattttta taggttaatg tcatgataat 3240
   aatggtttct tagacgtcag gtggcacttt tcggggaaat gtgcgcggaa cccctatttg 3300
   tttatttttc taaatacatt caaatatgta tccgctcatg agacaataac cctgataaat 3360
   gcttcaataa tattgaaaaa ggaagagtat gagtattcaa catttccgtg tcgcccttat 3420
   tccctttttt gcggcatttt gccttcctgt ttttgctcac ccagaaacgc tggtgaaagt 3480
   aaaagatgct gaagatcagt tgggtgcacg agtgggttac atcgaactgg atctcaacag 3540
   cggtaagatc cttgagagtt ttcgccccga agaacgtttt ccaatgatga gcacttttaa 3600
   agttctgcta tgtggcgcgg tattatcccg tattgacgcc gggcaagagc aactcggtcg 3660
   ccgcatacac tattctcaga atgacttggt tgagtactca ccagtcacag aaaagcatct 3720
   tacggatggc atgacagtaa gagaattatg cagtgctgcc ataaccatga gtgataacac 3780
   tgcggccaac ttacttctga caacgatcgg aggaccgaag gagctaaccg cttttttgca 3840
   caacatgggg gatcatgtaa ctcgccttga tcgttgggaa ccggagctga atgaagccat 3900
   accaaacgac gagcgtgaca ccacgatgcc tgtagcaatg gcaacaacgt tgcgcaaact 3960
   attaactggc gaactactta ctctagcttc ccggcaacaa ttaatagact ggatggaggc 4020
   ggataaagtt gcaggaccac ttctgcgctc ggcccttccg gctggctggt ttattgctga 4080
   taaatctgga gccggtgagc gtgggtctcg cggtatcatt gcagcactgg ggccagatgg 4140
   taagccctcc cgtatcgtag ttatctacac gacggggagt caggcaacta tggatgaacg 4200
   aaatagacag atcgctgaga taggtgcctc actgattaag cattggtaac tgtcagacca 4260
   agtttactca tatatacttt agattgattt aaaacttcat ttttaattta aaaggatcta 4320
   ggtgaagatc ctttttgata atctcatgac caaaatccct taacgtgagt tttcgttcca 4380
   ctgagcgtca gaccccgtag aaaagatcaa aggatcttct tgagatcctt tttttctgcg 4440
   cgtaatctgc tgcttgcaaa caaaaaaacc accgctacca gcggtggttt gtttgccgga 4500
   tcaagagcta ccaactcttt ttccgaaggt aactggcttc agcagagcgc agataccaaa 4560
   tactgtcctt ctagtgtagc cgtagttagg ccaccacttc aagaactctg tagcaccgcc 4620
   tacatacctc gctctgctaa tcctgttacc agtggctgct gccagtggcg ataagtcgtg 4680
   tcttaccggg ttggactcaa gacgatagtt accggataag gcgcagcggt cgggctgaac 4740
   ggggggttcg tgcacacagc ccagcttgga gcgaacgacc tacaccgaac tgagatacct 4800
   acagcgtgag ctatgagaaa gcgccacgct tcccgaaggg agaaaggcgg acaggtatcc 4860
   ggtaagcggc agggtcggaa caggagagcg cacgagggag cttccagggg gaaacgcctg 4920
   gtatctttat agtcctgtcg ggtttcgcca cctctgactt gagcgtcgat ttttgtgatg 4980
   ctcgtcaggg gggcggagcc tatggaaaaa cgccagcaac gcggcctttt tacggttcct 5040
   ggccttttgc tggccttttg ctcacatgtt ctttcctgcg ttatcccctg attctgtgga 5100
   taaccgtatt accgcctttg agtgagctga taccgctcgc cgcagccgaa cgaccgagcg 5160
   cagcgagtca gtgagcgagg aagcggaaga gcgcccaata cgcaaaccgc ctctccccgc 5220
   gcgttggccg attcattaat gcagctggca cgacaggttt cccgactgga aagcgggcag 5280
   tgagcgcaac gcaattaatg tgagttagct cactcattag gcaccccagg ctttacactt 5340
   tatgcttccg gctcgtatgt tgtgtggaat tgtgagcgga taacaatttc acacaggaaa 5400
   cagctatgac catgattacg ccaagctagc                                  5430
<210> 17
   <211> 7332
   <212> DNA
   <213> Artificial Sequence
<220>
   <223> Description of Artificial Sequence: CMV-LacZ
<400> 17
   aaacagtccg atgtacgggc cagatatacg cgttgacatt gattattgac tagttattaa 60
   tagtaatcaa ttacggggtc attagttcat agcccatata tggagttccg cgttacataa 120
   cttacggtaa atggcccgcc tggctgaccg cccaacgacc cccgcccatt gacgtcaata 180
   atgacgtatg ttcccatagt aacgccaata gggactttcc attgacgtca atgggtggac 240
   tatttacggt aaactgccca cttggcagta catcaagtgt atcatatgcc aagtacgccc 300
   cctattgacg tcaatgacgg taaatggccc gcctggcatt atgcccagta catgacctta 360
   tgggactttc ctacttggca gtacatctac gtattagtca tcgctattac catggtgatg 420
   cggttttggc agtacatcaa tgggcgtgga tagcggtttg actcacgggg atttccaagt 480
   ctccacccca ttgacgtcaa tgggagtttg ttttggcacc aaaatcaacg ggactttcca 540
   aaatgtcgta acaactccgc cccattgacg caaatgggcg gtaggcgtgt acggtgggag 600
   gtctatataa gcagagctct ctggctaact agagaaccca ctgcttactg gcttatcgaa 660
   attaatacga ctcactatag ggagacccaa gctgactcta gacttaatta agcgttgggg 720
   tgagtactcc ctctcaaaag cgggcatgac ttctgcgcta agattgtcag tttccaaaaa 780
   cgaggaggat ttgatattca cctggcccgc ggtgatgcct ttgagggtgg ccgcgtccat 840
   ctggtcagaa aagacaatct ttttgttgtc aagcttgagg tgtggcaggc ttgagatctg 900
   gccatacact tgagtgacat tgacatccac tttgcctttc tctccacagg tgtccactcc 960
   cagggcggcc gcaattcccg gggatcgaaa gagcctgcta aagcaaaaaa gaagtcacca 1020
   tgtcgtttac tttgaccaac aagaacgtga ttttcgttgc cggtctggga ggcattggtc 1080
   tggacaccag caaggagctg ctcaagcgcg atcccgtcgt tttacaacgt cgtgactggg 1140
   aaaaccctgg cgttacccaa cttaatcgcc ttgcagcaca tccccctttc gccagctggc 1200
   gtaatagcga agaggcccgc accgatcgcc cttcccaaca gttgcgcagc ctgaatggcg 1260
   aatggcgctt tgcctggttt ccggcaccag aagcggtgcc ggaaagctgg ctggagtgcg 1320
   atcttcctga ggccgatact gtcgtcgtcc cctcaaactg gcagatgcac ggttacgatg 1380
   cgcccatcta caccaacgta acctatccca ttacggtcaa tccgccgttt gttcccacgg 1440
   agaatccgac gggttgttac tcgctcacat ttaatgttga tgaaagctgg ctacaggaag 1500
   gccagacgcg aattattttt gatggcgtta actcggcgtt tcatctgtgg tgcaacgggc 1560
   gctgggtcgg ttacggccag gacagtcgtt tgccgtctga atttgacctg agcgcatttt 1620
   tacgcgccgg agaaaaccgc ctcgcggtga tggtgctgcg ttggagtgac ggcagttatc 1680
   tggaagatca ggatatgtgg cggatgagcg gcattttccg tgacgtctcg ttgctgcata 1740
   aaccgactac acaaatcagc gatttccatg ttgccactcg ctttaatgat gatttcagcc 1800
   gcgctgtact ggaggctgaa gttcagatgt gcggcgagtt gcgtgactac ctacgggtaa 1860
   cagtttcttt atggcagggt gaaacgcagg tcgccagcgg caccgcgcct ttcggcggtg 1920
   aaattatcga tgagcgtggt ggttatgccg atcgcgtcac actacgtctg aacgtcgaaa 1980
   acccgaaact gtggagcgcc gaaatcccga atctctatcg tgcggtggtt gaactgcaca 2040
   ccgccgacgg cacgctgatt gaagcagaag cctgcgatgt cggtttccgc gaggtgcgga 2100
   ttgaaaatgg tctgctgctg ctgaacggca agccgttgct gattcgaggc gttaaccgtc 2160
   acgagcatca tcctctgcat ggtcaggtca tggatgagca gacgatggtg caggatatcc 2220
   tgctgatgaa gcagaacaac tttaacgccg tgcgctgttc gcattatccg aaccatccgc 2280
   tgtggtacac gctgtgcgac cgctacggcc tgtatgtggt ggatgaagcc aatattgaaa 2340
   cccacggcat ggtgccaatg aatcgtctga ccgatgatcc gcgctggcta ccggcgatga 2400
   gcgaacgcgt aacgcgaatg gtgcagcgcg atcgtaatca cccgagtgtg atcatctggt 2460
   cgctggggaa tgaatcaggc cacggcgcta atcacgacgc gctgtatcgc tggatcaaat 2520
   ctgtcgatcc ttcccgcccg gtgcagtatg aaggcggcgg agccgacacc acggccaccg 2580
   atattatttg cccgatgtac gcgcgcgtgg atgaagacca gcccttcccg gctgtgccga 2640
   aatggtccat caaaaaatgg ctttcgctac ctggagagac gcgcccgctg atcctttgcg 2700
   aatacgccca cgcgatgggt aacagtcttg gcggtttcgc taaatactgg caggcgtttc 2760
   gtcagtatcc ccgtttacag ggcggcttcg tctgggactg ggtggatcag tcgctgatta 2820
   aatatgatga aaacggcaac ccgtggtcgg cttacggcgg tgattttggc gatacgccga 2880
   acgatcgcca gttctgtatg aacggtctgg tctttgccga ccgcacgccg catccagcgc 2940
   tgacggaagc aaaacaccag cagcagtttt tccagttccg tttatccggg caaaccatcg 3000
   aagtgaccag cgaatacctg ttccgtcata gcgataacga gctcctgcac tggatggtgg 3060
   cgctggatgg taagccgctg gcaagcggtg aagtgcctct ggatgtcgct ccacaaggta 3120
   aacagttgat tgaactgcct gaactaccgc agccggagag cgccgggcaa ctctggctca 3180
   cagtacgcgt agtgcaaccg aacgcgaccg catggtcaga agccgggcac atcagcgcct 3240
   ggcagcagtg gcgtctggcg gaaaacctca gtgtgacgct ccccgccgcg tcccacgcca 3300
   tcccgcatct gaccaccagc gaaatggatt tttgcatcga gctgggtaat aagcgttggc 3360
   aatttaaccg ccagtcaggc tttctttcac agatgtggat tggcgataaa aaacaactgc 3420
   tgacgccgct gcgcgatcag ttcacccgtg caccgctgga taacgacatt ggcgtaagtg 3480
   aagcgacccg cattgaccct aacgcctggg tcgaacgctg gaaggcggcg ggccattacc 3540
   aggccgaagc agcgttgttg cagtgcacgg cagatacact tgctgatgcg gtgctgatta 3600
   cgaccgctca cgcgtggcag catcagggga aaaccttatt tatcagccgg aaaacctacc 3660
   ggattgatgg tagtggtcaa atggcgatta ccgttgatgt tgaagtggcg agcgatacac 3720
   cgcatccggc gcggattggc ctgaactgcc agctggcgca ggtagcagag cgggtaaact 3780
   ggctcggatt agggccgcaa gaaaactatc ccgaccgcct tactgccgcc tgttttgacc 3840
   gctgggatct gccattgtca gacatgtata ccccgtacgt cttcccgagc gaaaacggtc 3900
   tgcgctgcgg gacgcgcgaa ttgaattatg gcccacacca gtggcgcggc gacttccagt 3960
   tcaacatcag ccgctacagt caacagcaac tgatggaaac cagccatcgc catctgctgc 4020
   acgcggaaga aggcacatgg ctgaatatcg acggtttcca tatggggatt ggtggcgacg 4080
   actcctggag cccgtcagta tcggcggaat tacagctgag cgccggtcgc taccattacc 4140
   agttggtctg gtgtcaaaaa taataataac cgggcaggcc atgtctgccc gtatttcgcg 4200
   taaggaaatc cattatgtac tatttaaaaa acacaaactt ttggatgttc ggtttattct 4260
   ttttctttta cttttttatc atgggagcct acttcccgtt tttcccgatt tggctacatg 4320
   acatcaacca tatcagcaaa agtgatacgg gtattatttt tgccgctatt tctctgttct 4380
   cgctattatt ccaaccgctg tttggtctgc tttctgacaa actcggcctc gactctaggc 4440
   ggccgcgtcg acctcgagat ccaggcgcgg atcaataaaa gatcattatt ttcaatagat 4500
   ctgtgtgttg gttttttgtg tgccttgggg gagggggagg ccagaatgag gcgcggccaa 4560
   gggggagggg gaggccagaa tgaccttggg ggagggggag gccagaatga ccttggggga 4620
   gggggaggcc agaatgaggc gcgcccccgg gtaccgagct cgaattcact ggccgtcgtt 4680
   ttacaacgtc gtgactggga aaaccctggc gttacccaac ttaatcgcct tgcagcacat 4740
   ccccctttcg ccagctggcg taatagcgaa gaggcccgca ccgatcgccc ttcccaacag 4800
   ttgcgcagcc tgaatggcga atggcgcctg atgcggtatt ttctccttac gcatctgtgc 4860
   ggtatttcac accgcatatg gtgcactctc agtacaatct gctctgatgc cgcatagtta 4920
   agccagcccc gacacccgcc aacacccgct gacgcgccct gacgggcttg tctgctcccg 4980
   gcatccgctt acagacaagc tgtgaccgtc tccgggagct gcatgtgtca gaggttttca 5040
   ccgtcatcac cgaaacgcgc gagacgaaag ggcctcgtga tacgcctatt tttataggtt 5100
   aatgtcatga taataatggt ttcttagacg tcaggtggca cttttcgggg aaatgtgcgc 5160
   ggaaccccta tttgtttatt tttctaaata cattcaaata tgtatccgct catgagacaa 5220
   taaccctgat aaatgcttca ataatattga aaaaggaaga gtatgagtat tcaacatttc 5280
   cgtgtcgccc ttattccctt ttttgcggca ttttgccttc ctgtttttgc tcacccagaa 5340
   acgctggtga aagtaaaaga tgctgaagat cagttgggtg cacgagtggg ttacatcgaa 5400
   ctggatctca acagcggtaa gatccttgag agttttcgcc ccgaagaacg ttttccaatg 5460
   atgagcactt ttaaagttct gctatgtggc gcggtattat cccgtattga cgccgggcaa 5520
   gagcaactcg gtcgccgcat acactattct cagaatgact tggttgagta ctcaccagtc 5580
   acagaaaagc atcttacgga tggcatgaca gtaagagaat tatgcagtgc tgccataacc 5640
   atgagtgata acactgcggc caacttactt ctgacaacga tcggaggacc gaaggagcta 5700
   accgcttttt tgcacaacat gggggatcat gtaactcgcc ttgatcgttg ggaaccggag 5760
   ctgaatgaag ccataccaaa cgacgagcgt gacaccacga tgcctgtagc aatggcaaca 5820
   acgttgcgca aactattaac tggcgaacta cttactctag cttcccggca acaattaata 5880
   gactggatgg aggcggataa agttgcagga ccacttctgc gctcggccct tccggctggc 5940
   tggtttattg ctgataaatc tggagccggt gagcgtgggt ctcgcggtat cattgcagca 6000
   ctggggccag atggtaagcc ctcccgtatc gtagttatct acacgacggg gagtcaggca 6060
   actatggatg aacgaaatag acagatcgct gagataggtg cctcactgat taagcattgg 6120
   taactgtcag accaagttta ctcatatata ctttagattg atttaaaact tcatttttaa 6180
   tttaaaagga tctaggtgaa gatccttttt gataatctca tgaccaaaat cccttaacgt 6240
   gagttttcgt tccactgagc gtcagacccc gtagaaaaga tcaaaggatc ttcttgagat 6300
   cctttttttc tgcgcgtaat ctgctgcttg caaacaaaaa aaccaccgct accagcggtg 6360
   gtttgtttgc cggatcaaga gctaccaact ctttttccga aggtaactgg cttcagcaga 6420
   gcgcagatac caaatactgt ccttctagtg tagccgtagt taggccacca cttcaagaac 6480
   tctgtagcac cgcctacata cctcgctctg ctaatcctgt taccagtggc tgctgccagt 6540
   ggcgataagt cgtgtcttac cgggttggac tcaagacgat agttaccgga taaggcgcag 6600
   cggtcgggct gaacgggggg ttcgtgcaca cagcccagct tggagcgaac gacctacacc 6660
   gaactgagat acctacagcg tgagctatga gaaagcgcca cgcttcccga agggagaaag 6720
   gcggacaggt atccggtaag cggcagggtc ggaacaggag agcgcacgag ggagcttcca 6780
   gggggaaacg cctggtatct ttatagtcct gtcgggtttc gccacctctg acttgagcgt 6840
   cgatttttgt gatgctcgtc aggggggcgg agcctatgga aaaacgccag caacgcggcc 6900
   tttttacggt tcctggcctt ttgctggcct tttgctcaca tgttctttcc tgcgttatcc 6960
   cctgattctg tggataaccg tattaccgcc tttgagtgag ctgataccgc tcgccgcagc 7020
   cgaacgaccg agcgcagcga gtcagtgagc gaggaagcgg aagagcgccc aatacgcaaa 7080
   ccgcctctcc ccgcgcgttg gccgattcat taatgcagct ggcacgacag gtttcccgac 7140
   tggaaagcgg gcagtgagcg caacgcaatt aatgtgagtt agctcactca ttaggcaccc 7200
   caggctttac actttatgct tccggctcgt atgttgtgtg gaattgtgag cggataacaa 7260
   tttcacacag gaaacagcta tgaccatgat tacgccaagc tagcccgggc tagcttgcat 7320
   gcctgcaggt tt                                                     7332
<210> 18
   <211> 5878
   <212> DNA
   <213> Artificial Sequence
<220>
   <223> Description of Artificial Sequence: CAGGS-cre
<400> 18
   ttcgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggact atttacggta aactgcccac ttggcagtac 240
   atcaagtgta tcatatgcca agtacgcccc ctattgacgt caatgacggt aaatggcccg 300
   cctggcatta tgcccagtac atgaccttat gggactttcc tacttggcag tacatctacg 360
   tattagtcat cgctattacc atgggtcgag gtgagcccca cgttctgctt cactctcccc 420
   atctcccccc cctccccacc cccaattttg tatttattta ttttttaatt attttgtgca 480
   gcgatggggg cggggggggg gggggcgcgc gccaggcggg gcggggcggg gcgaggggcg 540
   gggcggggcg aggcggagag gtgcggcggc agccaatcag agcggcgcgc tccgaaagtt 600
   tccttttatg gcgaggcggc ggcggcggcg gccctataaa aagcgaagcg cgcggcgggc 660
   gggagtcgct gcgttgcctt cgccccgtgc cccgctccgc gccgcctcgc gccgcccgcc 720
   ccggctctga ctgaccgcgt tactcccaca ggtgagcggg cgggacggcc cttctcctcc 780
   gggctgtaat tagcgcttgg tttaatgacg gctcgtttct tttctgtggc tgcgtgaaag 840
   ccttaaaggg ctccgggagg gccctttgtg cgggggggag cggctcgggg ggtgcgtgcg 900
   tgtgtgtgtg cgtggggagc gccgcgtgcg gcccgcgctg cccggcggct gtgagcgctg 960
   cgggcgcggc gcggggcttt gtgcgctccg cgtgtgcgcg aggggagcgc ggccgggggc 1020
   ggtgccccgc ggtgcggggg ggctgcgagg ggaacaaagg ctgcgtgcgg ggtgtgtgcg 1080
   tgggggggtg agcagggggt gtgggcgcgg cggtcgggct gtaacccccc cctgcacccc 1140
   cctccccgag ttgctgagca cggcccggct tcgggtgcgg ggctccgtgc ggggcgtggc 1200
   gcggggctcg ccgtgccggg cggggggtgg cggcaggtgg gggtgccggg cggggcgggg 1260
   ccgcctcggg ccggggaggg ctcgggggag gggcgcggcg gccccggagc gccggcggct 1320
   gtcgaggcgc ggcgagccgc agccattgcc ttttatggta atcgtgcgag agggcgcagg 1380
   gacttccttt gtcccaaatc tggcggagcc gaaatctggg aggcgccgcc gcaccccctc 1440
   tagcgggcgc gggcgaagcg gtgcggcgcc ggcaggaagg aaatgggcgg ggagggcctt 1500
   cgtgcgtcgc cgcgccgccg tccccttctc catctccagc ctcggggctg ccgcaggggg 1560
   acggctgcct tcggggggga cggggcaggg cggggttcgg cttctggcgt gtgaccggcg 1620
   gctctagtaa gcgttggggt gagtactccc tctcaaaagc gggcatgact tctgcgctaa 1680
   gattgtcagt ttccaaaaac gaggaggatt tgatattcac ctggcccgcg gtgatgcctt 1740
   tgagggtggc cgcgtccatc tggtcagaaa agacaatctt tttgttgtca agcttgaggt 1800
   gtggcaggct tgagatctgg ccatacactt gagtgacatt gacatccact ttgcctttct 1860
   ctccacaggt gtccactccc agggcggcct cgaccatgcc caagaagaag aggaaggtgt 1920
   ccaatttact gaccgtacac caaaatttgc ctgcattacc ggtcgatgca acgagtgatg 1980
   aggttcgcaa gaacctgatg gacatgttca gggatcgcca ggcgttttct gagcatacct 2040
   ggaaaatgct tctgtccgtt tgccggtcgt gggcggcatg gtgcaagttg aataaccgga 2100
   aatggtttcc cgcagaacct gaagatgttc gcgattatct tctatatctt caggcgcgcg 2160
   gtctggcagt aaaaactatc cagcaacatt tgggccagct aaacatgctt catcgtcggt 2220
   ccgggctgcc acgaccaagt gacagcaatg ctgtttcact ggttatgcgg cggatccgaa 2280
   aagaaaacgt tgatgccggt gaacgtgcaa aacaggctct agcgttcgaa cgcactgatt 2340
   tcgaccaggt tcgttcactc atggaaaata gcgatcgctg ccaggatata cgtaatctgg 2400
   catttctggg gattgcttat aacaccctgt tacgtatagc cgaaattgcc aggatcaggg 2460
   ttaaagatat ctcacgtact gacggtggga gaatgttaat ccatattggc agaacgaaaa 2520
   cgctggttag caccgcaggt gtagagaagg cacttagcct gggggtaact aaactggtcg 2580
   agcgatggat ttccgtctct ggtgtagctg atgatccgaa taactacctg ttttgccggg 2640
   tcagaaaaaa tggtgttgcc gcgccatctg ccaccagcca gctatcaact cgcgccctgg 2700
   aagggatttt tgaagcaact catcgattga tttacggcgc taaggatgac tctggtcaga 2760
   gatacctggc ctggtctgga cacagtgccc gtgtcggagc cgcgcgagat atggcccgcg 2820
   ctggagtttc aataccggag atcatgcaag ctggtggctg gaccaatgta aatattgtca 2880
   tgaactatat ccgtaacctg gatagtgaaa caggggcaat ggtgcgcctg ctggaagatg 2940
   gcgattagcc attaacgcgt aaatgattgc agatccacta gttctagggc cgcgtcgacc 3000
   tcgagatcca ggcgcggatc aataaaagat cattattttc aatagatctg tgtgttggtt 3060
   ttttgtgtgc cttgggggag gggaggccag aatgaggcgc ggccaagggg ggagggggag 3120
   gccagaatga ccttgggggg ggggaggccg agaatgacct tgggggaggg ggaggccaga 3180
   atgaggcgcg cccccgggta ccgagctcga attcactggc cgtcgtttta caacgtcgtg 3240
   actgggaaaa ccctggcgtt acccaactta atcgccttgc agcacatccc cctttcgcca 3300
   gctggcgtaa tagcgaagag gcccgcaccg atcgcccttc ccaacagttg cgcagcctga 3360
   atggcgaatg gcgcctgatg cggtattttc tccttacgca tctgtgcggt atttcacacc 3420
   gcatatggtg cactctcagt acaatctgct ctgatgccgc atagttaagc cagccccgac 3480
   acccgccaac acccgctgac gcgccctgac gggcttgtct gctcccggca tccgcttaca 3540
   gacaagctgt gaccgtctcc gggagctgca tgtgtcagag gttttcaccg tcatcaccga 3600
   aacgcgcgag acgaaagggc ctcgtgatac gcctattttt ataggttaat gtcatgataa 3660
   taatggtttc ttagacgtca ggtggcactt ttcggggaaa tgtgcgcgga acccctattt 3720
   gtttattttt ctaaatacat tcaaatatgt atccgctcat gagacaataa ccctgataaa 3780
   tgcttcaata atattgaaaa aggaagagta tgagtattca acatttccgt gtcgccctta 3840
   ttcccttttt tgcggcattt tgccttcctg tttttgctca cccagaaacg ctggtgaaag 3900
   taaaagatgc tgaagatcag ttgggtgcac gagtgggtta catcgaactg gatctcaaca 3960
   gcggtaagat ccttgagagt tttcgccccg aagaacgttt tccaatgatg agcactttta 4020
   aagttctgct atgtggcgcg gtattatccc gtattgacgc cgggcaagag caactcggtc 4080
   gccgcataca ctattctcag aatgacttgg ttgagtactc accagtcaca gaaaagcatc 4140
   ttacggatgg catgacagta agagaattat gcagtgctgc cataaccatg agtgataaca 4200
   ctgcggccaa cttacttctg acaacgatcg gaggaccgaa ggagctaacc gcttttttgc 4260
   acaacatggg ggatcatgta actcgccttg atcgttggga accggagctg aatgaagcca 4320
   taccaaacga cgagcgtgac accacgatgc ctgtagcaat ggcaacaacg ttgcgcaaac 4380
   tattaactgg cgaactactt actctagctt cccggcaaca attaatagac tggatggagg 4440
   cggataaagt tgcaggacca cttctgcgct cggcccttcc ggctggctgg tttattgctg 4500
   ataaatctgg agccggtgag cgtgggtctc gcggtatcat tgcagcactg gggccagatg 4560
   gtaagccctc ccgtatcgta gttatctaca cgacggggag tcaggcaact atggatgaac 4620
   gaaatagaca gatcgctgag ataggtgcct cactgattaa gcattggtaa ctgtcagacc 4680
   aagtttactc atatatactt tagattgatt taaaacttca tttttaattt aaaaggatct 4740
   aggtgaagat cctttttgat aatctcatga ccaaaatccc ttaacgtgag ttttcgttcc 4800
   actgagcgtc agaccccgta gaaaagatca aaggatcttc ttgagatcct ttttttctgc 4860
   gcgtaatctg ctgcttgcaa acaaaaaaac caccgctacc agcggtggtt tgtttgccgg 4920
   atcaagagct accaactctt tttccgaagg taactggctt cagcagagcg cagataccaa 4980
   atactgtcct tctagtgtag ccgtagttag gccaccactt caagaactct gtagcaccgc 5040
   ctacatacct cgctctgcta atcctgttac cagtggctgc tgccagtggc gataagtcgt 5100
   gtcttaccgg gttggactca agacgatagt taccggataa ggcgcagcgg tcgggctgaa 5160
   cggggggttc gtgcacacag cccagcttgg agcgaacgac ctacaccgaa ctgagatacc 5220
   tacagcgtga gctatgagaa agcgccacgc ttcccgaagg gagaaaggcg gacaggtatc 5280
   cggtaagcgg cagggtcgga acaggagagc gcacgaggga gcttccaggg ggaaacgcct 5340
   ggtatcttta tagtcctgtc gggtttcgcc acctctgact tgagcgtcga tttttgtgat 5400
   gctcgtcagg ggggcggagc ctatggaaaa acgccagcaa cgcggccttt ttacggttcc 5460
   tggccttttg ctggcctttt gctcacatgt tctttcctgc gttatcccct gattctgtgg 5520
   ataaccgtat taccgccttt gagtgagctg ataccgctcg ccgcagccga acgaccgagc 5580
   gcagcgagtc agtgagcgag gaagcggaag agcgcccaat acgcaaaccg cctctccccg 5640
   cgcgttggcc gattcattaa tgcagctggc acgacaggtt tcccgactgg aaagcgggca 5700
   gtgagcgcaa cgcaattaat gtgagttagc tcactcatta ggcaccccag gctttacact 5760
   ttatgcttcc ggctcgtatg ttgtgtggaa ttgtgagcgg ataacaattt cacacaggaa 5820
   acagctatga ccatgattac gccaagctag cccgggctag cttgcatgcc tgcaggtt   5878

## Description

The present invention relates to an *ex-vivo* method that enables constitutive and inducible gene knock down in non-human vertebrates using a short hairpin RNA expression vector integrated into the genome of the vertebrate.

### Background of the Invention

RNA interference (RNAi) has been discovered some years ago as a tool for inhibition of gene expression (Fire, A. et al., Nature 391, 806-811 (1998)). It based on the introduction of double stranded RNA (dsRNA) molecules into cells, whereby one strand is complementary to the coding region of a target gene. Through pairing of the specific mRNA with the introduced RNA molecule, the mRNA is degraded by a cellular mechanism. Since long dsRNA provokes an interferon response in mammalian cells, the technology was initially restricted to organisms or cells showing no interferon response (Bass, B.L., Nature 411, 428-429 (2001)). The finding that *short* (<30 bp) *interfering RNAs* (siRNA) circumvent the interferon response extended the application to mammalian cells (Elbashir, S.M. et al., Nature 411, 494-498 (2001)).

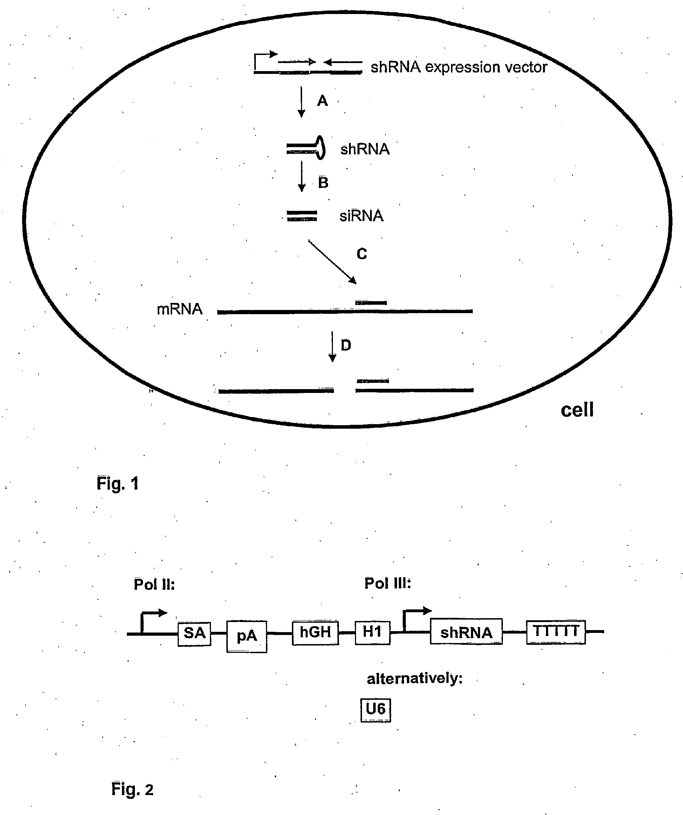

Although RNAi in mice has been in principle demonstrated, the current technology does not allow performing systematic gene function analysis *in vivo*. So far the inhibition of gene expression has been achieved by injection of *purified* siRNA into the tail vain of mice (McCaffrey, A.P. et al., Nature 418, 38-39 (2002); Lewis, D.H. et al., Nature Genet. 32, 107-108 (2002)). Using this approach, gene inhibition is restricted to specific organs and persists only a few days. A further improvement of the siRNA technology is based on the intracellular transcription of short hairpin RNA (shRNA) molecules using gene expression vectors (see Fig. 1; Brummelkamp, T.R. et al., Science 296, 550-553 (2002); Paddison, P.J. et al, Genes Dev. 16, 948-958 (2002); Yu, J.Y. et al., Proc. Natl. Acad. Sci. USA 99, 6047-6052 (2002); Sui, G. et al., Proc. Natl. Acad. Sci. USA 99, 5515-5520 (2002); Paul, C.P. et al., Nature Biotechnol. 20, 505-508 (2002); Xia, H. et al., Nat. Biotechnol. 10, 1006-10 (2002); Jacque, J.M. et al., Nature 418(6896):435-8 ( 2002)). The activity of shRNA in mice has been demonstrated by McCaffrey et al., 2002 through injection of shRNA expression vectors into the tail vain. Again, gene inhibition was temporally and spatially restricted. Although these results demonstrate that the mechanism of shRNA mediated gene silencing is functional in mice, they do not clarify whether constitutive RNAi can be achieved in transgenic animals. Brummelkamp, T.R. et al., Science 296, 550-553 (2002), Paddison, P.J. et al., Genes Dev. 16, 948-958 (2002), Hemann, M.T. et al., Nat. Genet. 33(3):396-400 (2003); and Devroe, E. et al., BMC Biotechnol. 2(1):15 (2002) have shown the long-term inhibition of gene expression through stable integration of shRNA vectors in cultivated cell lines. These experiments included random integration of shRNA transgenes and screening for clones with appropriate siRNA expression, which is not applicable for testing of a large number of different shRNA transgenes in mice. Finally, several reports have demonstrated shRNA-mediated gene silencing in transgenic mice and rats (Hasuwa, H. et al., FEBS Lett. 532(1-2):227=30 (2002); Carmell, M.A. et al., Nat. Struct. Biol. 10(2):91-2 (2003); Rubinson, D.A. et al., Nat. Genet. 33(3):401-6 (2003); Kunath, T. et al., Nat. Biotechnol. (Apr. 7 2003)). However, these experiments again included random integration of shRNA transgenes resulting in variable levels and patterns of shRNA expression. Thus, testing of ES cell clones or mouse lines with appropriate shRNA expression had been required, which is a laborious and time-consuming undertaking.

The *in vivo* validation of genes by RNAi mediated gene repression in a large scale setting requires the expression of siRNA at sufficiently high levels and with a predictable pattern in multiple organs. Targeted transgenesis provides the only approach to achieve reproducible expression of transgenes in the living organism (e.g. mammalians such as mice). It has been, however, questionable whether a single copy of a siRNA expression vector integrated into the genome would result in sufficiently high levels of siRNA required for RNAi-mediated gene inhibition in multiple organs of the living organism.

Most siRNA expression vectors are based on polymerase III dependent (Pol III) promoters (U6 or H1) that allow the production of transcripts carrying only a few non-homologous bases at their 3' ends. It has been shown that the presence of non-homologous RNA at the ends of the shRNA stretches lower the efficiency of RNAi mediated gene silencing (Xia et al., Nat. Biotechnol. 10, 1006-10 (2002)). However, it has been unpredictable for a person skilled in the art which genomic region within the living organism promotes an appropriate expression pattern of the Pol III promoter driven shRNA constructs required for RNAi-mediated gene inhibition in multiple organs of the living organism.

For the temporal control of RNAi mediated gene silencing in transgenic cells lines and living organism, a tight system for inducible siRNA expression is needed. Inducible gene expression systems based on the tetracycline dependent repressor are known. It is, however, not obvious whether a stably integrated, single copy configuration of these systems can be created that allows inducible RNAi in multiple organs without background activity.

### Summary of the Invention

It has now been found that the modification of murine embryonic stem (ES) cells by the introduction of shRNA expression vectors through targeted transgenesis allows the inhibition of gene expression in mice generated from these modified ES cells. The invention thus provides
(1) an *ex-vivo* method for constitutive and/or inducible gene knock down in a tissue culture or in cells of a cell culture derived from a vertebrate, which comprises stably integrating an expression vector
   comprising a short hairpin RNA (shRNA) construct under control of a ubiquitous promoter selected from polymerase I, II and III dependent promoters,
   being suitable for stable integration into the genome of the tissue culture or into the genome of the cells of the cell culture, and
   containing homologous sequences suitable for integration at a polymerase II dependent locus through homologous recombination in the genome of the tissue culture or in the genome of the cells of the cell culture including embryonic cells
   into the genome of the tissue culture or into the genome of the cells of the cell culture, provided that the method is not applied to human embryonic cells;
(2) a non-human vertebrate, or tissue or cell culture derived from a vertebrate having stably integrated at a polymerase II dependent locus of the genome of the vertebrate, tissue culture or cells of the cell culture, an expression vector as defined in (1) above, provided that said tissue or cell culture does not include human embryonic cells; and
(3) an expression vector as defined in (1) above.

### Short Description of the Figures

Fig. 1: Mechanism of RNAi through shRNA expression. A: expression of shRNA. B: shRNA processing. C: Pairing of siRNA with complementary mRNA strands. D: Cleavage of mRNA.
Fig 2: Pol III dependent shRNA expression vector (constitutive). Insertion of an shRNA expression vector into a ubiquitously expressed genomic locus. Transcription through the upstream Pol II dependent promoter will be stopped by a synthetic polyadenylation signal (pA) and the hGH pA. The Pol III dependent promoter controls the expression of shRNA. The transcript is stopped by five thymidine bases. The exact sequence of suitable target vectors is given in SEQ ID Nos:6 and 8.

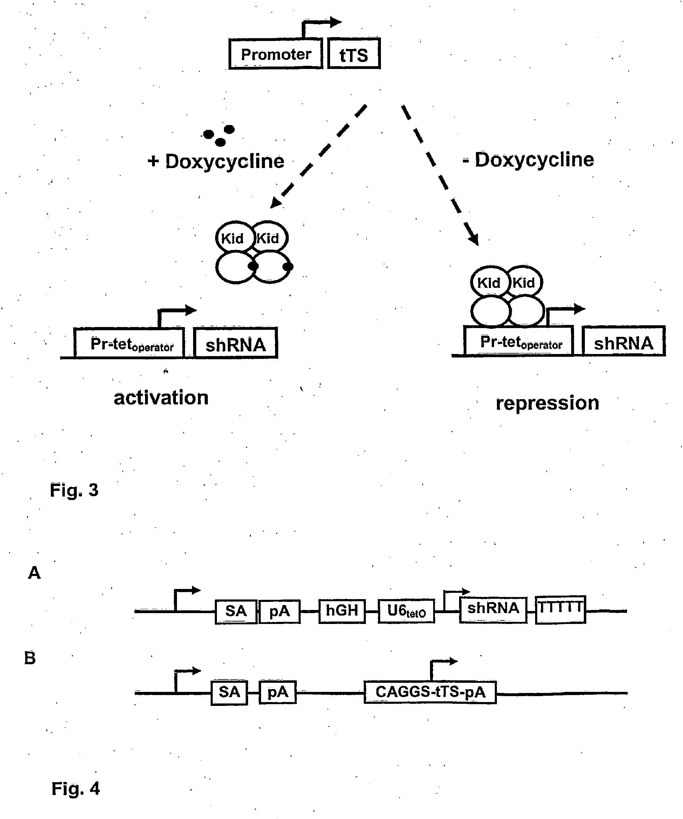

Fig. 3: Tet repression system. tTS is a doxycycline controlled transcriptional silencer consisting of the tet repressor and the KRAB-AB domain of the Kid-1 protein. This fusion protein binds to the pol III dependent promoter via the tet operator sequences only in the absence of doxycycline and represses transcription.
Fig. 4: Vectors for Pol III dependent promoter based tet-repression system (inducible). Insertion of a shRNA expression vector into a ubiquitous expressed genomic locus. The transcription of the Pol II dependent promoter will be stopped by the synthetic polyadenylation signal (pA) and a hGH pA. An inducible Pol III dependent promoter controls the expression of shRNA. The transcript is stopped by five thymidine bases.

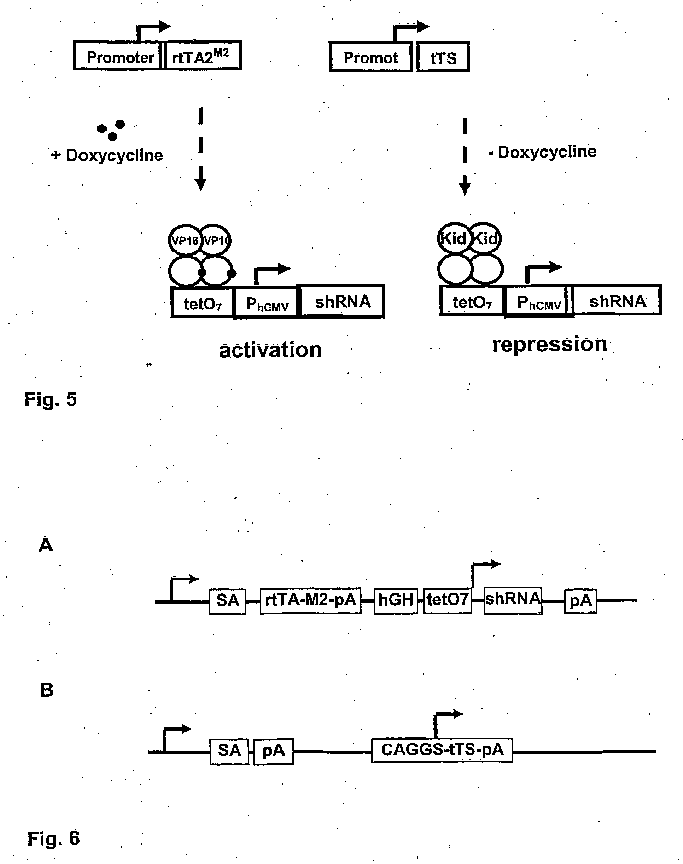

Fig. 5: Principle of the Doxycycline inducible gene expression system. rtTA2M2 is a doxycycline-controlled transcriptional activator consisting of the reverse tet repressor and the VP16 activation domain of the Herpes simplex virus VP16 protein. This fusion protein binds to a minimal CMV-promoter via the tet operator sequences in the presence of doxycycline and activates transcription, tTS functions in a similar fashion as explained in Fig. 3.
Fig. 6: Vectors for Pol II dependent promoter based tet-system (inducible). Insertion of a shRNA expression vector into a ubiquitous expressed genomic locus. The rtTA2M2 is expressed under the control of the ubiquitous Pol II dependent promoter, stopped by a synthetic polyadenylation signal (pA) and a hGH pA. An inducible Pol II dependent promoter controls the expression of shRNA. The transcript is stopped by a short polyadenylation signal.

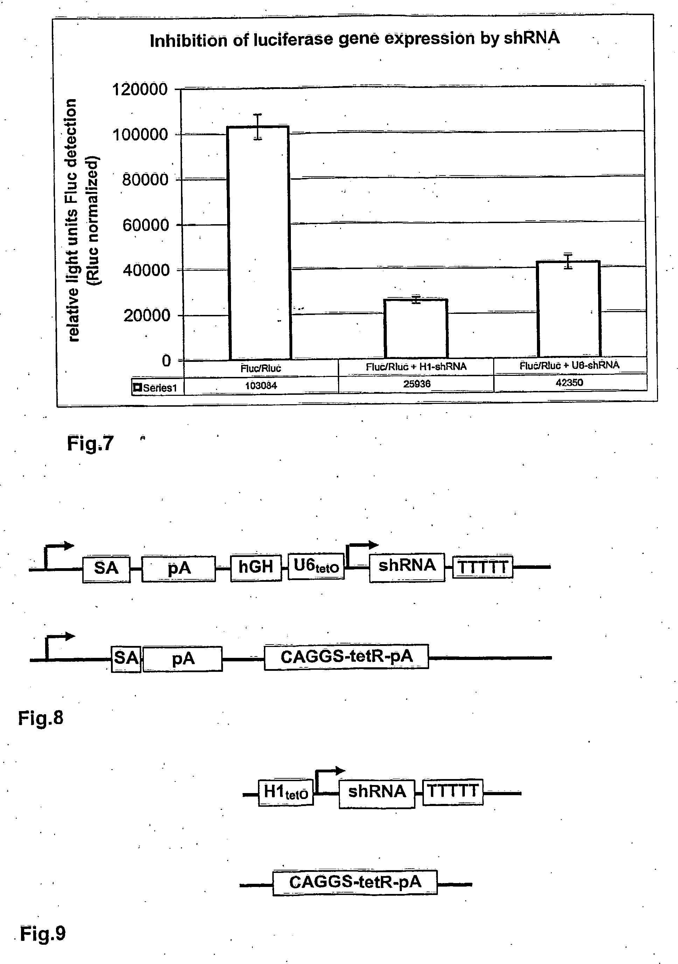

Figure 7: ShRNA-mediated inhibition of luciferase gene expression in embryonic stem cells. All cells carried stably integrated expression cassettes of firefly= (Fluc) and Renilla luciferases (Rluc). Simultaneous expression of a Fluc-specific siRNA under control of the H1- (H1-shRNA) or U6-promoter (U6-shRNA) resulted in a ∼75% and ∼60% reduction of firefly luciferase activity, respectively. The values of Fluc activity were normalized by using the Rluc activity for reference.
Fig. 8: Vectors for a U6 promoter based tet-repression system (inducible). Transcription of the Pol II promoter will be stopped by the synthetic polyadenylation signal (pA) and a hGH pA sequence. The inducible U6 promoter controls expression of the Fluc-specific shRNA. Transcription of the shRNA is stopped by five thymidine bases. TetR is a doxycycline controlled repressor (Gossen and Bujard, PNAS. 89: 5547-5551 (1992)) expressed by the CAGGS-promoter. The tetR binds to the pol III promoter via the tet operator sequences only in the absence of doxycycline and blocks transcription.
Fig. 9: Vectors for H1 promoter based tet-repression system (inducible). The inducible H1 promoter controls the expression of shRNA. TetR is a doxycycline controlled repressor expressed by the CAGGS-promoter. The tetR binds to the pol III promoter via the tet operator sequences only in the absence of doxycycline and blocks transcription.

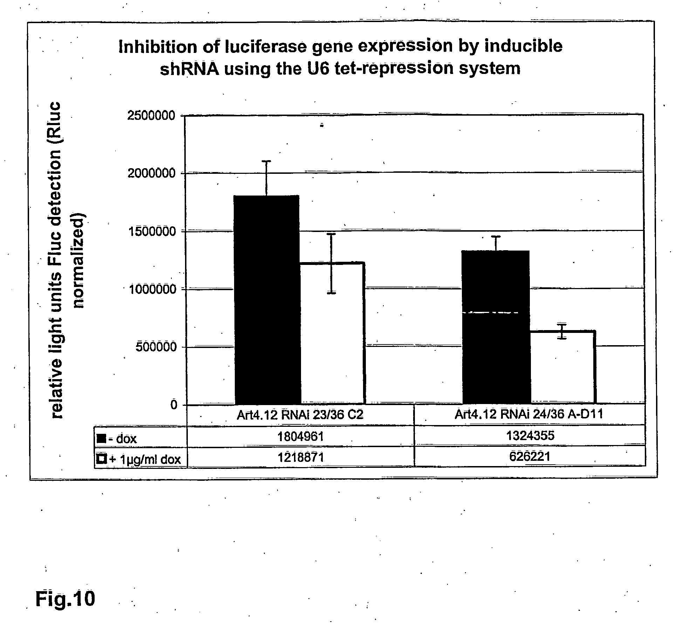

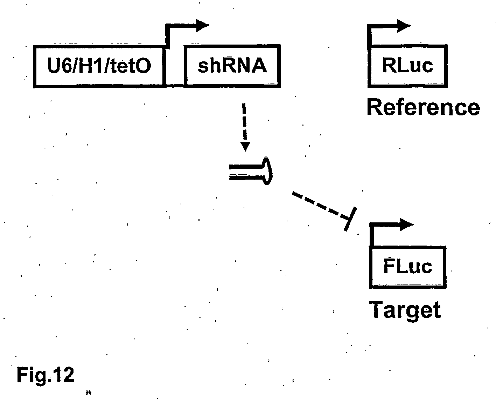

Figure 10: Inducible RNA interference in embryonic stem cells using tetracycline dependent U6 promoters. All cells carried stably integrated expression cassettes of firefly luciferase (Fiuc), Renilla luciferase (Rluc), and the tet repressor. Doxycycline-inducible expression of a Fluc-specific shRNA under control of the U6_{tetO1}- (Art4.12 RNAi 23/36 C2) or U6_{tetO2}-promoter (Art4.12 RNAi 24/36 A-D11) resulted in a ∼30% and ∼50% reduction of firefly luciferase activity, respectively. The values of Fluc activity were normalized by using the Rluc activity for reference (figure 12).

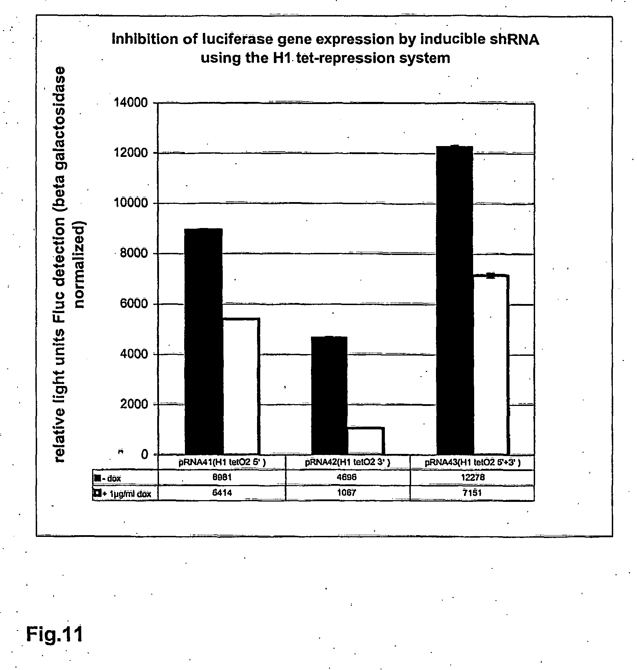

Figure 11: Inducible RNA interference using tetracycline- dependent H1 promoters NIH3T3. Cells were transiently transfected with firefly luciferase. (Fluc), tet repressor, LacZ, and shRNA expression vectors. Doxycycline-inducible expression of the Fluc-specifc shRNA under control of the H1_{tetO2/5'}-, H1_{tetO2/3'}-, or H1_{tetO2/5'+3'}-promoter resulted in a ∼40%, ∼80%, and ∼40% reduction of firefly luciferase activity, respectively. The values of Fluc activity were normalized by using the LacZ activity for reference.
Figure 12: Experimental strategy. Genes of the firefly and Renilla luciferases are expressed through the endogenous rosa26 promoter via splice acceptor sites. Specific shRNA mediated repression of the firefly luciferase gene can be measured by using the Renilla luciferase activity as a reference.

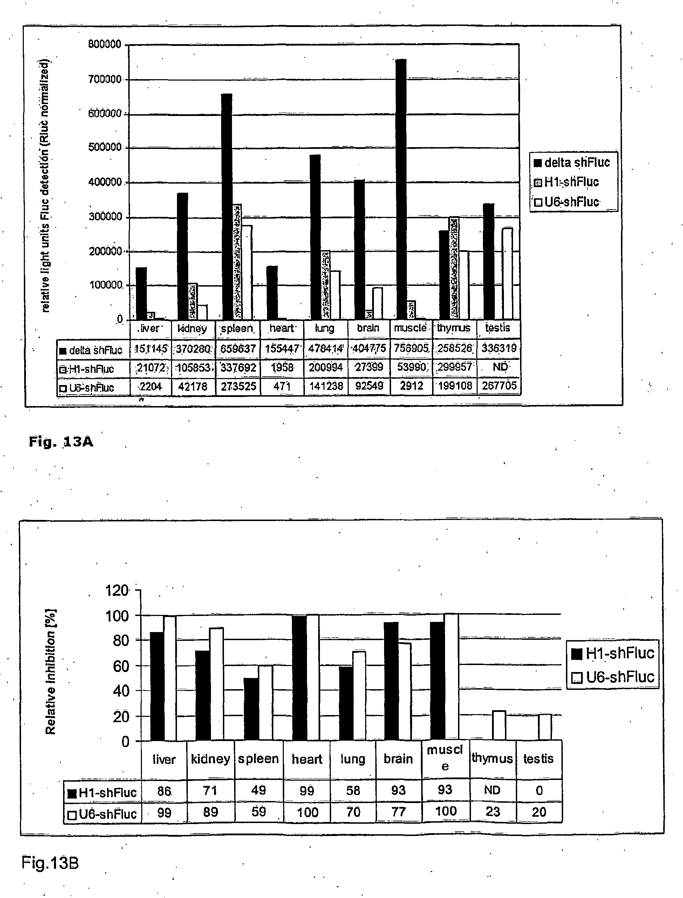

Figure 13: ShRNA-mediated inhibition of luciferase expression in mice.
   A: Firefly luciferase activity in mice in the absence (delta shFluc, black bars) or presence of the U6- (U6-shFluc, white bars) and H1-shRNA transgenes (H1-shFluc, grey bars), respectively. All mice carried the firefly and the Renilla luciferase transgenes. Relative values of Firefly luciferase activitiy in different organs are given as indicated. All values of Fluc activity were normalized by using the Rluc activity for reference.
   B: Efficiency of shRNA-mediated repression of firefly luciferase expression in mice. Percentages of U6- (white bars) and H1-shRNA mediated (grey bars) repression of Firefly luciferase activity are shown. ND: not determined.

### Detailed Description of the Invention

The terms "vertebrates" and "non-human vertebrates" according to the present invention relates to living multi-cell organisms such as mammals (e.g. non-human animals such as rodents (including mice and rats) and humans) or non-mammals (e.g. fish). Most preferred vertebrates are mice and fish.

"Tissue culture" according to the present invention refers to parts of the above defined vertebrates (including organs) which are cultured *in vitro*.

"Cell culture" according to the present invention includes cells isolated from the above defined vertebrates which are cultured *in vitro.* These cells can be transformed (immortalized) or untransformed (directly derived from living organisms; primary cell culture).

The expression vector according to the invention of the present application is suitable for stable integration into the vertebrates or into cells of the cell culture (vectors for transient integration are also contemplated). It contains homologous sequences suitable for targeted integration at a polymerase II dependent locus of the living organisms or cells of the cell culture. Such polymerase II dependent loci include, but are not limited to, Rosa26 locus (the murine Rosa26 locus being depicted in SEQ ID NO:1), collagen, RNA polymerase, actin and HPRT.

The expression vector may further contain functional sequences selected from splice acceptor sequences (such as a splice acceptor of adenovirus), polyadenylation sites (such as synthetic polyadenylation sites, the polyadenylation site of human growth hormones), selectable marker sequences (such as the neomycin phosphotransferase gene of *E. coli* transposon).

The ubiquitous promoter in the vector according to the invention is preferably selected from polymerase I, II and III dependent promoters, preferably is a polymerase II or III dependent promoter including, but not limited to, a CMV promoter, a CAGGS promoter, a snRNA promoter such as U6, a RNAse P RNA promoter such as H1, a tRNA promoter, a 7SL RNA promoter, a 5 S rRNA promoter.
The ubiquitous promoter can be a constitutive promoter, or can be an inducible promoter. Suitable inducible promoters are the above-mentioned polymerase I, II and III dependent promoters containing an operator sequence including, but not limited to, tet, Gal4 and lac.

The expression vector of the invention is suitable for the following particularly preferred approaches (for constitutive and inducible expression):
A. a Pol III dependent promoter (constitutive U6 or H1) driven shRNA construct (to be integrated into a ubiquitously active Pol II dependent locus (see Fig. 2));
B. a Pol III dependent promoter (inducible U6 or H1) driven shRNA construct (to be integrated into a ubiquitously active Pol II dependent locus (Fig. 3 and 4)); or
C. a polymerase II (Pol II) dependent promoter (inducible CMV) driven shRNA construct (to be integrated into a ubiquitously active Pol II dependent locus (Fig. 5 and 6)).

The shRNA within the vector of the invention preferably comprises
(I) at least one DNA segment A-B-C wherein
   A is a 15 to 35, preferably 19 to 29 bp DNA sequence being at least 95%, preferably 100% complementary to the gene to be knocked down (e.g. firefly luciferase, p53);
   B is a spacer DNA sequence having 5 to 9 bp forming the loop of the expressed RNA hair pin molecule; and
   C is a 15 to 35, preferably 19 to 29 bp DNA sequence being at least 85% complementary to the sequence A; and
(II) a stop and/or polyadenylation sequence.

Suitable shRNA sequences for the knock down of a given target gene are well known in the art (see e.g. the particular shRNA sequences mentioned in Tables 1 and 2 below) or can readily be determined by the skilled artisan.

**Table 1:**

| target | shRNA sequence /SEQ ID NO | Reference |
|---|---|---|
| gene | | |
| CDH-1 | TgagaagtctcccagtcagTTCAAGAGActgactgggagacttctca (19) | Brummelkamp |
| p53 | GactccagtggtaatctacTTCAAGAGAgtagattaccactggagtc (20) | et al., |
| CDC20 | CggcaggactccgggccgaTTCAAGAGAtcggcccggagtcctgccg (21) | Science, 296: |
| | | 550-3 (2002). |
| CYLD | CctcatgcagttctctttgTTCAAGAGAcaaagagaactgcatgagg (22) | Kovalenko et |
| | | al, Nature, |
| | | 424:801-5 |
| | | (2003). |
| Ras- | AagatgaagccactccctatttCAAGAGAaaatagggagtggcttcatctt (23) | Kunath et al., |
| Gap | | Nature |
| | | Biotechnology, |
| | | 21:559-561 |
| | | (2003). |
| tubulin | GacagagccaagtggactcACAgagtccacttggctctgtc (24) | Yu et al., |
| | | PNAS, 99: |
| | | 6047-52 |
| | | (2002) |
| lamin | Ctggacttccagaagaacattcgtgttcttctggaagtccag (25) | Paul et al., |
| | | Nature Bio- |
| | | technology, |
| | | 20:505-8 |
| | | (2002). |

**Table 2 shRNA sequences known from Brummelkamp et al., Nature, 424:797-801 (2003):**

| **Target** | **shRNA Sequence / SEQ ID NO** |
|---|---|
| **Gene** | |
| UBIQUITIN | GAGATTGGTCCAGAACAGTTTCAAGAGAACTGTTCTGGACCAATCTC (26) |
| CARBOXYL- | GCCCTTCCGATCATGGTAGTTCAAGAGACTACCATGATCGGAAGGGC (27) |
| TERMINAL | TCTTTAGAATTCTTAAGTATTCAAGAGATACTTAAGAATTCTAAAGA (28) |
| HYDROLASE 12 | CATTAGCTATATCAACATGTTCAAGAGACATGTTGATATAGCTAATG (29) |
| | |
| UBIQUITIN | ACCACAAACGGCGGAACGATTCAAGAGATCGTTCCGCCGTTTGTGGT (30) |
| CARBOXYL- | GAGGGTCTTGGAGGTCTTCTTCAAGAGAGAAGACCTCCAAGACCCTC (31) |
| TERMINAL | GTCCATGCCCAGCCGTACATTCAAGAGATGTACGGCTGGGCATGGAC (32) |
| HYDROLASE 11 | GCTGGACACCCTCGTGGAGTTCAAGAGACTCCACGAGGGTGTCCAGC (33) |
| | |
| UBIQUITIN | GAATATCAGAGAATTGAGTTTCAAGAGAACTCAATTCTCTGATATTC (34) |
| CARBOXYL- | TGGACTTCATGAGGAAATGTTCAAGAGACATTTCCTCATGAAGTCCA (35) |
| TERMINAL | TATTGAATATCCTGTGGACTTCAAGAGAGTCCACAGGATATTCAATA (36) |
| HYDROLASE 10 | TTGTACTGAGAGAAACTGCTTCAAGAGAGCAGTTTCTCTCAGTACAA (37) |
| | |
| HASP | GATCAATGATAGGTTTGAATTCAAGAGATTCAAACCTATCATTGATC (38) |
| | GGAGTTTGAGAAGTTTAAATTCAAGAGATTTAAACTTCTCAAACTCC (39) |
| | GAACTCCTCGCTTGCTGAGTTCAAGAGACTCAGCAAGCGAGGAGTTC (40) |
| | CCGAATTTAACAGAGAGAATTCAAGAGATTCTCTCTGTTAAATTCGG (41) |
| | |
| UBIQUITIN | GACAGCAGAAGAATGCAGATTCAAGAGATCTGCATTCTTCTGCTGTC (42) |
| CARBOXYL- | ATAAAGCTCAACGAGAACCTTCAAGAGAGGTTCTCGTTGAGCTTTAT (43) |
| TERMINAL | GGTGAAGTGGCAGAAGAATTTCAAGAGAATTCTTCTGCCACTTCACC (44) |
| HYDROLASE8 | GTATTGCAGTAATCATCACTTCAAGAGAGTGATGATTACTGCAATAC (45) |
| | |
| FLJ10785 | GATATGGGGTTCCATGTCATTCAAGAGATGACATGGAACCCCATATC (46) |
| | GGAGACATGGTTCTTAGTGTTCAAGAGACACTAAGAACCATGTCTCC (47) |
| | AGCACCAAGTTCGTCTCAGTTCAAGAGACTGAGACGAACTTGGTGCT (48) |
| | GATGCAACACTGAAAGAACTTCAAGAGAGTTCTTTCAGTGTTGCATC (49) |
| | |
| KIAA0710 | GTCAATGGCAGTGATGATATTCAAGAGATATCATCACTGCCATTGAC (50) |
| | CCTGCTAGCTGCCTGTGGCTTCAAGAGAGCCACAGGCAGCTAGCAGG (51) |
| | CCACCTTTGCCAGAAGGAGTTCAAGAGACTCCTTCTGGCAAAGGTGG (52) |
| | CCCTATTGAGGCAAGTGTCTTCAAGAGAGACACTTGCCTCAATAGGG (53) |
| | |
| FLJU12552/ | GAAGGAAAACTTGCTGACGTTCAAGAGACGTCAGCAAGTTTTCCTTC (54) |
| FLJ14256 | CTCACCTGGGTCCATGAGATTCAAGAGATCTCATGGACCCAGGTGAG (55) |
| | GCTGTCTTACCGTGTGGTCTTCAAGAGAGACCACACGGTAAGACAGC (56) |
| | CCTGGACCGCATGTATGACTTCAAGAGAGTCATACATGCGGTCCAGG (57) |
| | |
| KIAA1203 | GTCAATGGCAGTGATGATATTCAAGAGATATCATCACTGCCATTGAC (58) |
| | CCTGCTAGCTGCCTGTGGCTTCAAGAGAGCCACAGGCAGCTAGCAGG (59) |
| | CCACCTTTGCCAGAAGGAGTTCAAGAGACTCCTTCTGGCAAAGGTGG (60) |
| | CCCTATTGAGGCAAGTCTCTTCAAGAGAGACACTTGCCTCAATAGGG (61) |
| | |
| FLJ23277 | GGAAATCCGAATTGCTTGGTTCAAGAGACCAAGCAATTCGGATTTCC (62) |
| | CACATTTCTTCAAGTGTGGTTCAAGAGACCACACTTGAAGAAATGTG (63) |
| | CAGCAGGATGCTCAAGAATTTCAAGAGAATTCTTGAGCATCCTGCTG (64) |
| | GCTGAATACCTACATTGGCTTCAAGAGAGCCAATGTAGGTATTCAGC (65) |
| | |
| FLJ14914 (similar | GGGCTTGTGCCTGGCCTTGTTCAAGAGACAAGGCCAGGCACAAGCCC (66) |
| to UBP4) | GCCTTGTCCTGCCAAGAAGTTCAAGAGACTTCTTGGCAGGACAAGGC (67) |
| | GATTGAAGCCAAGGGAACGTTCAAGAGACGTTCCCTTGGCTTCAATC (68) |
| | TGGCGCCTGCTCCCCATCTTTCAAGAGAAGATGGGGAGCAGGCGCCA (69) |
| | |
| UBIQUITIN CAR- | GAACCAGCAGGCTCTGTGGTTCAAGAGACCACAGAGCCTGCTGGTTC (70) |
| BOXYL-TERMINAL | GGAAGCATAATTATCTGCCTTCAAGAGAGGCAGATAATTATGCTTCC (71) |
| HYDROLASE | AGAAGAAGATGCTTTTCACTTCAAGAGAGTGAAAAGCATCTTCTTCT (72) |
| ISOZYME L5 | CTTGCAGAGGAGGAACCCATTCAAGAGATGGGTTCCTCCTCTGCAAG (73)' |
| | |
| UBIQUITIN CAR- | GCAAACAATCAGCAATGCCTTCAAGAGAGGCATTGCTGATTGTTTGC (74) |
| BOXYL-TERMIIVAL | TTGGACTGATTCATGCTATTTCAAGAGAATAGCATGAATCAGTCCAA (75) |
| HYDROLASE | CTGGCAATTCGTTGATGTATTCAAGAGATACATCAACGAATTGCCAG (76) |
| ISOZYME L3 | TTAGATGGGCGGAAGCCATTTCAAGAGAATGGCTTCCGCCCATCTAA (77) |
| | |
| UBIQUITIN CAR- | GAGGAGTCTCTGGGCTCGGTTCAAGAGACCGAGCCCAGAGACTCCTC (78) |
| BOXYL-TERMINAL | GAGCTGAAGGGACAAGAAGTTCAAGAGACTTCTTGTCCCTTCAGCTC (79) |
| HYDROLASE | TGTCGGGTAGATGACAAGGTTCAAGAGACCTTGTCATCTACCCGACA (80). |
| ISOZYME L1 | CACAGCTGTTCTTCTGTTCTTCAAGAGAGAACAGAAGAACAGCTGTG (81) |
| | |
| KIAA1891 / | GTGGAAGCCTTTACAGATCTTCAAGAGAGATCTGTAAAGGCTTCCAC (82) |
| FU25263 | CAACAGCTGCCTTCATCTGTTCAAGAGACAGATGAAGGCAGCTGTTG (83) |
| | CCATAGGCAGTCCTCCTAATTCAAGAGATTAGGAGGACTGCCTATGG (84) |
| | TGTATCACTGCCACTGGTTTTAAGAGAAACCAGTGGCAGTGATACA (85) |
| | |
| FLJ14528 (similar | CATGTTGGGCAGCTGCAGCTTCAAGAGAGCTGCAGCTGCCCAACATG (86) |
| to UBP8) | CACAACTGGAGACCTGAAGTTCAAGAGACTTCAGGTCTCCAGTTGTG (87) |
| | GTATGCCTCCAAGAAAGAGTTCAAGAGACTCTTTCTTGGAGGCATAC (88) |
| | CTTCACAGTACATTTCTCTTTCAAGAGAAGAGAAATGTACTGTGAAG (89) |
| | |
| U4/U6 TRI SNRNP | GTACTTTCAAGGCCGGGGTTTCAAGAGAACCCCGGCCTTGAAAGTAC (90) |
| 65 kDa protein | CTTGGACAAGCAAGCCAAATTCAAGAGATTTGGCTTGCTTGTCCAAG (91) |
| | GACTATTGTGACTGATGTTTTCAAGAGAAACATCAGTCACAATAGTC (92) |
| | GGAGAACTTTCTGAAGCGCTTCAAGAGAGCGCTTCAGAAAGTTCTCC (93) |
| | |
| XM_089437 | GACGAGAGAAACCTTCACCTTCAAGAGAGGTGAAGGTTTCTCTCGTC (94) |
| | ACATTATTCTACATTCTTTTTCAAGAGAAAAGAATGTAGAATAATGT (95) |
| | AGATTCGCAAATGGATGTATTCAAGAGATACATCCATTTGCGAATCT (96) |
| | CATTCCCACCATGAGTCTGTTCAAGAGACAGACTCATGGTGGGAATG (97) |
| | |
| KIAA1453 | GATCGCCCGACACTTCCGCTTCAAGAGAGCGGAAGTGTCGGGCGATC (98) |
| | CCAGCAGGCCTACGTGCTGTTCAAGAGACAGCACGTAGGCCTGCTGG (99) |
| | GCCAGCTCCTCCACAGCACTTCAAGAGAGTGCTGTGGAGGAGCTGGC (100) |
| | CGCCGCCAAGTGGAGCAGATTCAAGAGATCTGCTCCACTTGGCGGCG (101) |
| | |
| FLJ12697 | GAAGATGCCCATGAATTCCTTCAAGAGAGGAATTCATGGGCATCTTC (102) |
| | CAAACAGGCTGCGCCAGGCTTCAAGAGAGCCTGGCGCAGCCTGTTTC (103) |
| | ACGGCCTAGCGCCTGATGGTTCAAGAGACCATCAGGCGCTAGGCCGT (104) |
| | CTGTAACCTCTCTGATCGGTTCAAGAGACCGATCAGAGAGGTTACAG (105) |
| | |
| UBIQUITIN | TCTGTCAGTCCATCCTGGCTTCAAGAGAGCCAGGATGGACTGACAGA (106) |
| SPECIFIC | TGAAGCGAGAGTCTTGTGATTCAAGAGATCACAAGACTCTCGCTTCA (107) |
| PROTEASE 18 | GATGGAGTGCTAATGGAAATTCAAGAGATTTCCATTAGCACTCCATC (108) |
| (USP18) | CCTTCAGAGATTGACACGCTTCAAGAGAGCGTGTCAATCTCTGAAGG (109) |
| | |
| UBIQUITIN | CCTGACCACGTTCCGACTGTTCAAGAGACAGTCGGAACGTGGTCAGG (110) |
| CARBOXYL- | GAGTTCCTTCGCTGCCTGATTCAAGAGATCAGGCAGCGAAGGAACTC (111) |
| TERMINAL | GACTGCCTTGCTGCCTTCTTTCAAGAGAAGAAGGCAGCAAGGCAGTC (112) |
| HYDROLASE 20 | CGCCGAGGGCTACGTACTCTTCAAGAGAGAGTACGTAGCCCTCGGCG (113) |
| | |
| UBIQUITIN | GGCGAGAAGAAAGGACTGTTTCAAGAGAACAGTCCTTTCTTCTCGCC (114) |
| CARBOXYL- | GGACGAGAATTGATAAAGATTCAAGAGATCTTTATCAATTCTCGTCC (115) |
| TERMINAL | GCACGAGAATTTGGGAATCTTCAAGAGAGATTCCCAAATTCTCGTGC (116) |
| HYDROLASE 24 | CTACTTCATGAAATATTGGTTCAAGAGACCAATATTTCATGAAGTAG (117) |
| | |
| KIAA1594 | GATAACAGCTTCTTGTCTATTCAAGAGATAGACAAGAAGCTGTTATC (118) |
| | GAGAATAGGACATCAGGGCTTCAAGAGAGCCCTGATGTCCTATTCTG (119) |
| | CTTGGAAGACTGAACCTGTTTCAAGAGAACAGGTTCAGTCTTCCAAG (120) |
| | CAACTCCTTTGTGGATGCATTCAAGAGATGCATCCACAAAGGAGTTG (121) |
| | |
| KIAA1350 | GATGTTGTCTCCAAATGCATTCAAGAGATGCATTTGGAGACAACATC (122) |
| | CGTGGGGACTGTACCTCCCTTCAAGAGAGGGAGGTACAGTCCCCACG (123) |
| | GTACAGCTTCAGAACCAAGTTCAAGAGACTTGGTTCTGAAGCTGTAC (124) |
| | |
| UBIQUITIN | GATGATCTTCAGAGAGCAATTCAAGAGATTGCTCTCTGAAGATCATC (125) |
| CARBOXYL- | GGAACATCGGAATTTGCCTTTCAAGAGAAGGCAAATTCCGATGTTCC (126) |
| TERMINAL | GAGCTAGTGAGGGACTCTTTTCAAGAGAAAGAGTCCCTCACTAGCTC (127) |
| HYDROLASE 25 | GGAGGGTTCTTTAAGGCAATTCAAGAGATTGCCTTAAAGAACCCTGC(128) |
| | |
| UBIQUITIN | TCGATGATTCCTCTGAAACTTCAAGAGAGTTTCAGAGGAATCATCGA (129) |
| CARBOXYL- | GATAATGGAAATATTGAACTTCAAGAGAGTTCAATATTTCCATTATC (130) |
| TERMINAL | GTTCTTCATTTAAATGATATTCAAGAGATATCATTTAAATGAAGAAC (131) |
| HYDROLASE 16 | GTTAACAAACACATAAAGTTTCAAGAGAACTTTATGTGTTTGTTAAC (132) |
| | |
| USP9X | GTTAGAGAAGATTCTTCGTTTCAAGAGAACGAAGAATCTTCTCTAAC (133) |
| | GTTGATTGGACAATTAAACTfCAAGAGAGTTTAATTGTCCAATCAAC (134) |
| | GGTTGATACCGTAAAGCGCTTCAAGAGAGCGCTTTACGGTATCAACC (135) |
| | GCAATGAAACGTCCAATGGTTCAAGAGACCATTGGACGTTTCATTGC (136) |
| | |
| USP9Y | AGCTAGAGAAAATTCTTCGTTCAAGAGACGAAGAATTTTCTCTAGCT (137) |
| | GATCCTATATGATGGATGATTCAAGAGATCATCCATCATATAGGATC (138) |
| | GTTCTTCTTGTCAGTGAAATTCAAGAGATTTCACTGACAAGAAGAAC (139) |
| | CTTGAGCTTGAGTGACCACTTCAAGAGAGTGGTCACTCAAGCTCAAG (140) |
| | |
| UBIQUITIN | GACCGGCCAGCGAGTCTACTTCAAGAGAGTAGACTCGCTGGCCGGTC (141) |
| CARBOXYL- | GGACCTGGGCTACATCTACTTCAAGAGAGTAGATGTAGCCCAGGTCC (142) |
| TERMINAL | CTCTGTGGTCCAGGTGCTCTTCAAGAGAGAGCACCTGGACCACAGAG (143) |
| HYDROLASE 5 | GACCACACGATTTGCCTCATTCAAGAGATGAGGCAAATCGTGTGGTC (144) |
| | |
| UBIQUITIN | TGGCTTGTTTATTGAAGGATTCAAGAGATCCTTCAATAAACAAGCCA (145) |
| CARBOXYL- | GTGAATTTGGGGAAGATAATTCAAGAGATTATCTTCCCCAAATTCAC (146) |
| TERMINAL | CGCTATAGCTTGAATGAGTTTCAAGAGAACTCATTCAAGCTATAGCG (147) |
| HYDROLASE 26 | GATATCCTGGCTCCACACATTCAAGAGATGTGTGGAGCCAGGATATC (148) |
| | |
| KIAA1097 | GAGCCAGTCGGATGTAGATTTCAAGAGAATCTACATCCGACTGGCTC (149) |
| | GTAAATTCTGAAGGCGAATTTCAAGAGAATTCGCCTTCAGAATTTAC (150) |
| | GCCCTCCTAAATCAGGCAATTCAAGAGATTGCCTGATTTAGGAGGGC (151) |
| | GTTGAGAAATGGAGTGAAGTTCAAGAGACTTCACTCCATTTCTCAAC (152) |
| | |
| UBIQUITIN | GCTTGGAAATGCAAGGCGTTCAAGAGACGCCTTGCATTTTCCAAGC (153) |
| SPECIFIC | CTGCATCATAGACCAGATCTTCAAGAGAGATCTGGTCTATGATGCAG (154) |
| PROTEASE 22 | GATCACCACGTATGTGTCCTTCAAGAGAGGACACATACGTGGTGATC (155) |
| (USP22) | TGACAACAAGTATTCCCTGTTCAAGAGACAGGGAATACTTGTTGTCA (156) |
| | |
| UBIQUITIN- | GAAATATAAGACAGATTCCTTCAAGAGAGGAATCTGTCTTATATTTC (157) |
| SPECIFIC | CCCATCAAGTTTAGAGGATTTCAAGAGAATCCTCTAAACTTGATGGG (158) |
| PROCESSING | GGTGTCCCATGGGAATATATTCAAGAGATATATTCCCATGGGACACC (159) |
| PROTEASE 29 | GAATGCCGACCTACAAAGATTCAAGAGATCTTTGTAGGTCGGCATTC (160) |
| | |
| CYLD | CAGTTATATTCTGTGATGTTTCAAGAGAACATCACAGAATATAACTG (161) |
| | GAGGTGTTGGGGACAAAGGTTCAAGAGACCTTTGTCCCCAACACCTC (162) |
| | GTGGGCTCATTGGCTGAAGTTCAAGAGACTTCAGCCAATGAGCCCAC (163) |
| | GAGCTACTGAGGACAGAAATTCAAGAGATTTCTGTCCTCAGTAGCTC (164) |
| | |
| UBIQUITIN | TCAGCAGGATGCTCAGGAGTTCAAGAGACTCCTGAGCATCCTGCTGA (165) |
| CARBOXYL- | GAAGTTCTCCATCCAGAGGTTCAAGAGACCTCTGGATGGAGAACTTC (166) |
| TERMINAL | GCCGGTCCCCACCAGCAGCTTCAAGAGAGCTGCTGGTGGGGACCGGC (167) |
| HYDROLASE 2 | CACTCGGGAGTTGAGAGATTTCAAGAGAATCTCTCAACTCCCGAGTG (168) |
| | |
| UBIQUITIN | GCCCTTGGGTCTGTTTGACTTCAAGAGAGTCAAACAGACCCAAGGGC (169) |
| SPECIFIC | CTCAACACTAAACAGCAAGTTCAAGAGACTTGCTGTTTAGTGTTGAG (170) |
| PROTEASE 3 | GATTTCATTGGACAGCATATTCAAGAGATATGCTGTCCAATGAAATC (171) |
| (USP3) | CATGGGGCACCAACTAATTTTCAAGAGAAATTAGTTGGTGCCCCATG (172) |
| | |
| UBIQUITIN | GGTGTCTCTGCGGGATTGTTTCAAGAGAACAATCCCGCAGAGACACC (173) |
| CARBOXYL- | AGTTCAGTAGGTGTAGACTTTCAAGAGAAGTCTACACCTACTGAACT (174) |
| TERMINAL | GAGTTCCTGAAGCTCCTCATTCAAGAGATGAGGAGCTTCAGGAACTC (175) |
| HYDROLASE 23 | GGATTTGCTGGGGGCAAGGTTCAAGAGACCTTGCCCCCAGCAAATCC (176) |
| | |
| UBP-32.7 | CTCAGAAAGCCAACATTCATTCAAGAGATGAATGTTGGCTTTCTGAG (177) |
| | CGCATTGTAATAAGAAGGTTTCAAGAGAACCTTCTTATTACAATGCG (178) |
| | GGGAGGAAAATGCAGAAATTTCAAGAGAATTTCTGCATTTTCCTCCC (179) |
| | TTAGAAATTTAGGAAATACTTCAAGAGAGTATTGCTAAATTTGTAA (180) |
| | |
| HOMO SAPIENS | GTTATGAATTGATATGCAGTTCAAGAGACTGCATATCAATTCATAAC (181) |
| UBIQUITIN SPE- | GTGATAACACAACTAATGGTTCAAGAGACCATTAGTTGTGTTATCAC (182) |
| CIFIC PROTEASE | GTAGAGGAGAGTTCTGAAATTCAAGAGATTTCAGAACTCTCCTCTAC (183) |
| 13 (ISOPEP- | GCCTCTAATCCTGATAAGGTTCAAGAGACCTTATCAGGATTAGAGGC (184) |
| TIDASE T-3) | |
| | |
| UBIQUITIN | GATGATCTTCAGGCTGCCATTCAAGAGATGGCAGCCTGAAGATCATC (185) |
| CARBOXYL- | GTATGGACAAGAGCGTTGGTTCAAGAGACCAACGCTCTTGTCCATAC (186) |
| TERMINAL | CGAACCCTTCTGGAACAGTTTCAAGAGAACTGTTCCAGAAGGGTTCG (187) |
| HYDROLASE 28 | GTGGCATGAAGATTATAGTTTCAAGAGAACTATAATCTTCATGCCAC (188) |
| | |
| UBIQUITIN | GGTGAACAAGGACAGTATCTTCAAGAGAGATACTGTCCTTGTTCACC (189) |
| CARBOXYL- | GCAATAGAGGATGATTCTGTTCAAGAGACAGAATCATCCTCTATTGC (190) |
| TERMINAL | TCTGTGAATGCCAAAGTTCTTCAAGAGAGAACTTTGGCATTCACAGA (191) |
| HYDROLASE 14 | CACACCAGGGAAGGTCTAGTTCAAGAGACTAGACCTTCCCTGGTGTG (192) |
| | |
| DUB1 | GCAGGAAGATGCCCATGAATTCAAGAGATTCATGGGCATCTTCCTGC (193) |
| | GAATGTGCAATATCCTGAGTTCAAGAGACTCAGGATATTGCACATTC (194) |
| | TGGATGATGCCAAGGTCACTTCAAGAGAGTGACCTTGGCATCATCCA (195) |
| | GCTCCGTGCTAAACCTCTCTTCAAGAGAGAGAGGTTTAGCACGGAGC (196) |
| | |
| MOUSE USP27 | GCCTCCACCTCAACAGAGGTTCAAGAGACCTCTGTTGAGGTGGAGGC (197) |
| HOMOLOG | CTGCATCATAGACCAAATCTTCAAGAGAGATTTGGTCTATGATGCAG (198) |
| | GATCACTACATACATTTCCTTCAAGAGAGGAAATGTATGTAGTGATC (199) |
| | GTAAAGAGAGCAGAATGAATTCAAGAGATTCATTCTGCTCTCTTTAC (200) |
| | |
| UBIQUITIN | CGCGGGGCGCAGTGGTATCTTCAAGAGAGATACCACTGCGCCCCGCG (201) |
| CARBOXYL- | CAGAAGGCAGTGGGGAAGATTCAAGAGATCTTCCCCACTGCCTTCTG (202) |
| TERMINAL | GCCTGGGAGAATCACAGGTTTCAAGAGAACCTGTGATTCTCCCAGGC (203) |
| HYDROLASE4 | ACCAGACAAGGAAATACCCTTCAAGAGAGGGTATTTCCTTGTCTGGT(204) |
| | |
| TRE-2 | CACATCCACCACATCGACCTTCAAGAGAGGTCGATGTGGTGGATGTG (205) |
| | GTCACAACCCAAGACCATGTTCAAGAGACATGGTCTTTGGGTTGTGAC (206) |
| | CTCAACAGGACAAATCCCATTCAAGAGATGGGATTTGTCCTGTTGAG (207) |
| | TAGATCAATTATTGTGGATTTCAAGAGAATCCACAATAATTGATCTA (208) |
| | |
| UBIQUITIN CAR- | GGAACACCTTATTGATGAATTCAAGAGATTCATCAATAAGGTGTTCC (209) |
| BOXYL-TERMINAL | CTTTAACAGAAATTGTCTCTTCAAGAGAGAGACAATTTCTGTTAAAG (210) |
| HYDROLASE 15 | CCTATGCAGTACAAAGTGGTTCAAGAGACCACTTTGTACTGCATAGG (211) |
| (UNPH-2) | GATCTTTCTTGCTTTGGATTCAAGAGATCCAAAGCAAGAAAAGATC (212) |
| | |
| KIAA1372 | CAGCATCCTTCAGGCCTTATTCAAGAGATAAGGCCTGAAGGATGCTG (213) |
| | GATAGTGACTCGGATCTGCTTCAAGAGAGCAGATCCGAGTCACTATC (214) |
| | GACATCACAGCCCGGGAGTTTCAAGAGAACTCCCGGGCTGTGATGTC (215) |
| | GGACACAGCCTATGTGCTGTTCAAGAGACAGCACATAGGCTGTGTCC (216) |
| | |
| BRCA1 | GTGGAGGAGATCTACGACCTTCAAGAGAGGTTCGTTAGATCTCCTCCAC (217) |
| ASSOCIATED | CTCTTGTGCAACTCATGCCTTCAAGAGAGGCATGAGTTGCACAAGAG (218) |
| PROTEIN-1 | ACAGGGCCCCTGCAGCCTCTTCAAGAGAGAGGCTGCAGGGGCCCTGT (219) |
| | GAAGACCTGGCGGGAGGTGTTCAAGAGAGAGCTGGGGCGAGGTCTTC(220) |

A preferred embodiment of the method (1) of the invention concerns the following steps:
1. Generation of the short hairpin DNA containing the antisense- and sense-strand of the coding region of a gene (e.g. firefly luciferase; p53). Antisense and sense-strand are separated by a spacer of 5 to 9 bp.
2. Generation of constructs for the expression of the above mentioned shRNA under the control of a constitutive or inducible promoter (Pol II or Pol III dependent).
3. Insertion of the mentioned expression constructs into a ubiquitous expressed Pol II dependent locus by homologous recombination in ES cells.
4. Analysis of the constitutive and inducible inhibition of gene expression (e.g. firefly luciferase; p53) in ES cells (e.g. through Western blot analysis).
5. Generation of mice using the mentioned ES cells and analysis of the inhibition of gene expression in several tissues (e.g. firefly luciferase; p53; e.g. through Western blot analysis).

The vector according to embodiment (3) of the invention is suitable for stable or transient integration. Said vector is suitable for gene transfer.

The technology of the present application provides for the following advantages:
(i) A stable and body wide inhibition of gene expression by generating transgenic animals (such as mice).
(ii) A reversible inhibition of gene expression using the inducible constructs.

The invention is furthermore described by the following examples.

### Examples

### Methods

Plasmid construction: All plasmid constructs were generated by standard DNA cloning methods. The U6- and the H1-promoter fragments were amplified from human genomic DNA. The Renilla luciferase (Rluc) and firefly luciferase (Fluc) coding regions (Promega) were placed 3' of the adenovirus splice acceptor site (Friedrich and Soriano, Genes Dev. 9:1513-23 (1991); see SEQ ID NOs: 3, 5 and 7) to facilitate transcription from the endogenous *rosa26* promoter. For homologous recombination in embryonic stem cells, the expression cassettes were flanked by homology regions for the *Rosy26 locus* (see SEQ. ID NO: 2).

Cell culture: Culture and targeted mutagenesis of F1 (C57BL/6 x 129Sv/Ev) ES cells were carried out as previously described (Hogan,B., Beddington,R., Costantini, F. and Lacy, E. (1994). A Laboratory Manual, In Manipulating the Mouse Embryo. (Cold Spring Harbor Laboratory Press, Cold Spring Harbor NY.), pp. 253-289.). For Cre-mediated deletion, ES cells were transiently transfected with 5 µg of the cre expression plasmid pCAGGScre (SEQ ID NO: 18). 24 h after transfection, 1000 ES cells were plated on a 10 cm dish. Individual clones were isolated and analyzed for cre-mediated recombination by Southern blot using standard protocols.
For induction, cells were cultivated in cell culture medium containing 1 µg/ml Doxycycline (Sigma) for 2 days.
For transient transfection of cells, mixtures of 350 ng supercoiled plasmid DNA pre-complexed with the FuGene6 transfection reagent (Roche Diagnostics GmbH, Mannheim, Germany) were added to the cells in 250 µl medium, according to the manufacturers protocol. Each plasmid mixture contained 50 ng of the beta-galactosidase expression vector CMV-IacZ (SEQ ID NO: 17), 100 ng of CAGGS-Fluc (SEQ ID NO: 15), 100 ng of the tet inducible H1shRNA vector (SEQ ID NO: 11, 12 or 13), and pUC19 to a total amount of 350 ng DNA. For each sample to be tested four individual wells were transfected. One day after the addition of the DNA preparations, the cell culture medium was exchanged.

Luciferase measurement: Stably transfected cells: 25.000 cells were plated on a 24 well plate and analyzed 48h later. Luciferase activity was detected using the Dual-Glo^{™} Luciferase Assay System (Promega) and a Lumat LB 9507 (EG&G Berthold) according to the manufacturers protocols.
Transiently transfected cells: The cells were lysed 48 hours after transfection with 100 µl/well of lysate reagent supplemented with protease inhibitors (B-galactosidase reporter gene assay kit, Roche Diagnostics). The lysates were centrifuged and 20 µl were used to determine the µ-galactosidase activities using the β-galactosidase reporter gene assay (Roche Diagnostics) according to the manufacturers protocol in a Lumat LB 9507 luminometer (Berthold). To measure Luciferase activity, 20 µl of lysate was diluted into 250 µl assay buffer (50mM glycylglycin, 5mM MgCl₂, 5mM ATP) and the relative light units (RLU) were counted in a Lumat LB 9507 luminometer after addition of 100 µl of a 1 mM Luciferin (Roche Diagnostics) solution.

Generation of chimeric mice: Recombinant ES cells were injected into blastocysts from Balb/C mice and chimeric mice were obtained upon transfer of blastocysts into pseudopregnant females using standard protocols (Hogan et al. Manipulating the Mouse Embryo: A Laboratory Manual. Cold Sparing Harbor Laboratory Press, Cold Spring Harbor NY. 253-289 (1994)).

Luciferase measurement in organs: Organs were homogenized at 4 °C in lysis buffer (0.1M KH₂PO₄, 1mM DTT, 0.1% Triton^{®} X-100) using a tissue grinder. Spin for 5' at 2000xg (4 °C) to pellet debris and assay supernatant for Luc activities using the Dual Luciferase Assay (Promega, Inc.) according to the manufacturer protocol.

### Example 1

the firefly luciferase gene along with a splice acceptor sequence (Friedrich and Soriano, Genes Dev. 9:1513-23 (1991)) was inserted into the first allele of the *rosa26 locus* by homologous recombination in ES cells. The shRNA expression cassettes under the control of the H1 or the U6 promoter and a Renilla luciferase gene were inserted into the second allele of the rosa26 locus by homologous recombination. The Renilla luciferase gene was used as a reference to normalize the values of firefly luciferase activity.

Deleting the loxP-flanked shRNA expression cassette by transient transfection of a cre expression plasmid generated the negative control. Figure 7 shows the expression of the firefly luciferase in the absence and the presence of the siRNA expression cassette. Expression of the shRNA under the control of the H1 promoter resulted in a ∼75% reduction of firefly luciferase activity, whereas the repression mediated by the U6-shRNA construct was ∼60%.

### Example 2

The shRNA expression cassette under the control of the U6 promoter containing tet-operator sequences, and a Renilla luciferase gene were inserted into the first allele of the rosa26 locus (figure 8 and 12; SEQ ID NOs:9 and 10). The firefly luciferase gene under the control of the endogenous *rosa26* promoter and a tet repressor expression cassette (Gossen and Bujard, PEAS. 89: 5547-5551 (1992)) was inserted into the second allele of *rosa26* by homologous recombination in ES cells (figure 8; SEQ ID NO:14). To determine the extent of shRNA-mediated gene silencing, the cells were treated for 2 days in the presence or in the absence of 1 µg/ml Doxycycline in the cell culture medium. Figure 10 shows the firefly luciferase, activity measured in the cell extract. Doxycycline inducible expression of the shRNA under the control of the U6_{tetO1} promoter (SEQ ID NO:9) resulted in a ∼30% reduction of firefly luciferase activity, whereas repression mediated by the U6_{tetO2} -construct (SEQ ID NO 10) reached ∼50%.

### Example 3

NIH3T3 cells were transiently transected with constructs expressing the luciferase and tet repressor genes (SEQ ID NOs:15 and 16) together with the shRNA constructs under the control of the H1 promoter containing tet-operator sequences (sequence ID NOs:11, 12 and 13; figure 9). Figure 11 shows the expression of firefly luciferase in the absence and in the presence of 1 µg/ml doxycycline. The highest degree of doxycycline inducible shRNA expression was achieved with the H1_{tetO2/3}'-promoter, resulting in a ∼80% reduction of firefly luciferase activity.

### Example 4

To examine the activity of the Rosa26/U6- and H1-shRNA transgenes in vivo, recombinant ES cells were injected into diploid blastocysts and chimeric mice were obtained upon transfer of blastocysts into pseudopregnant females. Figure 13 shows the activity of firefly luciferase in different organs in the presence and absence of the shRNA expression cassettes. The Renilla luciferase gene at the second Rosa26 allele served as a reference to normalized the values of firefly luciferase activity. Expression of the shRNA under the control of both, the U6 as well as the H1 promoter resulted in efficient repression of firefly luciferase activity in most organs (Fig. 13A), with the highest degree of reduction (>90%) in liver, hearty brain and muscle (Fig. 13B).

## Claims

1. An *ex-vivo* method for constitutive and/or inducible gene knock down in a tissue culture or in cells of a cell culture derived from a vertebrate, which comprises stably integrating an expression vector
comprising a short hairpin RNA (shRNA) construct under control of a ubiquitous promoter selected from polymerase I, II and III dependent promoters,
being suitable for stable integration into the genome of the tissue culture or into the genome of the cells of the cell culture, and
containing homologous sequences suitable for integration at a polymerase II dependent locus through homologous recombination in the genome of the tissue culture or in the genome of the cells of the cell culture including embryonic cells
into the genome of the tissue culture or into the genome of the cells of the cell culture, provided that the method is not applied to human embryonic cells.

2. The method of claim 1, wherein the expression vector further contains functional sequences selected from splice acceptor sequences, polyadenylation sites and selectable marker sequences.

3. The method of claim 1 or 2, wherein the polymerase II dependent locus is selected from a Rosa26, collagen, RNA polymerase, actin and HPRT locus.

4. The method according to any one of claims 1 to 3, wherein
(i) the ubiquitous promoter is a polymerase II or III dependent promoter, preferably is a CMV promoter, a CAGGS promoter, a snRNA promoter such as U6, a RNAse P RNA promoter such as H1, a tRNA promoter, a 7SL RNA promoter or a 5 S rRNA promoter; and/or
(ii) the ubiquitous promoter is a constitutive promoter, or is an inducible promoter, preferably is a promoter containing an operator sequence selected from tet, Gal4 and lac; and/or
(iii) said vertebrate is a non-human vertebrate, preferably is a mouse or fish.

5. The method according to any one of claims 1 to 4, wherein the expression vector comprises
(a) a Pol III dependent promoter, preferably constitutive H1 or U6, driven shRNA construct suitable to be integrated into a ubiquitously active Pol II dependent locus;
(b) a Pol III dependent promoter, preferably inducible U6 or H1, driven shRNA construct suitable to be integrated into a ubiquitously active Pol II dependent locus; or
(c) a Pol II dependent promoter, preferably inducible CMV, driven shRNA construct suitable to be integrated into a ubiquitously active Pol II dependent locus.

6. The method according to any one of claims 1 to 5, wherein the shRNA comprises
(I) at least one DNA segment A-B-C wherein
A is a 15 to 35, preferably 19 to 29 bp DNA sequence with at least 95%, preferably 100% complementarity to the gene to be knocked down,
B is a spacer DNA sequence having 5 to 9 bp forming the loop of the expressed RNA hair pin molecule, and
C is a 15 to 35, preferably 19 to 29 bp DNA sequence with at least 85% complementarity to the sequence A; and
(II) a stop and/or polyadenylation sequence.

7. The method according to any one of claim 1 to 6, wherein said vertebrate is a non-human vertebrate, preferably is a mouse or fish.

8. The method of claim 7, which comprises integrating the expression vector into ES cells derived from the non-human vertebrate.

9. A non-human vertebrate, or tissue or cell culture derived from a vertebrate having stably integrated at a polymerase II dependent locus of the genome of the vertebrate, tissue culture or cells of the cell culture, an expression vector as defined in any one of claims 1 to 6, provided that said tissue or cell culture does not include human embryonic cells.

10. The non-human vertebrate tissue or cell culture of claim 9, which is or is derived from a non-human vertebrate.

11. The non-human vertebrate tissue or cell culture of claim 10, which is or is derived from mouse or fish.

12. An expression vector as defined in any one of claims 1 to 6.

## Patentansprüche

1. Ex-vivo-Verfahren zum konstitutiven und/oder induzierbaren Gen-Knock-Down in einer Gewebekultur oder in Zellen einer Zellkultur, die von einem Wirbeltier stammt, umfassend das stabile Integrieren eines Expressionsvektors,
der ein kurzes Hairpin-RNA(shRNA)-Konstrukt unter der Kontrolle eines ubiquitären Promotors, der aus Polymerase-I-, -II- und -III-abhängigen Promotoren ausgewählt ist, umfasst;
der für die stabile Integration in das Genom der Gewebekultur oder in das Genom der Zellen der Zellkultur geeignet ist; und
der homologe Sequenzen enthält, die für die Integration an einem Polymerase-II-abhängigen Locus durch homologe Rekombination in das Genom der Gewebekultur oder in das Genom der Zellen der Zellkultur einschließlich embryonaler Zellen geeignet sind;
in das Genom der Gewebekultur oder in das Genom der Zellen der Zellkultur, mit der Maßgabe, dass das Verfahren nicht auf humane embryonale Zellen angewendet wird.

2. Verfahren gemäß Anspruch 1, wobei der Expressionsvektor weiterhin funktionelle Sequenzen enthält, die aus Spleiß-Akzeptorsequenzen, Polyadenylierungsstellen und Selektionsmarkersequenzen ausgewählt sind.

3. Verfahren gemäß Anspruch 1 oder 2, wobei der Polymerase-II-abhängige Locus aus einem Rosa26-, Collagen-, RNA-Polymerase-, Actin- und HPRT-Locus ausgewählt ist.

4. Verfahren gemäß einem der Ansprüche 1 bis 3, wobei
(i) der ubiquitäre Promotor ein Polymerase-II- oder -III-abhängiger Promotor ist, vorzugsweise ein CMV-Promotor, ein CAGGS-Promotor, ein snRNA-Promotor, wie U6, ein RNase-P-RNA-Promotor, wie H1, ein tRNA-Promotor, ein 7SL-RNA-Promotor oder ein 5S-rRNA-Promotor ist; und/oder
(ii) der ubiquitäre Promotor ein konstitutiver Promotor oder ein induzierbarer Promotor ist und vorzugsweise ein Promotor ist, der eine Operatorsequenz enthält, die aus tet, Gal4 und lac ausgewählt ist; und/oder
(iii) das Wirbeltier ein nichthumanes Wirbeltier ist, vorzugsweise eine Maus oder ein Fisch ist.

5. Verfahren gemäß einem der Ansprüche 1 bis 4, wobei der Expressionsvektor
(a) ein von einem Pol-III-abhängigen Promotor, vorzugsweise konstitutivem H1 oder U6, gesteuertes shRNA-Konstrukt, das für die Integration in einen ubiquitär aktiven Pol-II-abhängigen Locus geeignet ist;
(b) ein von einem Pol-III-abhängigen Promotor, vorzugsweise induzierbarem U6 oder H1, gesteuertes shRNA-Konstrukt, das für die Integration in einen ubiquitär aktiven Pol-II-abhängigen Locus geeignet ist; oder
(c) ein von einem Pol-II-abhängigen Promotor, vorzugsweise induzierbarem CMV, gesteuertes shRNA-Konstrukt, das für die Integration in einen ubiquitär aktiven Pol-II-abhängigen Locus geeignet ist;
umfasst.

6. Verfahren gemäß einem der Ansprüche 1 bis 5, wobei die shRNA
(I) wenigstens ein DNA-Segment A-B-C, wobei
A eine 15 bis 35 bp, vorzugsweise 19 bis 29 bp, lange DNA-Sequenz mit wenigstens 95%, vorzugsweise 100%, Komplementarität zu dem einem Knock-Down zu unterziehenden Gen ist;
B eine Spacer-DNA-Sequenz ist, die 5 bis 9 bp aufweist, die die Schleife des exprimierten RNA-Hairpin-Moleküls bilden; und
C eine 15 bis 35 bp, vorzugsweise 19 bis 29 bp, lange DNA-Sequenz mit wenigstens 85% Komplementarität zur Sequenz A ist; und
(II) eine Stop- und/oder Polyadenylierungssequenz;
umfasst.

7. Verfahren gemäß einem der Ansprüche 1 bis 6, wobei das Wirbeltier ein nichthumanes Wirbeltier ist, vorzugsweise eine Maus oder ein Fisch ist.

8. Verfahren gemäß Anspruch 7, das das Integrieren des Expressionsvektors in ES-Zellen, die von dem nichthumanen Wirbeltier stammen, umfasst.

9. Nichthumanes Wirbeltier oder Gewebe oder Zellkultur, das bzw. die von einem Wirbeltier abgeleitet ist, und das bzw. die einen Expressionsvektor, wie er in einem der Ansprüche 1 bis 6 definiert ist, aufweist, der stabil an einem Polymerase-II-abhängigen Locus des Genoms des Wirbeltiers, des Gewebes oder der Zellen der Zellkultur integriert ist, mit der Maßgabe, dass das Gewebe oder die Zellkultur keine humanen embryonalen Zellen umfasst.

10. Nichthumanes Wirbeltier, Gewebe oder Zellkultur gemäß Anspruch 9, bei dem es sich um ein nichthumanes Wirbeltier handelt bzw. das oder die von einem solchen abgeleitet ist.

11. Nichthumanes Wirbeltier, Gewebe oder Zellkultur gemäß Anspruch 10, bei dem es sich um eine Maus oder einen Fisch handelt bzw. das oder die von einer solchen oder einem solchen abgeleitet ist.

12. Expressionsvektor, wie er in einem der Ansprüche 1 bis 6 definiert ist.

## Revendications

1. Procédé *ex vivo* d'inactivation génique constitutive et/ou inductible dans une culture tissulaire ou dans des cellules d'une culture cellulaire dérivées d'un vertébré, qui comprend l'intégration, de manière stable, d'un vecteur d'expression
comprenant un produit de construction d'ARN court en épingle à cheveux (shARN) sous le contrôle d'un promoteur ubiquitaire choisi parmi les promoteurs dépendant de la polymérase I, II et III,
approprié à une intégration stable dans le génome de la culture tissulaire ou dans le génome des cellules de la culture cellulaire, et
contenant des séquences homologues appropriées à une intégration au niveau d'un locus dépendant de la polymérase II, par l'intermédiaire d'une recombinaison homologue dans le génome de la culture tissulaire ou dans le génome des cellules de la culture cellulaire comprenant des cellules embryonnaires,
dans le génome de la culture tissulaire ou dans le génome des cellules de la culture cellulaire, sous réserve que le procédé ne soit pas appliqué à des cellules embryonnaires humaines.

2. Procédé selon la revendication 1, dans lequel le vecteur d'expression contient en outre des séquences fonctionnelles choisies parmi des séquences de sites accepteurs d'épissage, de sites de polyadénylation et de marqueurs de sélection.

3. Procédé selon la revendication 1 ou 2, dans lequel le locus dépendant de la polymérase II est choisi parmi les locus d'un gène Rosa26, d'un collagène, d'une polymérase de l'ARN, d'une actine et d'un gène HPRT.

4. Procédé selon l'une quelconque des revendications 1 à 3, dans lequel :
(i) le promoteur ubiquitaire est un promoteur dépendant de la polymérase II ou III, de préférence un promoteur CMV, un promoteur CAGGS, un promoteur snARN tel que U6, un promoteur ARN de l'ARNase P tel que H1, un promoteur ARNt, un promoteur ARN 7SL ou un promoteur ARNr 5 S ; et/ou
(ii) le promoteur ubiquitaire est un promoteur constitutif ou un promoteur inductible, de préférence un promoteur contenant une séquence opératrice choisie parmi tet, Gal4 et lac ; et/ou
(iii) ledit vertébré est un vertébré non humain, de préférence une souris ou un poisson.

5. Procédé selon l'une quelconque des revendications 1 à 4, dans lequel le vecteur d'expression comprend :
(a) un produit de construction de shARN sous le contrôle d'un promoteur dépendant de Pol III, de préférence le promoteur constitutif H1 ou U6, approprié à une intégration dans un locus ubiquitairement actif dépendant de Pol II ;
(b) un produit de construction de shARN sous le contrôle d'un promoteur dépendant de Pol III, de préférence le promoteur inductible U6 ou H1, approprié à une intégration dans un locus ubiquitairement actif dépendant de Pol II ; ou
(c) un produit de construction de shARN sous le contrôle d'un promoteur dépendant de Pol II, de préférence le promoteur inductible CMV, approprié à une intégration dans un locus ubiquitairement actif dépendant de Pol II.

6. Procédé selon l'une quelconque des revendications 1 à 5, dans lequel le shARN comprend :
(I) au moins un segment d'ADN A-B-C, dans lequel :
A est une séquence d'ADN de 15 à 35, de préférence de 19 à 29 pb ayant une complémentarité d'au moins 95 %, de préférence de 100 %, avec le gène à inactiver,
B est une séquence espaceur d'ADN de 5 à 9 pb formant la boucle de la molécule d'ARN en épingle à cheveux exprimée, et
C est une séquence d'ADN de 15 à 35, de préférence de 19 à 29 pb, ayant une complémentarité d'au moins 85 % avec la séquence A ; et
(II) une séquence d'arrêt et/ou de polyadénylation.

7. Procédé selon l'une quelconque des revendications 1 à 6, dans lequel ledit vertébré est un vertébré non humain, de préférence une souris ou un poisson.

8. Procédé selon la revendication 7, qui comprend l'intégration du vecteur d'expression dans des cellules ES dérivées du vertébré non humain.

9. Vertébré non humain, ou culture tissulaire ou cellulaire dérivée d'un vertébré, présentant, intégré de manière stable au niveau d'un locus dépendant de la polymérase II du génome du vertébré, de la culture tissulaire ou des cellules de la culture cellulaire, un vecteur d'expression tel que défini dans l'une quelconque des revendications 1 à 6, sous réserve que ladite culture tissulaire ou cellulaire ne comprenne pas de cellules embryonnaires humaines.

10. Culture tissulaire ou cellulaire de vertébré non humain selon la revendication 9, qui provient, ou est dérivée, d'un vertébré non humain.

11. Culture tissulaire ou cellulaire de vertébré non humain selon la revendication 10, qui provient, ou est dérivée, de souris ou de poisson.

12. Vecteur d'expression tel que défini dans l'une quelconque des revendications 1 à 6.
